# EUROPÄISCHE PATENTSCHRIFT

(11) **EP 1 778 238 B1**
(45) Veröffentlichungstag und Bekanntmachung des Hinweises auf die Patenterteilung: **12.10.2011**
(21) Anmeldenummer: 05778137.9
(22) Anmeldetag: 11.08.2005
(51) Int. Cl.: A61K 31/519, A61K 31/4985, A61P 29/00, A61P 35/00

(54) **LAGERSTABILE INFUSIONSLÖSUNG VON DIHYDROPTERIDINONEN**
DIHYDROPTERIDINONE INFUSION SOLUTION HAVING A LONG SHELF LIFE
SOLUTIONS DE PERFUSION DE DIHYDROPTERIDINONES STABLES AU STOCKAGE

(30) Priorität: 14.08.2004 EP 04019363
(43) Veröffentlichungstag der Anmeldung: 02.05.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: MOHR, Detlef, 88400 Biberach (DE); VEIT, Claus, 88400 Biberach (DE); TRAULSEN, Fridtjof, 88400 Biberach (DE)
(74) Vertreter: Hammann, Heinz
(86) Internationale Anmeldenummer: PCT/EP2005/008735
(87) Internationale Veröffentlichungsnummer: WO 2006/018221

(56) Entgegenhaltungen:
- WO-A-01/78732
- WO-A-03/020722
- WO-A1-01/78732

## Beschreibung

Die vorliegende Erfindung betrift lagerstabile wässrige infusions- bzw. Injektions lösungen enthaltend einen Wirkstoff der Formel (I) 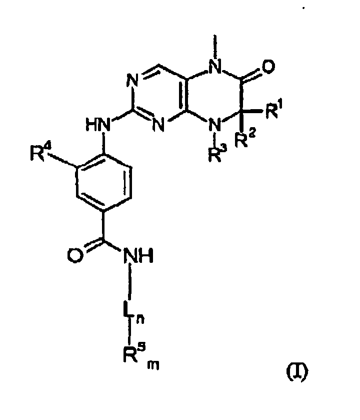 wobei die Reste L, R¹, R², R³, R⁴ und R⁵ die in den Ansprüche und der Beschreibung genannten Bedeutungen haben und eine zur Lösung des Wirkstoffs und zur Stabilisierung ausreichenden Menge einer physiologisch verträglichen Säure oder eines Säuregemisches, sowie gegebenenfalls weitere für die parenterale Anwendung geeignete Formulierungshilfsmittel, sowie ein Verfahren zur Herstellung der erfindungsgemäßen Infusions- bzw. Injektions lösungen.

### Hintergrund der Erfindung

Die erfindungsgemäßen Dihydroptoridinoae der Formel (I) und Anspruch 1 stellen ein innovatives neues zytostatisches Wirkprinzip in der onkologischen Therapie schnell wachsender Krebsarten dar. Üblicherweise kommen zytostatische Medikationen als parenterale Arzneiformen zur Anwendung, obwohl ihre orale Bioverfügbarkeit durchaus hinreichen sein kann. Grund dafür ist, daß die Therapie mit Zytostatika in der Regel begleitet ist durch ein gastrointestinales Nebenwirkungsprofil, das häufig durch Übelkeit, Erbrechen und/oder Durchfall gekennzeichnet ist, und dadurch ein zuverlässiger Therapieerfolg bei oraler Gabe gefährdet wäre.

Diese Umstände gelten sinngemäß auch für die Dihydropteridinone der Formel (I) nach Anspruch 1 und machen die Bereitstellung einer parenteralen Infusions- bzw. Injektionslösung notwendig.

Im Stand der Technik beschreiben EP 0219784 und WO 01/78732 Herstellverfahren und Maßnahnmen zur Stabilisierung von Ciprofloxaxinhaltigen Infusionslösungen durch Verwendung einer oder mehrerer physiologisch verträglicher Säure(n) organischen oder anorganischen Ursprungs. EP A 0287926 beschreibt, dass durch den Einsatz hochreiner Ciprofloxaxinqualitäten die Gefahr der Partikelbildung zurückgedrängt werden kann. EP 0143478 A1 beschreibt die Herstellung einer stabilen salzsauren Lösung des Cisplatins, geeignet zur Injektion, die insbesondere frei ist von weiteren Zusätzen- DE 197 03023 offenbart, dass sich die Stabilität von Infusonslösunge hinsichtlich Bildung von partikulären Verunreinigungen durch den Einsatz von Glasbehältnissen mit silikonisierten Oberflächen entscheidend verbessern lässt. WO031020722 offenbart Verbindungen der Formel (1) sowie deren Verwendung als Arzneimittel.

Es ist die Aufgabe der vorliegenden Erfindung eine stabile Infusions- bzw. Injektions lösung von Dihydropteddinonen der Formel (I) nach Anspruch (I) für den gewünschten therapieoptimierten Dosisbereich bereitzustellen. Als weitere Aufgabe der Erfindung sollte die stabile Infusions- bzw. Injektionslösung sowohl als gebrauchsfertige Lösung (ready-to-use), als auch als Konzentrat für weitere Verdünnungen mit für die parenterale Anwendung gängigen Lösungen wie beispielsweise isotoner NaCL-Lösung, isotoner Dextroselösung oder Ringerlactatlösung geeignet sein, um eine flexible Dosisanpassung zu ermöglichen.

### Beschreibung der Erfindung

Überraschend wird gefunden, dass lagerstabile wässrige Infusions- bzw. Injektions lösungen enthaltend einen Wirkstoff der allgemeinen Formel (I) nach Anspruch 1, die eine zur Lösung des Wirkstoffs und zur Stabilisierung ausreichenden Menge einer physiologisch verträglichen Säure oder eines Säuregemisches, sowie gegebenenfalls weitere für die parenterale Anwendung geeignete Formulierungshilfsmittel enthalten, unabgängig von der jeweils vorliegenden Wirkstoffqualität, insbesondere unabhängig vom Verbunreitigsprofil partikelfrei und langzeitstabil herstellbar sind.

Die vorliegende Erfindung offenbart lagerstabile wässrige Infusions- bzw. Injektionslösungen enthaltend den Wirkstoff der allgemeinen Formel (I) nach Anspruch 1

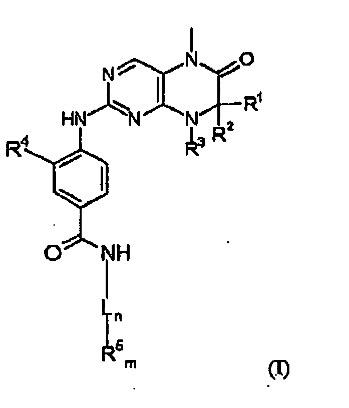

Langzeitstabil bedeutet eine Lagerstabilität von mindestens 12 Monaten bei 25°C/60% r.h. und 30°C/70% r.h., vorzugsweise mindestens 36 Monate bei 25°/60% r.h.und 30°C/70% r.h..

Die erfindungsgemäßen Infusions- bzw. Injektions lösungen können neben dem Zusatz einer physiologisch verträglichen Säure oder eines Säuregemisches frei sein von lösungsvermittelnden Zusatzstoffen oder organischen Cosolventien, insbesondere von organischen Cosolventien.

Bevorzugt sind wässrige Infusions- bzw. Injektions lösungen, worin der Gehalt an gelöstem Wirkstoff der Formel (I) 0,1 mg bis 10,0 mg, insbesondere 0,5 bis 5 mg, in 1 ml Infusions- bzw. Injektionslösung beträgt.

Weiterhin bevorzugt sind wässrige Infusions- bzw. Injektionslösungen, worin eine oder mehrere zur Erzielung der Lager- und Verdünnungsstabilität eingesetzten Säuren ausgewählt sind aus der Gruppe bestehend aus Salzsäure, Essigsäure, Hydroxyessigsäure, Methansulfonsäure, Ethansulfonsäure, Phosphorsäure, Salpetersäure, Schwefelsäure, Zitronensäure, Weinsäure, Fumarsäure, Bernsteinsäure, Glutarsäure, Adipinsäure, Propionsäure, Ascorbinsäure, Maleinsäure, Äpfelsäure, Glutaminsäure, Glukonsäure, Glukuronsäure, Galakturonsäure und Milchsäure, vorzugsweise aus Essigsäure, Salzsäure, Phosphorsäure, Weinsäure, Zitronensäure und Fumarsäure, insbesondere bevorzugt aus Salzsäure, Zitronensäure, und Essigsäure.

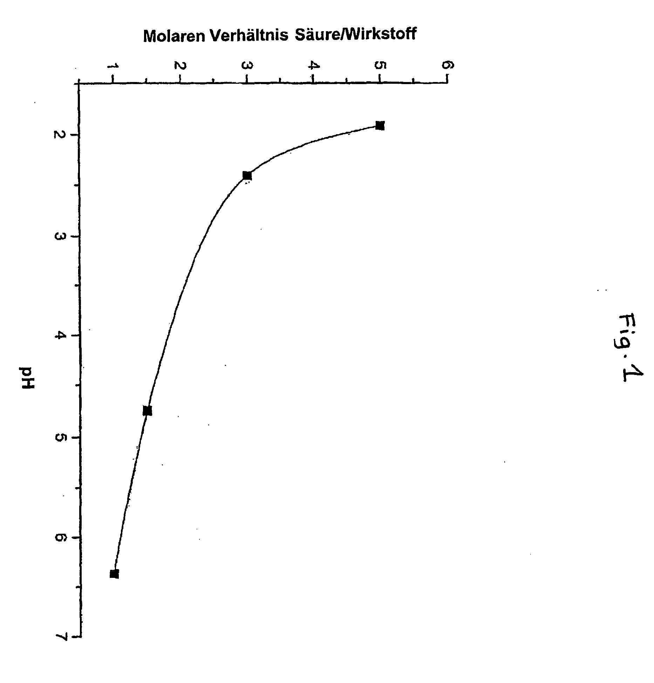

Aus pH-Verträglichkeitsgründen sind, wie aus Figur 1 ersichtlich, wässrige Infusions- bzw. Injektionslösungen bevorzugt, worin das molare Verhältnis der physiologisch verträglichen Säure oder des Säuregemisches zum Wirkstoff maximal 3:1, vorzugsweise 1,25:1 bis 3:1, besonders bevorzugt 1,5 :1 bis 3:1 beträgt, um einen pH-Wert größer als 2,4 zu gewährleisten.

Bevorzugt betrifft die Erfindung auch lnfusions- bzw. Injektions lösungen, die 0,1 mg bis 10,0 mg Wirkstoff pro Milliliter wässrige Lösung und bis zu 3,0 Mol Salzsäure, bezogen auf ein Mol Wirkstoff enthalten. Die Salzsäuremengen betragen dabei vorzugsweise 1,25 Mol bis 3,0 Mol, insbesondere 1,5 bis 2,4 Mol.

Ein weiterer Gegenstand der Erfindung sind Infusions- bzw. Injektionslösungen von 4-[[(7*R*)-8-cyclopentyl-7-ethyl-5,6,7,8-tetrahydro-5-methyl-6-oxo-2-pteridinyl]amino]-3-methoxy-N-(1-methyl-4-piperidinyl)-Benzamid, die 1,6 bis 2,0 Mol Salzsäure pro Mol Wirkstoff enthalten.

Die erfindungsgemäßen Infusions- bzw. Injektions lösungen können auch dahingehend modifiziert sein, dass sie bis zu 10 mg/ml des Wirkstoffes, und bis zu 1 Mol Salzsäure pro Mol Wirkstoffstoff enthalten, sowie eine oder mehrere weitere physiologisch verträgliche Säure(n) enthalten, mit der Maßgabe, dass die Gesamtsäuremenge mindestens 1,25 Mol pro Mol Wirkstoff beträgt, jedoch 3,0 Mol pro Mol Wirkstoff nicht übersteigt.

Die pro Mol Wirkstoff minimal notwendige Menge Säure hängt von der Wirkstoffkonzentration, und der (den) verwendeten Säure(n) ab, und ist somit nicht konstant. Sie kann jedoch in den erfindungsgemäßen Grenzen durch einfache Versuche wie beispielsweise in EP 0219784 und WO 01/78732 beschrieben ermittelt werden

Insbesondere bevorzugt sind wässrige Infusions- bzw. Injektionslösungen, die ein oder mehrere weitere Formulierungshilfsmittel ausgewählt aus der Gruppe bestehend aus Komplexierungsmitteln, Kristallisationsverzögerem, Verdickungsmitteln, Isotonisierungsmitteln, Konservierungsmitteln, Lichtschutzmitteln, und Antioxidantien, enthalten

Geeignete Komplexierungsmittel sind z.B. genuine und substituierte Cyclodextrine, EDTA, Albumine, sowie Zitronensäure, deren Salze und Derivate.

Geeignete Kristallisationsverzögerer sind z.B. PVP, Cellulosederivate, Alginate, Poloxamere und Polysorbate.

Geeignete Verdickungsmittel sind beispielsweise Dextrane, Glycerol und lösliche Cellulosederivate, insbesondere Carboxymethylcellulose und ihre Salze, sowie Hydroxyalkylcellulosen

Geeignete Isotonisierungsmittel sind beispielsweise NaCl, Mannit, Sorbit, Xylit, Saccharose, Lactose, Glucose und Glycerol, vorzugsweise NaCl, Mannit, Glucose, Saccharose und Glycerol, insbesondere bevorzugt NaCl, Mannit und Glucose.

Geeignete Konservierungsmittel sind beispielsweise die Ester der p-Hydroxybenzoesäure, Benzylalkohol, Sorbinsäure und Benzoesäure.

Geeignete Lichtschutzmittel sind beispielsweise Derivate der p-Hydroxybenzoesäure sowie Zimtsäure und deren Derivate.

Ein geeignetes Antioxidans ist beispielsweise Ascorbinsäure und ihre Salze.

Insbesondere bevorzugt sind ebenfalls wässrige Infusions- bzw. Injektionslösungen, worin die Osmolalität der Infusions- bzw. Injektionslösungen 200 - 600 mOsmol/kg, vorzugsweise 260 - 350 mOsmol/ kg beträgt. Sie können durch Isotonisierungsmittel wie NaCl, Mannit, Sorbit, Glucose, Saccharose, Xylit, Fruktose und Glycerin oder Gemische vorgenannter Substanzen eingestellt werden. Bevorzugt sind Infusions- bzw. Injektions lösungen die außer Wirkstoff, Wasser, Säure(n) und sonstigen Formulierungshilfsmitteln eine derartige Menge NaCl oder andere Isotonisierungsmittel enthalten, dass eine mit der Gewebeflüssigkeit des menschlichen oder tierischen Körpers isotone oder geringfügig hypo- oder hypertone Lösung vorliegt.

Überaus bevorzugt sind wässrige Infusions- bzw. Injektionslösungen, die einen pH-Wert im Bereich von 2,4 bis 5,3, vorzugsweise von 3,5 bis 5,0, besonders bevorzugt von 3,9 bis 4,5, aufweisen.

Die erfindungsgemäßen Infusions- bzw. Injektions lösungen sind darüber hinaus geeignet mit handelsüblichen Infusions- bzw. Injektionsträgerlösungen zur Elektrolytzufuhr ohne Kohlehydratsatz wie isotone NaCl-Lösung, isotone Glucoselösung, Ringerlactatlösung und ähnliche (Rote Liste 2004, Verzeichnis der Fertigarzneimittel der Mitglieder des Bundesverbandes der Pharmazeutischen Industrie e.V., Editio Cantor, Aulendorf/Württ., Hauptgruppen 52.1 und 52.2.1) auf die gewünschte Konzentration bzw. Dosis verdünnt zu werden, ohne physikalische oder chemische Inkompatibilitäten aufzuweisen.

Ebenfalls überaus bevorzugt sind wässrige Infusions- bzw. Injektionslösungen, die 1,25 bis 3,0 Mol, vorzugsweise 1,5 bis 2,4 Mol, Salzsäure pro Mol Wirkstoff, bezogen auf 100 ml Infusions- bzw. Injektionslösung, 0,75 bis 1,2 g NaCL, vorzugsweise 0,85 bis 0,95 g NaCL, enthalten und eine Osmolalität von 260 bis 350 mOsmol/kg sowie einen pH-Wert von 3,5 bis 5,0 aufweisen.

Ein weiterer Gegenstand der Erfindung sind Lyophilisate, Konzentrate und Suspensionen, welche durch Zugabe von Wasser eine der erfindungsgemäßen wässrigen Infusions- bzw. Injektionslösungen ergeben.

Ein weiterer Gegenstand der Erfindung sind die erfindungsgemäßen Infusions- bzw. Injektions lösungen zur Verwendung als Arzneimittel mit antiproliferativer Wirkung.

Ein weiterer Gegenstand der Erfindung ist die Verwendung der erfindungsgemäßen Infusions- bzw. Injektionslösungen zur Herstellung eines Arzneimittels zur Behandlung von Tumorerkrankungen, Infektionen, Entzündungs- und Autoimmunerkrankungen.

Ein weiterer Gegenstand der Erfindung ist eine Methode zur Behandlung und/oder Prävention von Tumorerkrankungen, Infektionen, Entzündungs- und Autoimmunerkrankungen, vorzugsweise von Tumorerkrankungen, wobei man einem Patienten eine effektive Menge einer erfindungsgemäßen Infusions- bzw. Injektions lösung verabreicht.

Ein weiterer Gegenstand der Erfindung ist die Verwendung der erfindungsgemäßen Infusions- bzw. Injektionslösungen, welche einem Dosierungsbereich von 0,1 bis 50 mg Wirkstoff /kg Körpergewicht, vorzugsweise 0,5 bis 25 mg Wirkstoff /kg Körpergewicht entspricht.

Die erfindungsgemäßen Infusions- bzw. Injektions lösungen können in geeigneten Glascontainern für Parenteralia oder in flexiblen Plastikcontainern vorliegen, vorzugsweise non-PVC Materialien z.B. auf Polyolefinbasis, mit entnehmbaren Volumina von 20 bis 1000 ml, bevorzugt 50 bis 500 ml. Die Behältnisse können so beschaffen sein, dass sie den erfindungsgemäßen Infusions- bzw. Injektionslösungen einen besonderen Schutz verleihen, z.B. vor Licht oder Sauerstoff. Eine besondere Oberflächenbehandlung der Primärpackmittel (z.B. (Einbrenn)-Silikonisierung von Glascontainer-oberflächen) zur Verbesserung der Stabilität der erfindungsgemäßen Infusions- bzw. Injektionslösungen ist weder notwendig noch schädlich. Flexible Plastikcontainer können einen zusätzlichen Schutz z.B. in Form einer Aluminiumumverpackung beinhalten.

Die erfindungsgemäßen Infusions- bzw. Injektions lösungen sind geeignet Terminal sterilisiert zu werden, z.B. mit gespanntem Wasserdampf, und können damit besonders wirtschaftlich und mit hoher Produktsicherheit (geringes Kontaminationsrisiko) steril und pyrogenfrei hergestellt werden.

Die Herstellung der erfindungsgemäßen Infusions- bzw. Injektions lösung kann nach aus der Literatur bekannten Herstellungsweisen für wässrige Flüssigformulierungen erfolgen.

So betrifft die vorliegende Erfindung ein Verfahren zur Herstellung der erfindungsgemäßen Infusions- bzw. Injektions lösungen, enthaltend 0,1 bis 10 mg pro Milliliter Wirkstoff der Formel (I). Das Verfahren ist dadurch gekennzeichnet, dass man eine geeignete Menge Wirkstoff, gegebenenfalls in Form eines Salzes, mit einem anionischen Gegenion, eines Hydrates oder Hydrates eines Salzes, oder auch Mischungen dieser Salze/Hydrate mit der Menge einer physiologisch verträglichen Säure oder eines Säuregemisches versetzt, die in Bezug auf die gerade zur Auflösung des Wirkstoffes bzw. seiner Salze oder Hydrate sowie zur Verhinderung physikalischer Instabilitäten einen Überschuß darstellt, gegebenenfalls weitere Formulierungshilfsmittel zusetzt, und mit Wasser (für Injektionszwecke) derart auffüllt, dass sich ein Konzentrationsbereich von 0,1 bis 10 mg Wirkstoff pro Milliliter Infusions- bzw. Injektionslösung ergibt.

Bei der Herstellung der Infusions- bzw. Injektionslösungen ist weiterhin zu beachten, dass die Lösung den bereits angeführten Eigenschaften hinsichtlich pH, Säuremengen, und Osmolalitäten entspricht. Für den Fall der Verwendung eines Salzes kann zweckmäßig eine Säure eingesetzt werden, deren Anion dem Anion des Wirkstoffsalzes bzw. dem des Salz-hydrates entspricht.

Gegebenfalls wird der Wirkstoff oder sein Salz oder Hydrat in Wasser suspendiert, und es werden bis zu 3,0 Mol physiologisch verträgliche Säure oder Säuregemisch, bevorzugt Salzsäure, pro Mol Wirkstoff zugegeben.

Abschließend werden die weiteren Formulierungshilfsmittel zugesetzt, insbesondere Isotonisierungsmittel, bevorzugt NaCL, das gegebenenfalls auch durch Neutralisierungsreaktion im Formulierungsansatz entstehen kann, bevor mit Wasser auf die gewünschte Wirkstofflconzentration eingestellt wird.

Der pH-Wert der erfindungsgemäßen Infusions- bzw. Injektions lösungen lässt sich mit (physiologisch) verträglichen Säuren und/oder Basen insbesondere NaOH auf die vorgenannten pH-Werte einstellen.

Zur Beschleunigung des Herstellverfahrens, insbesondere zur Auflösung der festen Bestandteile können die Lösungen insgesamt oder anteilig geringfügig erwärmt werden, vorzugsweise auf Temperaturen zwischen 20°C und 80°C.

Besonders wirtschaftlich können die Erfindungsmäßigen Lösungen hergestellt werden über konzentrierte Lösungen. Dazu wird die für einen Ansatz erforderliche Wirkstoffmenge mit der Hauptmenge (>90%) der physiologisch verträglichen Säure oder des Säuregemisches versetzt, und gegebenenfalls unter leichtem Erwärmenund/oder Zusatz einer geringen Menge Wassers gelöst. Dieses Konzentrat wird anschließend mit Wasser verdünnt bevor die weiteren Formulierungshilfsmittel zugesetzt werden, sowie abschließend die Restmengen Säure(n) bzw. Wasser, mit denen auf Nominalgewicht aufgefüllt wird.

Nach dem Herstellen der Lösung wird diese im allgemeinen über 0,2 µm Membran-oder Tiefenfilter filtriert, obwohl sie abschließend mit gespanntem Wasserdampf Terminal sterilisiert wird, um ggf. vorhandene Partikel und/oder Pyrogene abzutrennen. Details zu geeigneten Filtrationsmethoden sind aus dem Stand der Technik bekannt (M.J.Groves, Parenteral Technology Manual, Interpharm Press Inc., 2. ed. 1988). Die Partikelanzahl wird dabei auf das regulatorisch Notwendige und wirtschaftlich Sinnvolle begrenzt, beispielsweise 6000 Partikel = 10 µm und 600 Partikel = 25 µm pro Gebinde (Gebinde = 100 mL) bzw. 25 Partikel = 10 µm und 3 Partikel = 25 µm pro Milliliter (Gebinde > 100 mL), USP 27 <788>.

Die erfindungsgemäßen Lösungen zeigen eine hohe Lagerstabilität, die weder durch die Anzahl von Partikeln im sichtbaren und subvisuellen Bereich, noch durch signifikante Wirkstoffabbaureaktionen begrenzt ist.

Die erfindungsgemäßen Lösungen haben eine mit Hinblick auf die pharmakodynamischen Eigenschaften des Wirkstoffes hinreichende lokale Verträglichkeit, und sind nicht hämolytisch.

Die erfindungsgemäßen Infusions- bzw. Injektions lösungen sollen durch nachfolgende Beispiele erläutert werden. Die Beispiele dienen nur der Veranschaulichung und sind nicht als limitierend anzusehen

Abbildung 1 zeigt die Abhängigkeit des pH-Wertes der applikationsfertigen Lösung vom Molaren Verhältnis Säure/Säuregemisch zu Wirkstoff. Der Wirkstoff ist hier 4-[[(7R)-8-cyclopentyl-7-ethyl-5,6,7,8-tetrahydro-5-methyl-6-oxo-2-pteridinyl]amino]-3-methoxy-N-(1-methyl-4-piperidinyl)-Benzamid (Beispiel 46 aus Tabelle 1).

Aus Gründen der besseren lokalen Verträglichkeit für eine iv-Infusion/Injektion wird das maximale Molverhältnis Säure(n) zu Wirkstoff der erfindungsgemäßen Infusions- bzw. Injektionslösung auf maximal 3:1 begrenzt, um einen pH-Wert größer als 2,4 zu gewährleisten.

### Beispiele von parenteralen Infusion-bzw. Injektionslösungen

Die Abkürzung WFI steht für Wasser Für Injektionszwecke.

In folgendes allgemeines Beispiel 1 ist der Wirkstoff eine der Dihydropteridinone der allgemeinen Formel (I) wie vorhin beschrieben

Allgemeines

### Beispiel 1

| Wirkstoff | 1-10 mg/ml |
|---|---|
| Organische oder | 1,0 - 3,0 Mol |
| mineralische Säure, | (berechnet auf Basis |
| oder Säure | des Wirkstoffes) |
| Mischung | |
| Isotonisches Mittel | e.g. 9 mg/ml oder |
| (e.g. NaCl/Mannitol) | 50 mg/ml |
| WFI ad | Endvolumen, |
| | e.g. 1,0 ml |
| pH | 3,0 - 4,5 |

In folgende Beispiele ist der Wirkstoff 4-[[(7*R*)-8-cyclopentyl-7-ethyl-5,6,7,8-tetrahydro-5-methyl-6-oxo-2-pteridinyl]amino]-3-methoxy-*N*-(1-methyl-4-piperidinyl)-Benzamid (Beispiel 46 aus Tabelle 1).

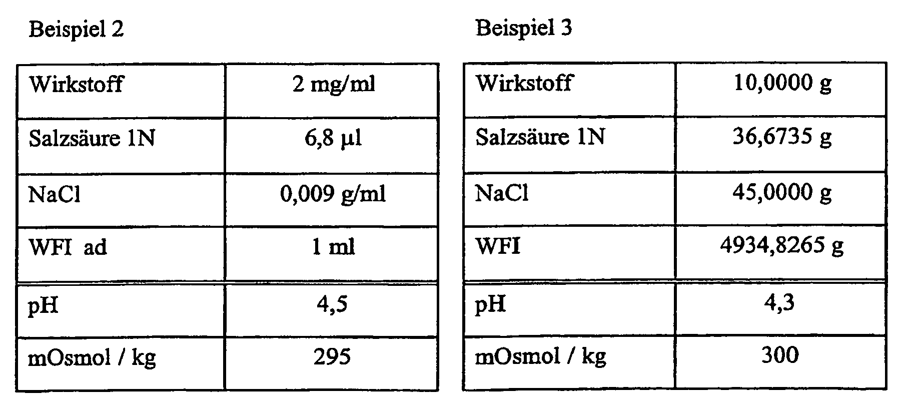

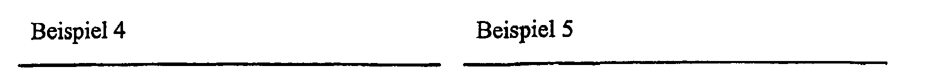

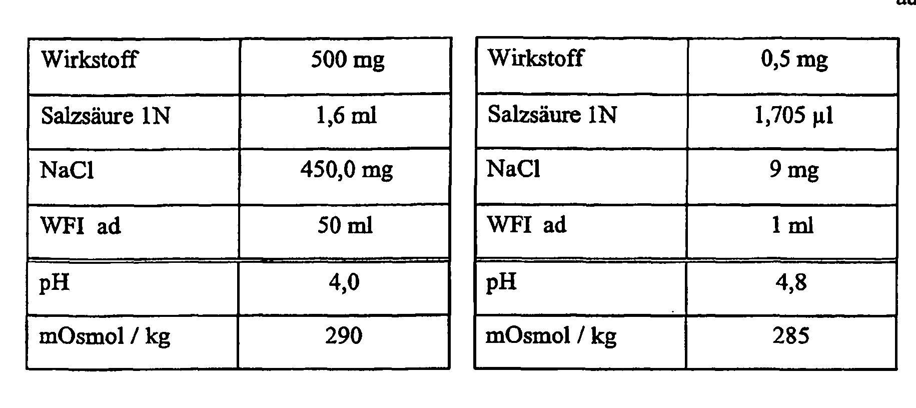

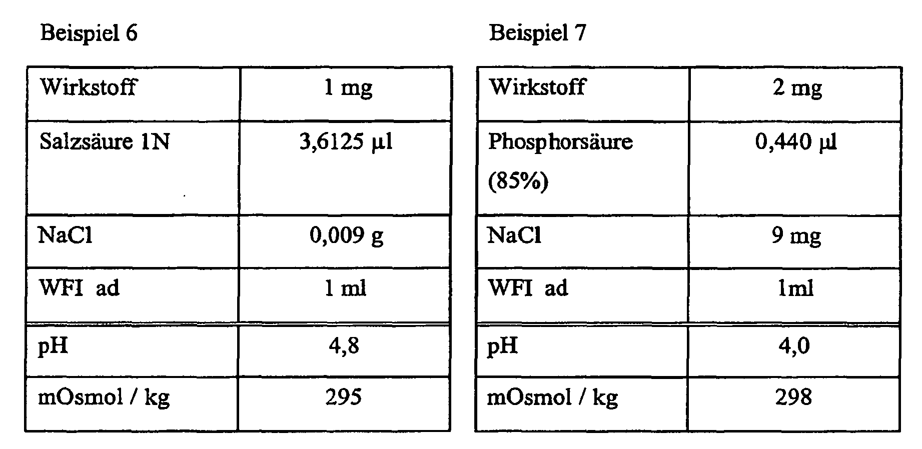

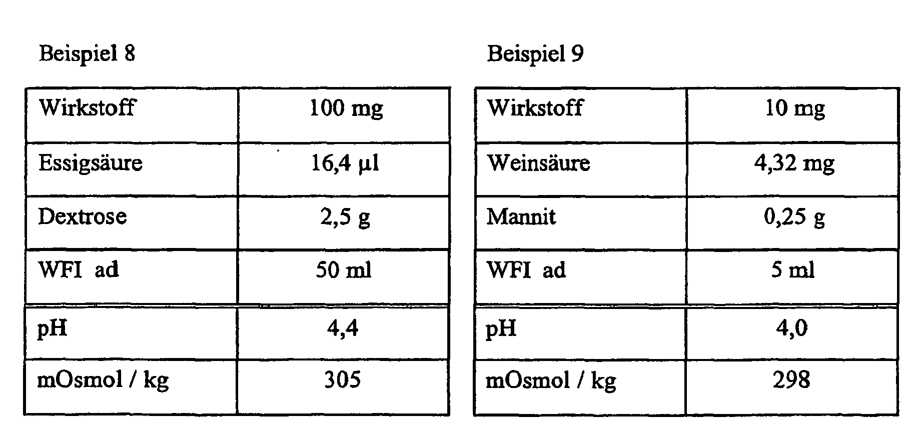

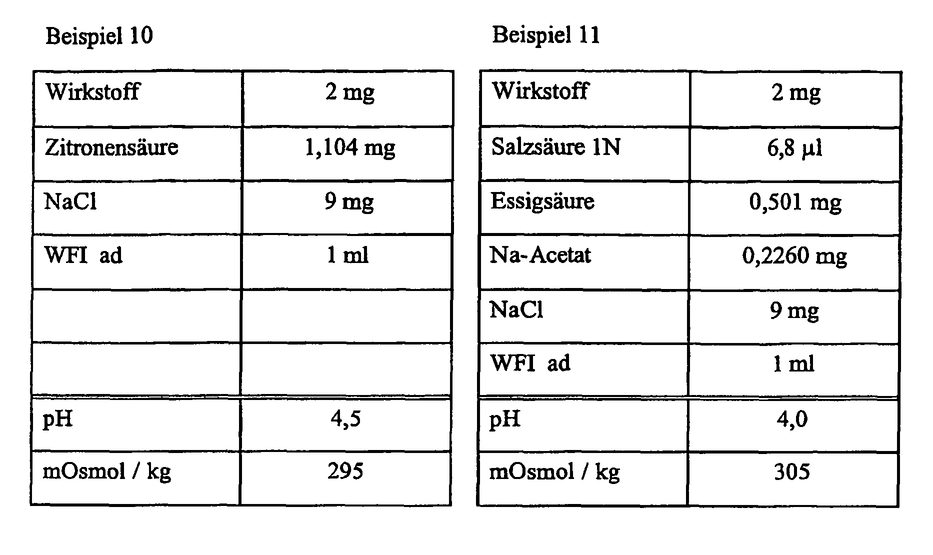

In folgende Beispiele ist der Wirkstoff N-[trans-4-[4-(cyclopropylmethyl)-1-piperazinyl]cyclohexyl]-4-[[(7R)-7-ethyl-5,6,7,8-tetrahydro-5-methyl-8-(1-methylethyl)-6-oxo-2-pteridinyl]amino]-3-methoxy-Benzamid (Beispiel 110 aus Tabelle 1).

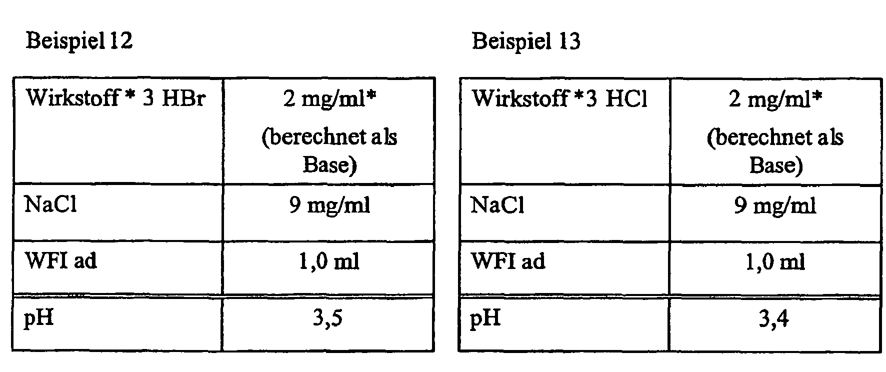

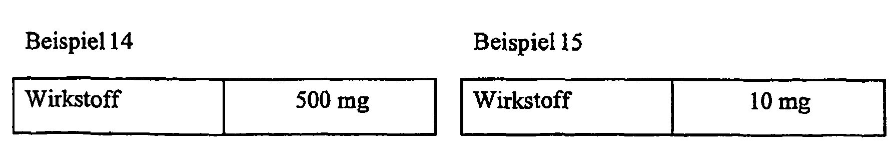

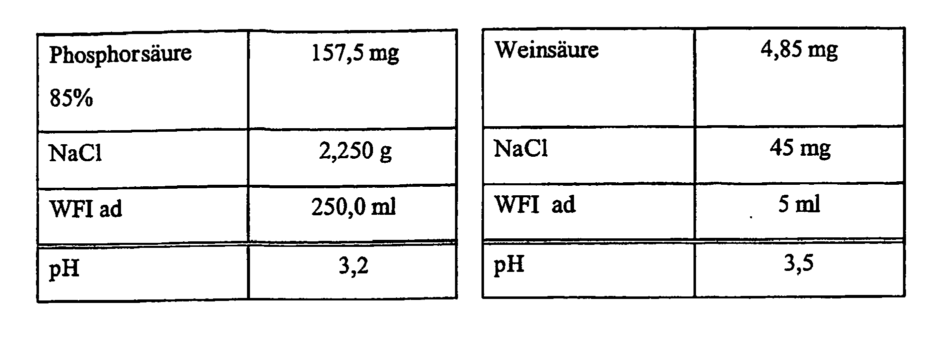

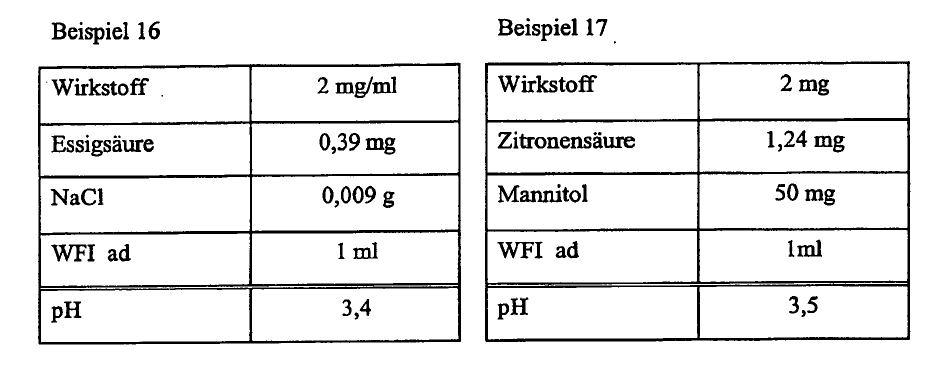

Die Herstellung der erfindungsgemäßen Verbindungen kann nach den im folgenden beschriebenen Syntheseverfahren A erfolgen, wobei die Substituenten der allgemeinen Formeln (A1) bis (A9) die zuvor genannten Bedeutungen haben. Dieses Verfahren ist als Erläuterung der Erfindung zu verstehen ohne selbige auf deren Gegenstand zu beschränken.

### Verfahren A

### Stufe 1A

Eine Verbindung der Formel (A1) wird mit einer Verbindung der Formel (A2) zu einer Verbindung der Formel (A3) umgesetzt (Schema 1A). Diese Reaktion kann gemäß WO 00/43369 oder WO 00/43372 durchgeführt werden. Verbindung (A1) ist kommerziell, beispielsweise bei City Chemical LLC, 139 Allings Crossing Road, West Haven, CT, 06516, USA, erhältlich. Verbindung (A2) kann nach Literaturbekannten Vorschriften, z.B. aus (a) F. Effenberger, U. Burkhart, J. Willfahrt Liebigs Ann. Chem. 1986, 314-333, (b) T. Fukuyama, C.-K. Jow, M. Cheung, Tetrahedron Lett. 1995, 36, 6373-6374, (c) R. K. Olsen, J. Org. Chem. 1970, 35, 1912-1915, (d) F.E. Dutton, B.H. Byung Tetrahedron Lett. 1998, 30, 5313-5316 oder (e) J. M. Ranajuhi, M. M. Joullie Synth. Commun. 1996, 26, 1379-1384, hergestellt werden.

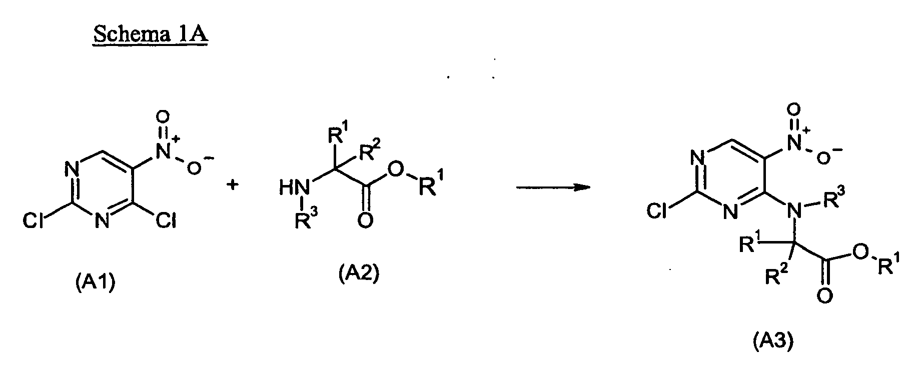

In Stufe 1A werden 1 Äquivalent der Verbindung (A1) und 1 bis 1,5 Äquivalente, bevorzugt 1,1 Äquivalente einer Base, vorzugsweise Kaliumcarbonat, Kaliumhydrogncarbonat, Natriumcarbonat, Natriumhydrogencarbonat oder Calciumcarbonat, besonders bevorzugt Kaliumcarbonat, in einem Verdünnungsmittel gegebenfalls mit Wasser genüscht, beispielsweise Aceton, Tetrahydrofuran, Diethylether, Cyclohexan, Petrolether oder Dioxan, vorzugsweise Cyclohexan oder Diethylether, gerührt.

Bei einer Temperatur von 0 bis 15 °C, bevorzugt 5 bis 10 °C, wird 1 Äquivalent einer Aminosäure der Formel (A2) gelöst in einem organischen Lösungsmittel, beispielsweise Aceton, Tetrahydrofuran, Diethylether, Cyclohexan oder Dioxan, zugetropft. Das Reaktionsgemisch wird unter Rühren auf eine Temperatur von 18°C bis 30 °C, vorzugsweise etwa 22°C, erwärmt und anschließend weitere 10 bis 24 Stunden, vorzugsweise etwa 12 Stunden, weitergerührt. Danach wird das Verdünnungsmittel abdestilliert, der Rückstand mit Wasser versetzt und das Gemisch zwei bis dreimal eines organischen Lösungsmittels beispielsweise, Diethylether oder Ethylacetat, vorzugsweise Ethylacetat extrahiert. Die vereinigten organischen Extrakte werden getrocknet und das Lösungsmittel abdestilliert. Der Rückstand (Verbindung A3) kann ohne vorherige Aufreinigung in Stufe 2 eingesetzt werden.

### Stufe 2A

Die in Stufe 1A erhaltene Verbindung (A3) wird an der Nitrogruppe reduziert und zur Verbindung der Formel (A4) zyklisiert (Schema 2A).

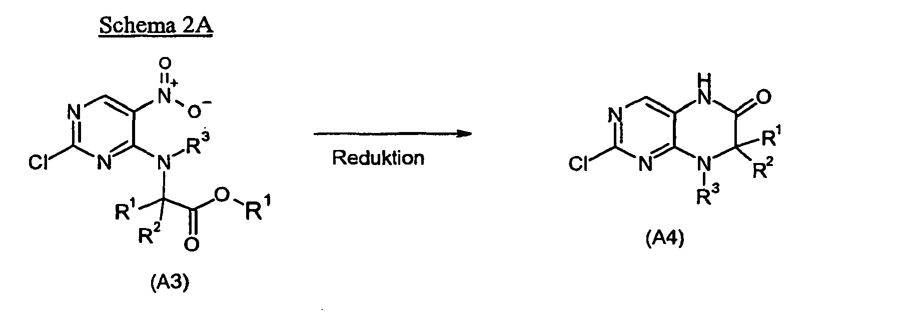

In Stufe 2A werden 1 Äquivalent der Nitroverbindung (A3) in einer Säure gelöst, vorzugsweise Eisessig, Ameisensäure oder wässrige Salzsäure, bevorzugt Eisessig, und auf 50 bis 70 °C, vorzugsweise etwa 60 °C, erwärmt. Anschließend wird ein Reduktionsnnittel, beispielsweise Zink, Zinn, oder Eisen, bevorzugt Eisenpulver, bis zum Abschluß der exothermen Reaktion hinzugefügt und 0,2 bis 2 Stunden, vorzugsweise 0,5 Stunden, bei 100 bis 125 °C, vorzugsweise bei etwa 117 °C, gerührt. Nach Abkühlung auf Raumtemperatur wird das Eisensalz abfiltriert und das Lösungsmittel abdestilliert. Der Rückstand wird in einem Lösungsmittel oder Lösungsmittelgemisch, beispielsweise Ethylacetat oder Dichlormethan/ Methanol 9/1 und halbgesättigte NaCl-Lösung, aufgenommen und beispielsweise über Kieselgur filtriert. Die organische Phase wird getrocknet und eingeengt. Der Rückstand (Verbindung (A4)) kann chromatographisch oder mittels Kristallisation gereinigt oder als Rohprodukt in Stufe 3A der Synthese eingesetzt werden.

### Stufe 3A

Die in Stufe 2A erhaltene Verbindung (A4) kann durch elektrophile Substitution gemäß Schema 3A zur Verbindung der Formel (A5) umgesetzt werden.

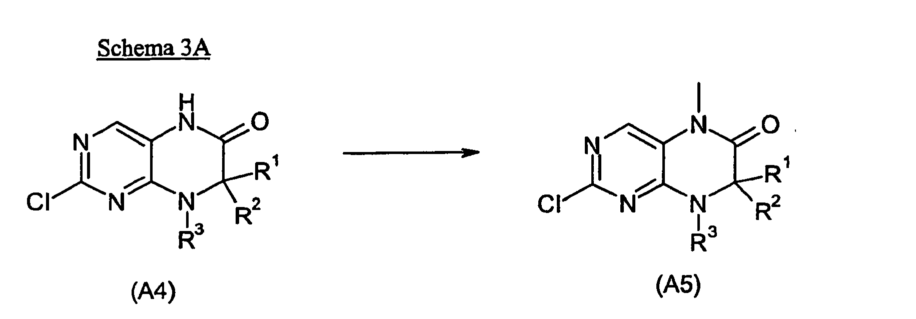

In Stufe 3A werden 1 Äquivalent des Amides der Formel (A4) in einem organischen Lösungsmittel, beispielsweise Dimethylformamid oder Dimethylacetamid, vorzugsweise Dimethylacetamid, gelöst und auf etwa -5 bis 5 °C, vorzugsweise 0°C, abgekühlt.

Anschließend wird 0,9 bis 1,3 Äquivalente Natriumhydrid und 0,9 bis 1,3 Äquivalente eines Methylierungsreagenzes, beispielsweise Methyliodid zugegeben. Das Reaktionsgemisch wird 0,1 - 3 Stunden, vorzugsweise etwa 1 Stunde, bei etwa 0 bis 10 °C, vorzugsweise bei etwa 5 °C, gerührt und kann gegebenenfalls weitere 12 Stunden bei diesem Temperaturbereich stehengelassen werden. Die Reaktionsmischung wird auf Eiswasser gegossen und der Niederschlag isoliert. Der Rückstand (Verbindung (A5)) kann chromatographisch, vorzugsweise über Kieselgel, oder mittels Kristallisation gereinigt oder als Rohprodukt in Stufe 4A der Synthese eingesetzt werden.

### Stufe 4A

Die Aminierung der in Stufe 3A erhaltenen Verbindung (A5) zur Verbindung der Formel (A9) (Schema 4A) kann nach den literaturbekannten Methoden der Varianten 4.1 A aus z.B. (a) M.P.V. Boarland, J.F.W. McOmie J. Chem. Soc. 1951, 1218-1221 oder (b) F. H. S. Curd, F. C. Rose J. Chem. Soc. 1946, 343-348, 4.2 A aus z.B. (a) Banks J. Am. Chem. Soc. 1944, 66, 1131, (b) Ghosh and Dolly J. Indian Chem. Soc. 1981, 58, 512-513 oder (c) N. P. Reddy and M. Tanaka Tetrahedron Lett. 1997, 38, 4807-4810, durchgeführt werden.

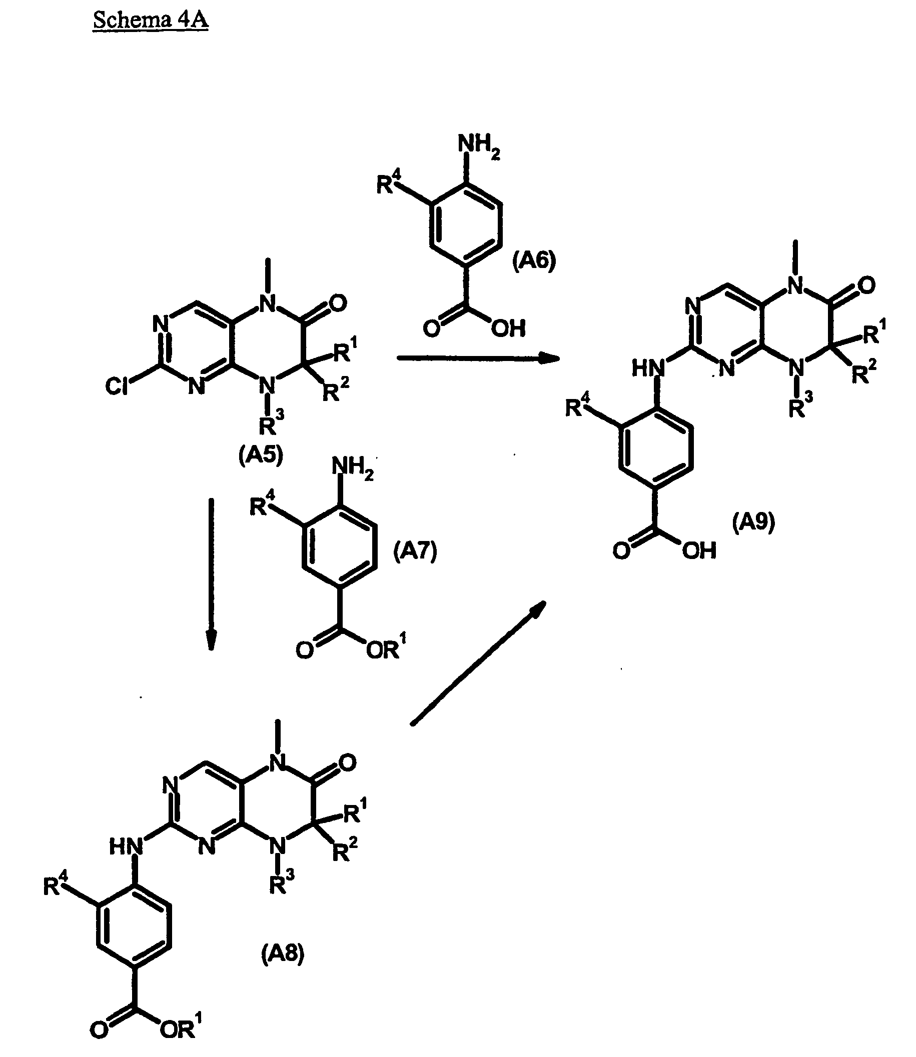

Beispielsweise werden in Variante 4.1 A 1 Äquivalent der Verbindung (A5) und 1 bis 3 Äquivalente, vorzugsweise etwa 2 Äquivalente der Verbindung (A6) ohne Lösungsmittel oder einem organischen Lösungsmittel wie beispielsweise Sulfolan, Dimethylformamid, Dimethylacetamid, Toluol, N-Methylpyrrolidon, Dimethylsulfoxid, oder Dioxan, bevorzugt Sulfolan über 0,1 bis 4 Stunden vorzugsweise 1 Stunde, bei 100 bis 220°C, vorzugsweise bei etwa 160 °C erhitzt. Nach dem Abkühlen wird durch Zugabe von org. Lösungsmitteln oder Lösungsmittelgemischen bespielsweise Diethylether/Methanol, Ethylacetat, Methylenchlorid, oder Diethylether, vorzugsweise Diethylether/Methanol 9/1, das Produkt (A9) kristallisiert oder chromatographisch gereinigt.

Beispielsweise wird in Variante 4.2 A 1 Äquivalent der Verbindung (A5) und 1 bis 3 Äquivalente der Verbindung (A6) mit Säure, beispielsweise 1-10 Äquivalente 10-38%ige Salzsäure und/oder einem Alkohol beispielsweise Ethanol, Propanol oder Butanol, bevorzugt Ethanol unter Rückfluss 1 bis 48 Stunden, vorzugsweise etwa 5 Stunden, gerührt.

Das ausgefallene Produkt (A9) wird abfiltriert und gegebenenfalls mit Wasser gewaschen, getrocknet und aus einem geeigneten org. Lösungsmittel kristallisiert.

Beispielsweise wird in Variante 4.3 A 1 Äquivalent der Verbindung (A5) und 1 bis 3 Äquivalente der Verbindung (A7), in einem Lösungsmittel, beispielsweise Toluol oder Dioxan gelöst und mit einem Phophinliganden, beispielsweise 2;2'-Bis-(diphenylphosphino)-1,1'-binaphthyl und einem Palladiumkatalysator, beispielsweise Tris(dibenzylidenaceton)-dipalladium(0) und einer Base, beispielsweise Cäsiumcarbonat versetzt und 1-24 Std., vorzugsweise 17 Std. unter Rückfluss gekocht. Die Reaktionsmischung wird beispielsweise über Kieselgel gereinigt und das Produkt (A8) aus der Lösung isoliert oder durch geeignete Kristallisation erhalten.

Das Produkt (A8) wird in einem geeigneten Lösungsmittel, beispielsweise Dioxan gelöst und mit Säure, beispielsweise halbkonzentrierter Salzsäure, beispielsweise im Verhältnis Lösungsmittel zu Säure 3: 1 zugegeben. Anschließend wird für 1 - 48 Std. unter Rückfluss erhitzt, beispielsweise 12 Std. und der ausgefallene Niederschlag isoliert. Gegebenfalls wird das Produkt (A9) durch Kristallisation gereinigt.

### Stufe 5A

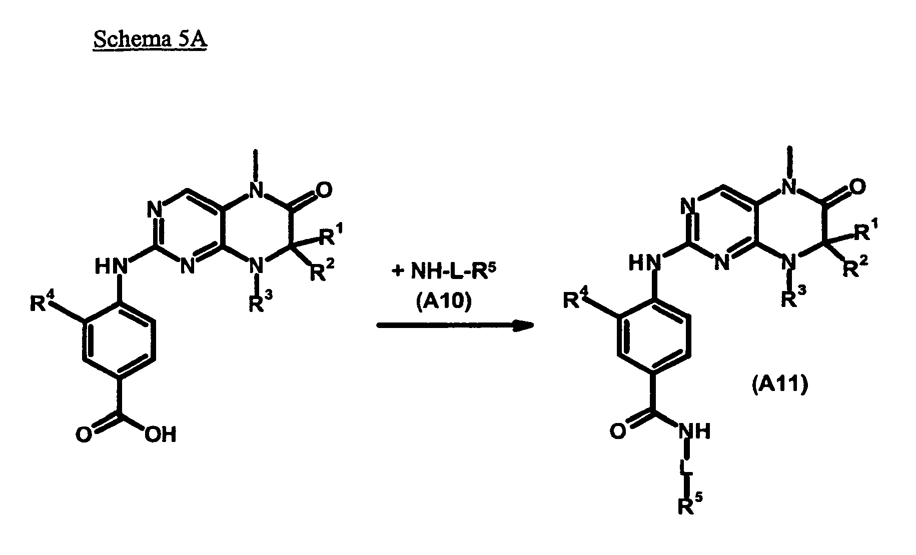

Beispielsweise werden 1 Äquivalent der Verbindung (A9) mit 1 Äquivalent eines Aktivierungsreagenzes, beispielesweise O-Benzotriazolyl-N,N,N',N'-tetramethyluroniumtetrafluoroborat (TBTU) und einer Base, beispielsweise etwa 1,5 Äquivalente, Diisopropylethylamin (DIPEA) in einem organischen Verdünnungsmittels, beispielsweise Dichlormethan, Tetrahydrofizran, Dimethylformamid, N-Methylpyrrolidon, Dimethylacetamid, vorzugsweise Dichlormethan oder Dimethylformamid, gelöst. Nach Zugabe von 1 Äquivalent des Amins (A10) wird das Reaktionsgemisch 0,1 bis 24 Stunden, vorzugsweise etwa 2 Stunden bei 20°C bis 100°C gerührt. Über beispielsweise Kristallisation oder chromatographische Reinigung wird das Produkt der Formel (A11) erhalten.

Die Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

Die Herstellung einiger zur Synthese der Beispiele eingesetzten Zwischenverbindungen wird ebenfalls im folgenden beschrieben.

### Herstellung der Säuren

Zur Synthese der Verbindungen Bsp. 94 und Bsp. 95 wird zunächst eine Zwischenverbindung Z1 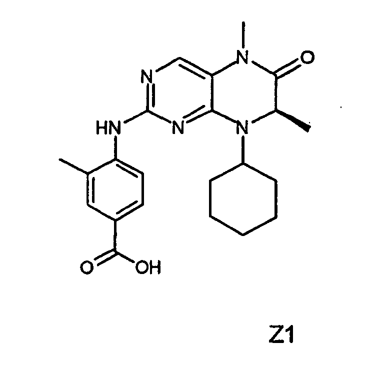 wie im folgenden beschrieben hergestellt.

50,0 g (0,48 Mol) D-Alaninmethylester x HCl und 49,1 g (0,50 Mol) Cyclohexanon werden in 300 mL Dichlormethan vorgelegt und anschließend mit 41,0 g (0,50 Mol) Natriumacetat und 159,0 g (0,75 Mol) Natriumtriacetoxyborhydrid versetzt. Es wird über Nacht gerührt und dann 300 mL 10%ige Natriumhydrogencarbonat-Lösung zugegeben. Die wässrige Phase wird mit Dichlormethan extrahiert. Die vereinten organischen Phasen werden mit 10%iger Natriumhydrogencarbonat-Lösung gewaschen, über Na₂SO₄ getrocknet und eingeengt. Ausbeute: 72,5 g einer Verbindung Z1a (klare Flüssigkeit)

72,5 g der Verbindung Z1a werden in 500 mL Wasser vorgelegt und 76,6 g (0,39 Mol) 2,4-Dichlor-5-nitropyrimidin in 500 mL Diethylether zugegeben. Bei einer Temperatur von-5°C werden 100 mL 10%ige Kaliumhydrogencarbonat-Lösung zugetropft. Es wird 3 Std. bei -5°C und weitere 12 Std. bei Raumtemperatur gerührt. Die organische Phase wird abgetrennt und über Na₂SO₄ getrocknet. Beim Einengen kristallisiert das Produkt aus.

Ausbeute: 48,0 g einer Verbindung Z1b (gelbe Kristalle)

48,0 g der Verbindung Z1b werden in 350 mL Eisessig gelöst und auf 60°C erwärmt. Es werden portionsweise 47,5 g Eisen-Pulver zugegeben, wobei die Temperatur auf 105°C steigt. Das Reaktionsgemisch wird drei Stunden bei 80°C gerührt, anschließend heiß über Cellulose filtriert und eingeengt. Der Rückstand wird in Wasser und Essigsäureethylester ausgerührt, abgesaugt und der hellgraue Niederschlag mit Essigsäureethylester gewaschen. Das Filtrat wird mit verdünntem Ammoniak und Wasser gewaschen, die organische Phase über Na₂SO₄ getrocknet, über Aktivkohle filtriert und eingeengt. Es wird weiterer hellgraue Feststoff erhalten.

Ausbeute: 29,5 g einer Verbindung Z1c (hellgraue Kristalle)

32,1 g der Verbindung Z1c werden in 300 mL Dimethylacetamid vorgelegt und mit 13 mL (0,2 Mol) Methyliodid versetzt. Bei -5°C werden portionsweise 6,4 g (0,16 Mol) Natriumhydrid als 60%ige Dispersion in Mineralöl zugegeben. Nach 2 Std. wird das Reaktionsgemisch auf 800 mL Eiswasser gegossen. Der ausgefallene Niederschla g wird abgesaugt und mit Petrolether gewaschen.

Ausbeute: 33,0 g einer Verbindung Z1d (beige Kristalle)

4,0 g der Verbindung Z1d und 2,3 g (15 mMol) 4-Amino-3-methylbenzoesäure werden in 50 mL Ethanol und 120 mL Wasser suspendiert, mit 2 mL konz. Salzsäure versetzt und 48 Std. unter Rückfluss gekocht. Der beim Abkühlen ausgefallene Niederschlag wird abgesaugt und mit Wasser, Ethanol und Diethylether gewaschen.

Ausbeute: 2,9 g einer Verbindung Z1 (farblose Kristalle)

Zur Synthese der Verbindungen Bsp. 188 und Bsp. 203 wird zunächst eine Zwischenverbindung Z2 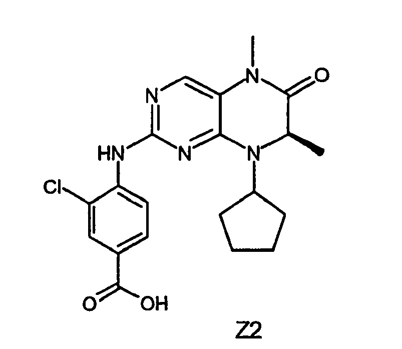 wie im folgenden beschrieben hergestellt.

Eine Lösung von128,2 g (0,83 Mol) D-Alaninethylester x HCl und 71,5 g (0,85 Mol) Cyclopentanon in 1500 mL Dichlormethan wird mit 70,1 (0,85 Mol) Natriumacetat und 265,6 g (1,25 Mol) Natriumtriacetoxyborhydrid versetzt. Die Reaktionsmischung wird 12 Std. gerührt und dann in 1,5 L einer 10%igen Natriumhydrogencarbonat-Lösung gegossen. Die wässrige Phase wird mit Dichlormethan extrahiert. Die vereinten organischen Phasen werden über Na₂SO₄ getrocknet und eingeengt.

Ausbeute: 143,4 g einer Verbindung Z2a (farbloses Öl)

66,0 g der Verbindung Z2a werden in 500 mL Wasser vorgelegt und mit 85,0 g (0,44 Mol) 2,4-Dichlor-5-nitropyrimidin in 500 mL Diethylether versetzt. Bei -5°C werden 100 mL 10%ige Kaliumhydrogencarbonat-Lösung zugetropft und die Reaktionsmischung 48 Std. bei Raumtemperatur gerührt. Die wässrige Phase wird mit Diethylether extrahiert, die vereinten organischen Phasen über Na₂SO₄ getrocknet und eingeengt. Der dunkelrote Feststoff wird mit Petrolether ausgerührt und abgesaugt. Ausbeute: 88,0 g einer Verbindung Z2b (gelbe Kristalle)

88,0 g der Verbindung Z2b werden in 1000 mL Eisessig gelöst und bei 60°C portionsweise mit 85 g Eisen-Pulver versetzt, wobei die Temperatur auf 110°C ansteigt. Es wird 1 Std. bei 60°C gerührt, anschließend heiß über Cellulose abgesaugt und eingeengt. Der braune Feststoff wird mit 700 mL Wasser ausgeführt und abgesaugt. Ausbeute: 53,3 g einer Verbindung Z2c (leicht braune Kristalle)

53,3 g der Verbindung Z2c werden in 300 mL Dimethylacetamid gelöst und mit 13 mL (0,21 Mol) Methyliodid versetzt. Bei -5°C werden 5,0 g (0,21 Mol) Natriumhydrid als 60%ige Dispersion in Mineralöl portionsweise zugegeben. Nach 12 Std. wird das Reaktionsgemisch auf 1000 mL Eiswasser gegossen und der entstandene Niederschlag abgesaugt.

Ausbeute: 40,0 g einer Verbindung Z2d (farblose Kristalle)

4,0 g der Verbindung Z2d und 2,8 g (16 mMol) 4-Amino-3-chlorbenzoesäure werden in 25 mL Ethanol und 60 mL Wasser suspendiert, mit 3 mL konz. Salzsäure versetzt und 43 Std. unter Rückfluss gekocht. Der beim Abkühlen ausgefallene Niederschlag wird abgesaugt und mit Wasser, Ethanol und Diethylether gewaschen.

Ausbeute: 0,9 g einer Verbindung Z2 (farblose Kristalle)

Zur Synthese der Verbindunge in den Referenzbeispielen, 19, 21, 22, 23, 45, 55, 58, 116, 128, 131, 133, 134, 136, 138, 177, 217, 231, 239, 46, 184, 166 und 187 wird zunächst eine Zwischenverbindung Z3 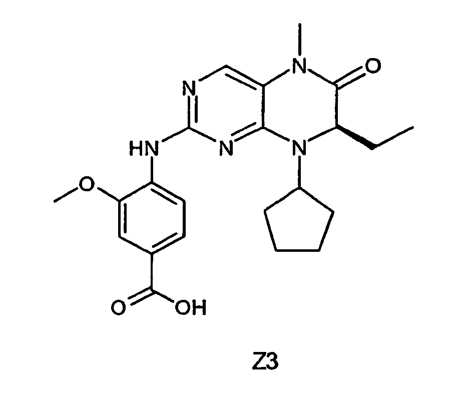 wie im folgenden beschrieben hergestellt.

54,0 g (0,52 Mol) D-2-Aminobuttersäure werden in 540 mL Methanol suspendiert und unter Eiskühlung langsam mit 132 g (1,1 Mol) Thionylchlorid versetzt. Es wird 1,5 Std. unter Rückfluß gekocht und anschließend eingeengt. Das verbliebene Öl wird mit 540 mL tert-Butylmethylether versetzt und die entstandenen farblosen Kristalle abgesaugt. Ausbeute: 78,8 g einer Verbindung Z3a (farblose Kristalle)

74,2 g der Verbindung Z3a und 43,5 mL (0,49 Mol) Cyclopentanon werden in 800 mL Dichlormethan ge löst. Nach Zugabe von 40,0 g (0,49 Mol) Natriumacetat und 150,0 g (0,71 Mol) Natriumtriacetoxyborhydrid bei 0°C wird 12 Std. bei Raumtemperatur gerührt und dann 500 mL 20%ige Natriumhydrogencarbonat-Lösung zugegeben. Die wässrige Phase wird mit Dichlormethan extrahiert. Die vereinten organischen Phasen werden mit Wasser gewaschen, über MgSO₄ getrocknet und eingeengt.

Ausbeute: 85,8 g einer Verbindung Z3b (leicht gelbes Öl)

40,0 g der Verbindung Z3b und 30,0 g (0,22 Mol) Kaliumcarbonat werden in 600 mL Aceton suspendiert und unter Eiskühlung mit 45,0 g (0,23 Mol) 2,4-Dichlor-5-nitropyrimidin in 200 mL Aceton versetzt. Nach 12 Std. werden weitere 5,0 g von 2,4-Dichlor-5-nitropyrimidin zugegeben und 3 Std. gerührt. Die Reaktionsmischung wird eingeengt, in 800 mL Essigsäureethylester und 600 mL Wasser aufgenommen und die wässrige Phase mit Essigsäureethylester extrahiert. Die vereinten organischen Phasen werden mit Wasser gewaschen, über MgSO₄ getrocknet und eingeengt.

Ausbeute: 75,0 g einer Verbindung Z3c (braunes Öl)

100 g der Verbindung Z3c werden in 650 mL Eisessig gelöst und bei 70°C mit 20 g Eisen-Pulver portionsweise versetzt. Es wirde 1 Std. bei 70°C, dann 1,5 Std. bei 100°C gerührt und anschließend heiß über Kieselgur abfiltriert. Die Reaktionsmischung wird eingeengt, in Methanol/Dichlormethan aufgenommen, auf Kieselgel aufgezogen und durch Soxhlet-Extraktion mit Essigsäureethylester gereinigt. Das Lösungsmittel wird entfernt und der Rückstand mit Methanol ausgerührt.

Ausbeute: 30,0 g einer Verbindung Z3d (hellbraune Kristalle)

25,0 g der Verbindung Z3d und 6,5 mL (0,1 Mol) Methyliodid werden in 250 mL Dimethylacetamid vorge legt und bei -10°C mit 3,8 g (0,95 Mol) Natriumhydrid als 60%ige Dispersion in Mineralöl versetzt. Es wird 20 Min. bei 0°C, dann 30 Min. bei Raumtemperatur gerührt und schließlich Eis zugegeben. Die Reaktionsmischung wird eingeengt und mit 300 mL Wasser versetzt. Der entstandene Niederschlag wird abgesaugt und mit Petrolether gewaschen.

Ausbeute: 23,0 g einer Verbindung Z3e (farbloser Feststoff)

6,0 g der Verbindung Z3e und 5,1 g (31 mMol) 4-Amino-3-methoxybenzoesäure werden in 90 mL Ethanol und 350 mL Wasser suspendiert, mit 3,5 mL konz. Salzsäure versetzt und 48 Std. unter Rückfluss gekocht. Die Reaktionsmischung wird eingeengt, der Rückstand mit Methanol/Diethylether verrührt und der entstandene Niederschlag abgesaugt.

Ausbeute: 6,3 g einer Verbindung Z3 (hellbeige Kristalle)

Zur Synthese der Verbindung in den Referenzbeispielen 81, 82, 93, 137 wird zunächst eine Zwischenverbindung Z4 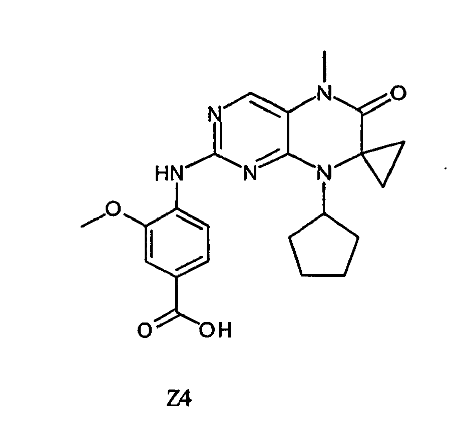 wie im folgenden beschrieben hergestellt.

25,0 g (0,19 Mol) 1-Aminocyclopropan-1-carbonsäureethylester x HCl und 16,8 g (0,20 Mol) Cyclopentanon werden in 300 mL Dichlormethan gelöst und mit 16,4 g (0,20 Mol) Natriumacetat und 61,7 g (0,29 Mol) Natriumtriacetoxyborhydrid versetzt. Es wird über Nacht gerührt und die Reaktionsmischung dann auf 400 mL 10%ige Natriumhydrogencarbonat Lösung gegossen. Die wässrige Phase wird mit Dichlormethan extrahiert. Die vereinten organischen Phasen werden über Na₂SO₄ getrocknet und eingeengt.

Ausbeute: 34,5 g einer Verbindung Z4a (farbloses Öl)

Zu einer Mischung von 34,5 g der Verbindung Z4a in 350 mL Wasser werden 42,5 g (0,22 Mol 2,4-Dichlor-5-nitropyrimidin in 350 mL Diethylether gegeben. Bei -5°C wird mit 80 mL 10%iger Kaliumhydrogencarbonat-Lösung versetzt und über Nacht bei Raumtemperatur gerührt. Die wässrige Phase wird mit Diethylether extrahiert. Die vereinten organischen Phasen werden über Na₂SO₄ getrocknet und eingeengt. Ausbeute: 53,8 g einer Verbindung Z4b (braunes Öl)

20,1 g der Verbindung Z4b werden in 200 mL Eisessig gelöst und bei 60°C mit 19,1 g Eisen-Pulver portionsweise versetzt, wobei die Temperatur auf 100°C ansteigt Es wird 3 Std. bei 60°C gerührt, anschließend über Cellulose abgesaugt und eingeengt. Der Rückstand wird in Wasser und Essigsäureethylester ausgerührt und der gelbe Niederschlag abgesaugt. Das Filtrat wird mit verdünntem Ammoniak und Wasser gewaschen, die organische Phase über Na₂SO₄ getrocknet und eingeengt. Nach Zugabe von Diethylether kristallisiert das Produkt.

Ausbeute: 4,0 g einer Verbindung Z4c (gelbe Kristalle)

7,8 g der Verbindung Z4c und 2,6 mL (0,04 Mol) Methyliodid werden in 100 mL Dimethylacetamid gelöst und bei -5°C mit 1,5 g (0,04 Mol) Natriumhydrid als 60%ige Dispersion in Mineralöl portionsweise versetzt. Nach 2 Std. wird das Reaktionsgemisch auf Eiswasser gegossen und der entstandene Niederschlag abgesaugt.

Ausbeute: 7,5 g einer Verbindung Z4d (leicht braune Kristalle)

3,0 g der Verbindung Z4d und 1,9 g (11 mMol) 4-Amino-3-methoxybenzoesäure werden in 40 mL Ethanol und 80 mL Wasser suspendiert, mit 2 mL konz. Salzsäure versetzt und 20 Std. unter Rückfluss gekocht. Es werden weitere 0,5 g 4-Amino-3-methoxybenzoesäure zugegeben und 48 Std. unter Rückfluss gekocht. Der beim Abkühlen ausgefallene Niederschlag wird abgesaugt und mit Wasser, Ethanol und Diethylether gewaschen.

Ausbeute: 2,1 g einer Verbindung Z4 (farblose Kristalle)

Zur Synthese der Verbindungen in den referenzbeispielen 162, 43, 53, 161, 202, 211, 215 und 212 wird zunächst eine Zwischenverbindung Z5 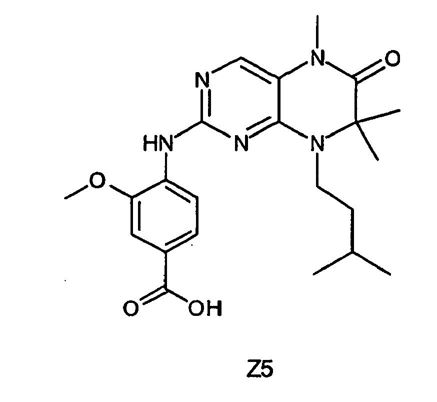 wie im folgenden beschrieben hergestellt.

Eine Mischung aus 73,4 mL (0,5 Mol 2-Bromisobuttersäureethylester, 87,1 mL (0,75 Mol) 3-Methyl-1-butylamin, 82,5 g (0,6 Mol) Natriumiodid und 76,0 g (0,6 Mol) Kaliumcarbonat in 1000 mL Essigsäureethylester wird 3 Tage unter Rückfluss gekocht. Vorhandene Salze werden abfiltriert und das Filtrat eingeengt.

Ausbeute: 97,0 g einer Verbindung Z5a (rotes Öl)

49,0 g (0,25 Mol) 2,4-Dichlor-5-nitropyrimidin und 38,3 g (0,28 Mol) Kaliumcarbonat werden in 500 mL Aceton suspendiert und bei 0°C mit 93,0 g der Verbindung Z5a in 375 mL Aceton versetzt. Die Reaktionsmischung wird über Nacht bei Raumtemperatur gerührt, filtriert und eingeengt. Der in Essigsäureethylester gelöste Rückstand wird mit Wasser gewaschen und die organische Phase über MgSO₄ getrocknet und eingeengt. Ausbeute: 102,7 g einer Verbindung Z5b (braunes Öl)

22,7 g der Verbindung Z5b werden in 350 mL Eisessig gelöst und bei 60°C mit 17,4 g Eisen-Pulver portionsweise versetzt. Nach beendeter Zugabe wird 0,5 Std. unter Rückfluss gekocht, heiß abfiltriert und eingeengt. Der Rückstand wird in 200 mL

Dichlormethan/Methanol (9:1) aufgenommen und mit Natriumchlorid-Lösung gewaschen. Die organische Phase wird über Kieselgur abgesaugt, über MgSO₄ getrocknet, eingeengt und mittels Säulenchromatographie (Laufmittel: Essigsäureethylester/Cyclohexan 1:1) gereinigt.

Ausbeute: 1,9 g einer Verbindung Z5c (farblose Kristalle)

1,9 g der Verbindung Z5c werden in 32 mL Dimethylacetamid gelöst und unter Eiskühlung mit 0,3 g (7 mMol) Natriumhydrid als 60%ige Dispersion in Mineralöl versetzt. Nach 10 Min. werden 0,5 mL (7 mMol) Methyliodid zugegeben und 3 Std. bei Raumtemperatur gerührt. Die Reaktionsmischung wird eingeengt und mit Wasser versetzt. Der entstandene Niederschlag wird abgesaugt und mit Petrolether gewaschen. Ausbeute: 1,6 g einer Verbindung Z5d (farblose Kristalle)

14,0 g der Verbindung Z5d und 10,0 g (0,06 Mol) 4-Amino-3-methoxybenzoesäure werden in 200 mL Dioxan und 80 mL Wasser suspendiert, mit 10 mL konz. Salzsäure versetzt und 40 Std. unter Rückfluss gekocht. Der beim Abkühlen ausgefallene Niederschlag wird abgesaugt und mit Wasser, Dioxan und Diethylether gewaschen.

Ausbeute: 13,9 g einer Verbindung Z5 (farblose Kristalle)

Zur Synthese der Verbindungen in den Referenzbeispielen 88, 194, 229 und 89 wird zunächst eine Zwischenverbindung Z6 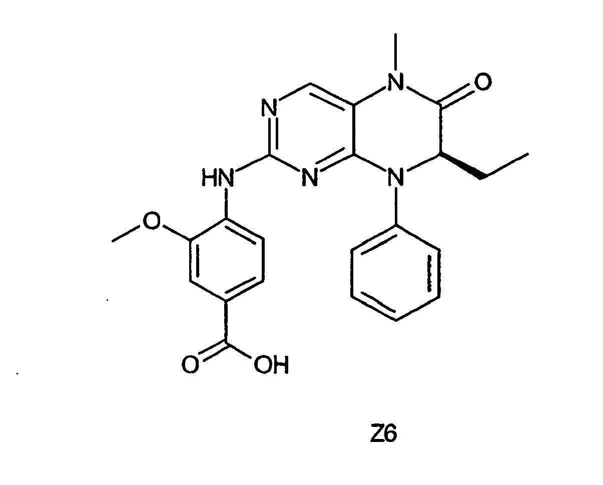 wie im folgenden beschrieben hergestellt.

6,0 g (0,06 Mol) L-2-Aminobuttersäure wird in 80 mL 0,5 M Schwefelsäure vorgelegt und bei 0°C mit 5,5 g (0,08 Mol) Natriumnitrit in 15 mL Wasser versetzt. Die Reaktionsmischung wird 22 Std. bei 0°C gerührt, mit Ammoniumsulfat versetzt und filtriert. Das Filtrat wird mit Diethylether extrahiert und die vereinten organischen über MgSO₄ getrocknet und eingeengt.

Ausbeute: 6,0 g einer Verbindung Z6a (gelbes Öl)

200 mL Methanol werden unter Eiskühlung nacheinander mit 65,0 mL (0,89 Mol) Thionylchlorid und 76,0 g der Verbindung Z6a in 50 mL Methanol versetzt. Es wird 1 Std. bei 0°C und 2 Std. bei Raumtemperatur gerührt und dann das Methanol und restliches Thionylchlorid bei 0°C im Vakuum entfernt.

Ausbeute: 40,0 g einer Verbindung Z6b (gelbes Öl)

30,0 mL (0,17 Mol) Trifluormethansulfonsäureanhydrid werden in 150 mL Dichlormethan vorgelegt und unter Eiskühlung innerhalb einer Stunde mit einer Lösung von 20,0 g der Verbindung Z6b und 14,0 mL (0,17 Mol) Pyridin in 50 mL Dichlormethan versetzt. Es wird 2 Std. bei Raumtemperatur gerührt, entstandene Salze abgesaugt und dann mit 100 mL Wasser gewaschen. Die organische Phase wird über MgSO₄ getrocknet und eingeengt.

Ausbeute: 42,0 g einer Verbindung Z6c (hellgelbes Öl)

Zu einer Lösung von 15,5 mL (0,17 Mol) Anilin und 24,0 mL (0,17 Mol) Triethylamin in 400 mL Dichlorme than wird unter Eiskühlung innerhalb einer Stunde 42,0 g der Verbindung Z6c in 200 mL Dichlormethan getropft. Es wird 1 Std. bei Raumtemperatur und weitere 2 Std. bei 35°C gerührt. Die Reaktionsmischung wird mit Wasser gewaschen, über MgSO₄ getrocknet und eingeengt. Der verbliebene Rückstand wird durch Destillation (95-100°C, 1*10⁻³ mbar) gereinigt.

Ausbeute: 14,0 einer Verbindung Z6d (farbloses Öl)

14,0 g der Verbindung Z6d und 16,0 g (0,1 Mol) Kaliumcarbonat werden in 100 mL Aceton suspendiert und bei 10°C mit 16,0 g (0,08 Mol) 2,4-Dichlor-5-nitropyrimidin versetzt. Es wird 4 Std. bei 40°C gerührt, entstandene Salze abgesaugt und das Filtrat eingeengt. Der Rückstand wird in 300 mL Essigsäureethylester aufgenommen und mit Wasser gewaschen. Die organische Phase wird über MgSO₄ getrocknet und eingeengt. Ausbeute: 31,0 g einer Verbindung Z6e (braunes Öl)

31,0 g der Verbindung Z6e werden in 200 mL Eisessig gelöst und bei 60°C mit 10 g Eisen-Pulver portionsweise versetzt, wobei die Temperatur auf 85°C ansteigt. Es wird eine weitere Stunde bei 60°C gerührt, über Kieselgur filtriert und eingeengt. Der Rückstand wird mit Methanol ausgeführt.

Ausbeute: 4,5 g einer Verbindung Z6f (braune Kristalle)

Zu einer Mischung von 4,5 g der Verbindung Z6f und 1,0 mL (16 mMol ) Methyliodid in 100 mL Dimethylacetamid werdenbei -20°C 0,6 g (16 mMol) Natriumhydrid als 60%ige Dispersion in Mineralöl portionsweise gegeben. Nach 1 Std. wird das Reaktionsgemisch mit 50 mL Wasser versetzt und eingeengt. Der Rückstand wird mit 200 mL Wasser verrührt, der Niederschlag abgesaugt und mit Petrolether gewaschen.

Ausbeute: 4,5 g einer Verbindung Z6g (farblose Kristalle)

Eine Suspension von 1,5 g der Verbindung Z6g und 1,4 g (8 mMol) 4-Amino-3-methoxybenzoesäuremethylester in 30 mL Toluol wird mit 0,4 g (0,6 mMol) 2,2'-Bis-(diphenylphosphino)-1,1'-binaphthyl, 0,23 g (0,3 mMol) Tris(dibenzylidenaceton)-dipalladium(0) und 7,0 g (21 mMol) Cäsiumcarbonat versetzt und 17 Std. unter Rückfluss gekocht. Die Reaktionsmischung wird auf Kieselgel aufgezogen und mittels Säulenchromatographie (Laufmittel: Dichlormethan/Methanol 9: 1) gereinigt. Ausbeute: 1,7 g einer Verbindung Z6h (gelbe Kristalle)

1,7 g der Verbindung Z6h werden in 50 mL Dioxan gelöst, mit 15 mL halbkonz. Salzsäure versetzt und 12 Std. unter Rückfluss gekocht. Nach dem Abkühlen wird der entstandene Niederschlag abgesaugt.

Ausbeute: 1,1 g einer Verbindung Z6 (farbloser Feststoff)

Zur Synthese der Verbindung in den referenzbeispielen 26, 20,32 56, 101, 112, 209 wird zunächst eine Zwischenverbindung Z7 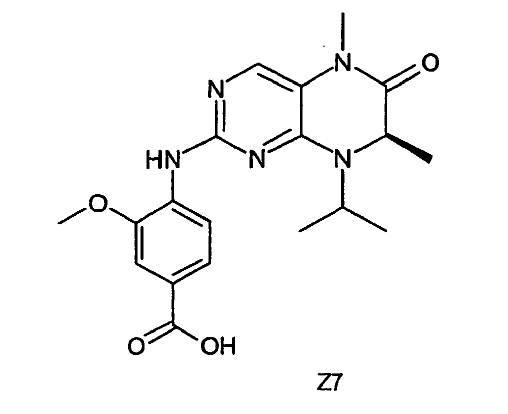 wie im folgenden beschrieben hergestellt.

50,0 g (0,36 Mol) D-Alaninmethylester x HCl wird in 500 mL Dichlormethan und 35 mL Aceton suspendiert und mit 30,0 g (0,37 Mol) Natriumacetat und 80,0 g (0,38 Mol) Natriumtriacetoxyborhydrid versetzt. Es wird 12 Std. gerührt und anschließend auf 400 mL 10%ige Natriumhydrogencarbonat-Lösung gegossen. Die organische Phase wird über Na₂SO₄ getrocknet, und eingeengt.

Ausbeute: 51,0 g einer Verbindung Z7a (gelbes Öl)

Eine Suspension von 51,0 g der Verbindung Z7a in 450 mL Wasser wird mit 80,0 g (0,41 Mol) 2,4-Dichlor-5-nitropyridin in 450 mL Diethylether versetzt. Bei -5°C werden 100 mL 10%ige Kaliumhydrogencarbonat-Lösung zugetropft. Das Reaktionsgemisch wird 3 Std. gerührt, die organische Phase über Na₂SO₄ getrocknet und eingeengt.

Ausbeute: 74 g einer Verbindung Z7b (gelbes Öl)

18,6 g der Verbindung Z7b werden in 200 mL Eisessig gelöst und bei 60°C mit 20,0 g Eisen-Pulver portionsweise versetzt. Es wird 2 Std. bei 60°C gerührt und dann über

aus Cellulose abgesaugt. Der Rückstand wird in Essigsäureethylester gelöst und mit Wasser und konz. Ammoniak gewaschen. Die organische Phase wird über Na₂SO₄ getrocknet und eingeengt. Der Rückstand wird aus Diethylether kristallisiert.

Ausbeute: 9,8 g einer Verbindung Z7c (farblose Kristalle)

17,0 g der Verbindung Z7c und 7 mL (0,1 Mol) Methyliodid werden in 200 mL Dimethylacetamid gelöst und bei -5°C mit 4,0 g (0,1 Mol) Natriumhydrid als 60%ige Dispersion in Mineralöl versetzt. Die Reaktionsmischung wird 30 Min. gerührt und dann auf 300 mL Eiswasser gegossen. Der entstandene Niederschlag wird abgesaugt und mit Petrolether ausgerührt.

Ausbeute: 14,8 g einer Verbindung Z7d (beige Kristalle)

0,9 g der Verbindung Z7d und 1,5 g (9 mMol) 4-Anzino-3-methoxybenzoesäure werden 30 Min. auf 210°C erhitzt. Nach dem Abkühlen wird der Rückstand mit Essigsäureethylester ausgerührt und der erhaltene Niederschlag abgesaugt.

Ausbeute: 1,2 g einer Verbindung Z7 (graue Kristalle)

Analog zu den beschriebenen Synthesen werden beispielsweise folgende Säuren hergestellt: 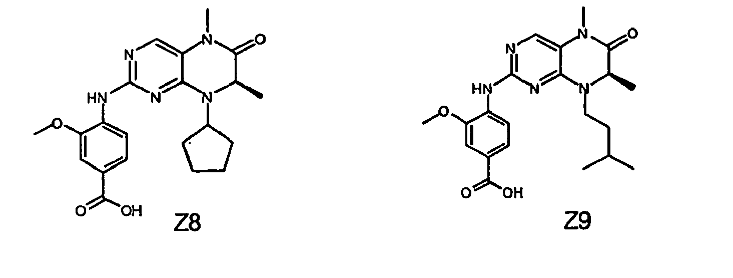 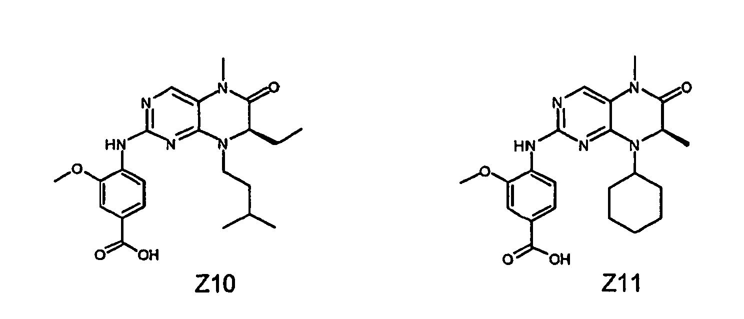 aus Synthese der Aminbausteine L-R5

Die folgenden Amine werden wie folgt erhalten.

1,1-Dimethyl-2-dipmethylamino-1-yl-ethylamin und 1,1-Dimethyl-2-piperidin-1-yl-ethylamin

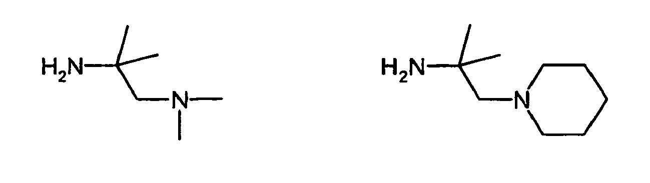

Die Verbindungen werden hergestellt nach folgenden Literaturstellen: (a) S. Schuetz et al. Arzneimittel-Forschung 1971, 21, 739-763, (b) V. M. Belikov et al.Tetrahedron 1970, 26, 1199-1216 und (c) E.B. Butler and McMillan J Amer. Chem. Soc. 1950, 72, 2978.

Weitere Amine werden in zu der oben beschriebenen Literatur modifizierter Art und Weise wie folgt hergestellt.

### 1,1-Dimethyl-2-morpholin-1-yl-ethylamin

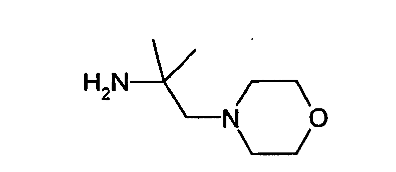

8,7 mL Morpholin und 9,3 mL 2-Nitropropan werden unter Eiskühlung vorgelegt, 7,5 mL Formaldehyd (37%) und 4 mL einer 0,5 Mol/L NaOH-Lsg. werden langsam zugetropft (<10°C). Danach wird 1h bei 25°C und 1h bei 50°C gerührt. Die Lösung wird mit Wasser und Ether behandelt und die wäßrige Phase 3x mit Ether extrahiert. Die vereinte org. Phase wird über NaSO₄ getrocknet und mit HCl in Dioxan (4Mol/l) versetzt, der entstandene Niederschlag wird abgesaugt. Ausbeute: 21,7 g weißes Pulver

aus 5 g des weißen Pulvers werden in 80 mL Methanol gelöst und unter Zusatz von 2 g RaNi mit Wasserstoff bei 35°C und 50 psi für 40 Minuten behandelt. Dies ergab 3,6 g 1.1- Dimethyl-2-morpholin-1-yl-ethylamin.

Analog zu dieser Vorschrift werden die folgenden Amine hergestellt.

### 1,1- Dimethyl-N-methylpiperazin-1-yl-ethylamin

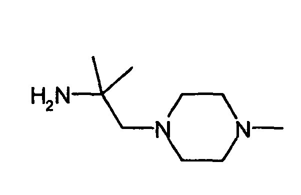

### 1,1-Dimethyl-2--pyffolidin-1-yl-ethylamin

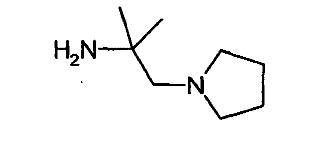

### Synthese von 1,3 Dimoryholin-2-aminopropan

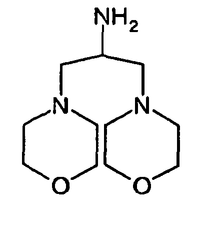

aus 5 g 1,3 Dimorpholin-2-nitropropan der Fa. Aldrich wird in 80 mL Methanol gelöst und unter Zusatz von 2 g RaNi mit Wasserstoff bei 30°C und 50 psi für 5,5 Std. behandelt. Dies ergab 4,2 g 1,3 Dimorpholin-2-amino-propan.

### 4-Aminobenzylmorpholin

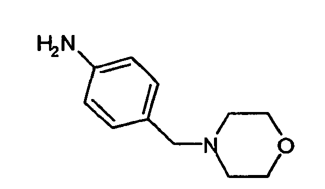

Die Herstellung dieses Amines ist in folgender Literaturstelle beschrieben: S. Mitsuru et al. J. Med. Chem. 2000, 43, 2049-2063

### 4-Amino-1-tetrahydro-4H-pyran-4-yl-piperidin

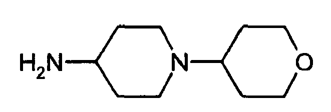

20 g (100 mMol)) 4-tert-Butyloxycarbonyl-aminopiperidin werden in 250 mL CH₂Cl₂ gelöst und mit 10 g (100 mMol) Tetrahydro-4H-pyran-4-on und 42 g (200 mMol) NaBH(OAc)₃ 12 Std. bei RT gerührt. Anschließend wird mit Wasser und Kaliumcarbonat versetzt, die org. Phase abgetrennt, getrocknet und im Vakuum das Lösungsmittel abgezogen. Der Rückstand wird in 200 mL CH₂Cl₂ gelöst und mit 100 mL Trifluoressigsäure 12 Std. bei RT gerührt. Das Lösungsmittel wird im Vakuum abgezogen, der Rückstand mit CHCl₃ aufgenommen und erneut eingedampft, anschließend in Aceton aufgenommen und mit etherischer HCl das Hydrochlorid gefällt. Ausbeute: 14,3 g (56%).

cis- und trans-4-Moroholino-cvclohexylamin

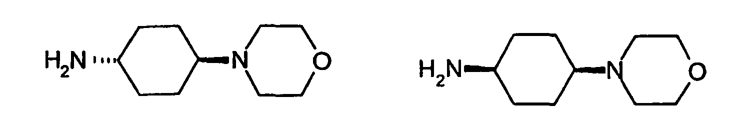

### Dibenzyl-4-moroholino-cyclohexvlamin

3,9 g (30 mMol)) 4-Dibenzylcyclohexanon werden in 100 mL CH₂Cl₂ gelöst und mit 3,9 g (45 mMol) Morpholin und 9,5 g (45 mMol) NaBH(OAc)₃ 12 Std. bei RT gerührt. Anschließend wird mit Wasser und Kaliumcarbonat versetzt, die org. Phase abgetrennt, getrocknet und im Vakuum das Lösungsmittel abgezogen. Der Rückstand wird über eine Kieselgelsäule gereinigt ( ca 20 mL Kieselgel; ca 500 mL Essigester 90/ Methanol 10+1% konz. Ammoniak). Die geeigneten Fraktionen werden im Vakuum eingeengt. Ausbeute 6,6 g (60%) cis-Isomer und 2 g (18%) trans-Isomer.

Alternativ kann das trans-Dibenzyl-4-morpholino-cyclohexylamin nach folgendem Weg dargestellt werden:
33 g (112 mMol) 4-Dibenzylcyclohexanon werden in 300 mL MeOH gelöst, mit 17,4 g (250 mMol) Hydroxylaminhydrochlorid versetzt und 4 Std. bei 60°C gerührt. Das Lösungsmittel wird im Vakuum eingeengt, mit 500 mL Wasser und 50 g Kaliumcarbonat versetzt und zweimal mit je 300 mL Dichlormethan extrahiert. Die org. Phase wird getrocknet, im Vakuum eingeengt, der Rückstand aus Petrolether kristallisiert, in 1,5 L EtOH gelöst und auf 70°C erwärmt. Es werden 166 g Natrium portionsweise hinzugefügt und bis zur Auflösung des Natriums unter Rückfluß gekocht. Das Lösungsmittel wird im Vakuum abgezogen, der Rückstand mit 100 mL Wasser versetzt und zweimal mit je 400 mL Ether extrahiert. Die org. Phase wird mit Wasser gewaschen, getrocknet, im Vakuum eingeengt und über eine Säule das trans-Isomere isoliert ( ca .1,5 L Kieselgel; ca. 2 L Essigester 80/ Methanol 20 + 2 % konz. Ammoniak). Ausbeute: 12,6 g (41,2%).
6,8 g (23 mMol) trans-1-Amino-4-Dibenzylaminocyclohexan wird in 90 mL DMF gelöst und mit 5 mL (42 mMol) 2,2'-Dichlorethylether und 5 g Kaliumcarbonat 8 Std. bei 100°C gerührt. Nach Abkühlung wird mit mit 30 mL Wasser versetzt, ausgefallene

Kristalle abgesaugt und über eine kurze Säule (ca. 20 mL Kieselgel, ca. 100 mL Essigester) gereinigt. Der Rückstand wird aus Methanol und konz. HCl als Dihydrochlorid kristallisiert. Ausbeute: 7,3 g (72,4%).

### trans-4-Morpholino-cyclohexvlamin

7,2 g (16,4 mMol) trans-Dibenzyl-4-morpholino-cyclohexylamin werden in 100 mL MeOH gelöst und an 1,4 g Pd/C (10%) bei 30-50 °C hydriert. Das Lösungsmittel wird im Vakuum abgezogen und der Rückstand aus Ethanol und konz. HCl kristallisiert. Ausbeute: 3,9 g (93%).

Das cis-Isomer kann analog dargestellt werden.

### cis- und trans-4-Piperidino-cyclohexylamin

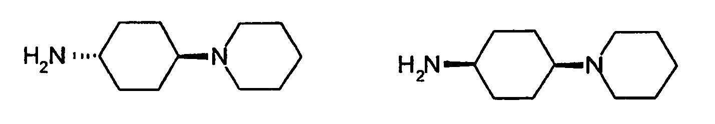

### trans-Dibenayl-4-piperidino-cyclohexylamin

2,0 g (6,8 mMol) trans-1Amino-4-Dibenzylaminocyclohexan (s. Bsp. 2) wird in 50 mL DMF gelöst und mit 1,6 g (7 mMol) 1,5-Dibrompentan und 2 g Kaliumcarbonat 48 Std. bei RT gerührt. Es wird abgekühlt, mit Wasser versetzt, zweimal mit je 100 mL Dichlormethan extrahiert, getrocknet und das Lösungsmittel im Vakuum abgezogen. Der Rückstand wird über eine Säule gereinigt ( ca. 100 mL Kieselgel, ca. 500 mL Essigester 80/Methanol 20 + 1% konz. Ammoniak). Die geeignete Fraktionen werden im Vakuum eingeengt und aus Petrolether kristallisiert. Ausbeute: 1,2 g (49%).

### trans-4-Piperidino-cyclohexylamin

1,7 g (4,8 mMol) trans-Dibenzyl-4-piperidino-cyclohexylamin werden in 35 mL MeOH gelöst und an 350 mg Pd/C (10%) bei 20 °C hydriert. Das Lösungsmittel wird im

Vakuum abgezogen und der Rückstand aus Ethanol und konz. HCl kristallisiert. Ausbeute: 1,1 g (78%).

Das cis-Isomer kann analog dargestellt werden.

### cis- und trans-4-(4-Phenyl-piperazin-1-yl)-cyclohexylamine

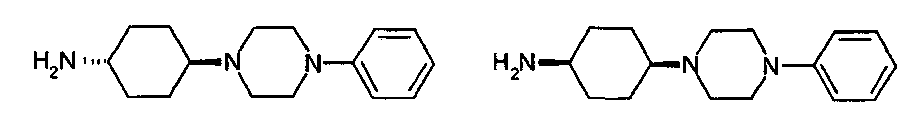

4,1 g (25,3 mMol) 4-Dibenzylcyclohexanon wird in 50 mL Dichlormethan gelöst und mit 7,4 g (25,3 mMol) N-Phenylpyperazin und 7,4 g (35 mMol) NaBH(OAc)₃ 12 Std. bei RT gerührt. Anschließend wird mit Wasser und Kaliumcarbonat versetzt, die org. Phase abgetrennt, getrocknet und das Lösungsmittel im Vakuum abgezogen. Der Rückstand wird über Kieselgelsäule gereinigt (Essigester 80/ Methanol 20 + 0,5% konz. Ammoniak). Ausbeute: 1,7 g (15,8%) cis-Isomer und 0,27 (2,5%) trans-Isomer.

### trans-4-(4-Phenyl-piperazin-1-yl)-cyclohexylamine

270 mg (0,61 mMol) trans-Dibenzyl-[4-(4-phenyl-piperazin-1-yl)-cyclohexyl]-amine werden in 5 mL MeOH gelöst und an 40 mg Pd/C (10%) bei 20-30 °C hydriert. Das Lösungsmittel wird im Vakuum abgezogen und der Rückstand aus Ethanol und konz. HCl kristallisiert. Ausbeute: 110 mg (69%).

Das cis-Isomer kann analog dargestellt werden.

### cis- und trans-4-(4-Cyclopropylmethyl-piperazin-1-yl-cycohexylamine

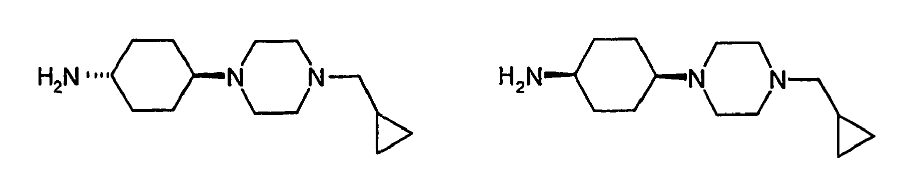

9,8 g (33,4 mMol) 4-Dibenzylcyclohexanon wird in 100 mL Dichlormethan gelöst und mit 5,6 g (40 mMol) N-Cyclopropylmethylpyperazin und 8,5 g (40 mMol) NaBH(OAc)₃ 12 Std. bei RT gerührt. Anschließend wird mit Wasser und Kaliumcarbonat versetzt, die org. Phase abgetrennt, getrocknet und das Lösungsmittel im Vakuum abgezogen. Der Rückstand wird über Kieselgelsäule gereinigt (ca. 50 mL Kieselgel, ca. 3 L Essigester 95/ Methanol 5 + 0,25% konz. Ammoniak). Die geeigneten Fraktionen werden im Vakuum eingeengt. Die schneller eluierende cis-Verbindung kristallisiert aus Essigester. Die trans-Verbindung wird aus Ethanol + konz. HCl kristallisiert. Ausbeute: 8,5 g (61%) cis-Isomer und 2,2 g (13%) trans-Isomer.

### cis-4-(4-Cyclopropylmethyl-Piperazin-1-yl)-cyclohexylamine

8,5 g (20 mMol) cis-Dibenzyl-[4-(4-cyclopropylmethyl-piperazin-1-yl)-cyclohexyl]-amine werden in 170 mL MeOH gelöst und an 1,7 g Pd/C (10%) bei 30-50 °C hydriert. Das Lösungsmittel wird im Vakuum abgezogen und der Rückstand aus Ethanol und konz. HCl kristallisiert. Ausbeute: 4,4 g (91%).

Das trans-Isomer kann analog dargestellt werden.

### Synthese der Verbindengen gemäβ Anspruch 1 und von Referenz-Beispielen

### Beispiel 152

0,15g der Verbindung Z10, 0,14 g TBTU, 0,13 mL DIPEA werden in Dichlormethan gelöst und 20 Minuten bei 25°C gerührt. Dann wird 90 µL 1-(3-Aminopropyl)-4-Methylpiperazin zugegeben und für weitere 2 Stunden Bei 25°C gerührt. Die Lösung wird dann mit Dichlormethan verdünnt und mit Wasser extrahiert. Durch Zugabe von Petrolether, Ether und Etyhlacetat zur organischen Phase wird das Produkt gefällt. Ausbeute: 0,16 g beiger Feststoff

### Beispiel 164

0,10g der Verbindung Z10, 0,1 g TBTU, 0,08 mL DIPEA werden in 4 mL Dichlormethan gelöst und 20 Minuten bei 25°C gerührt. Dann werden 44 µL Dimethylaminopropylamin zugegeben und für weitere 2 Stunden Bei 25°C gerührt. Die Lösung wird dann mit Dichlormethan verdünnt und mit Wasser extrahiert. Durch Zugabe von Petrolether, Ether und Aceton zur organischen Phase wird das Produkt gefällt. Ausbeute: 0,08 g gelber Feststoff.

### Beispiel 242

0,15g der Verbindung Z10, 0,14 g TBTU, 0,13 mL DIPEA werden in 5 mL Dichlormethan gelöst und 20 Minuten bei 25°C gerührt. Dann werden 75 µL 1-(2-Aminoethyl)-piperidin zugegeben und für weitere 2 Stunden Bei 25°C gerührt. Die Lösung wird dann mit Dichlormethan verdünnt und mit Wasser extrahiert. Durch Zugabe von Petrolether, Ether und Ethylacetat zur organischen Phase wird das Produkt gefällt. Ausbeute: 0,14 g gelber Feststoff.

### Beispiel 188

0,1 g der Verbindung Z2, 0,09 g TBTU, 0,05 mL DIPEA werden in 15 mL Dichlormethan gelöst und 20 Minuten bei 25°C gerührt. Dann werden 33 mg 1-Methyl-4-aminopiperidin zugegeben und für weitere 3 Stunden Bei 25°C gerührt. Die Lösung wird mit 20 mL Wasser extrahiert, dann im Vakkum eingeengt. Mit Hilfe von Ether wird das Produkt kristallisiert. Ausbeute: 0,047 g weiße Kristalle.

### Beispiel 203

0,1 g der Verbindung Z2, 0,09 g TBTU, 0,5 mL DIPEA werden in 15 mL Dichlormethan gelöst und 30 Minuten bei 25°C gerührt. Dann werden 50 mg 4-Amino-1-benzylpiperidin zugegeben und für weitere 3 Stunden Bei 25°C gerührt. Die Lösung wird mit 20 mL Wasser extrahiert, dann im Vakkum eingeengt. Anschließend wird über Kieselgel chromatographiert und das isolierte Produkt mit Hilfe von Ether kristallisiert. Ausbeute: 0,015 g weiße Kristalle.

### Beispiel 94

0,17 g der Verbindung Z1, 0,19 g TBTU, 0,11 mL DIPEA werden in 50 mL Dichlormethan gelöst und 30 Minuten bei 25°C gerührt. Dann werden 63 mg 1-Methyl-4-aminopiperidin zugegeben und für weitere 17 Stunden Bei 25°C gerührt. Zur Lösung werden 50 mL Wasser und 1 g Kaliumcarbonat gegeben und die organische Phase mittels Phasentrennkartusche abgetrennt, dann im Vakkum eingeengt. Anschließend wird das Produkt durch Kieselgelchromatograhie gereinigt und das gereinigte Produkt mit Hilfe von Ether kristallisiert. Ausbeute: 0,1 g weiße Kristalle.

### Beispiel 95

0,17 g der Verbindung Z1, 0,19 g TBTU, 0,11 mL DIPEA werden in 50 mL Dichlormethan gelöst und 30 Minuten bei 25°C gerührt. Dann werden 77 mg exo -3-β-Amino-Tropan zugegeben und für weitere 17 Stunden Bei 25°C gerührt. Zur Lösung werden 50 mL Wasser und 1 g Kaliumcarbonat gegeben und die organische Phase mittels Phasentrennkartusche abgetrennt, dann im Vakkum eingeengt. Anschließend wird das Produkt durch Kieselgelchromatograhie gereinigt und das gereinigte Produkt mit Hilfe von Ether kristallisiert. Ausbeute: 0,03 g weiße Kristalle.

### Beispiel 46

0,15 g der Verbindung Z3 , 0,12 g TBTU, 0,12 mL DIPEA werden in 5 mL Dichlormethan gelöst und 30 Minuten bei 25°C gerührt. Dann werden 50 mg 1-Methyl-4-aminopiperidin zugegeben und für weitere 2,5 Stunden Bei 25°C gerührt. Die Lösung wird dann mit Wasser extrahiert und anschließend eingeengt. Der Rückstand wird in warmen Ethylacetat gelöst und durch Ether und Petrolether kristallisiert. Ausbeute: 0,025 g weiße Kristalle.

### Beispiel 80

0,2 g der Verbindung Z8, 0,2 g TBTU, 0,1 mL DIPEA werden in 10 mL Dichlormethan gelöst und 30 Minuten bei 25°C gerührt. Dann werden 100 mg 1-Methyl-4-aminopiperidin zugegeben und für weitere 17 Stunden bei 25°C gerührt. Die Lösung wird dann mit einer verdünnten Kaliumcarbonatlösung extrahiert und eingeengt. Der Rückstand wird mit Hilfe von Ether kristallisiert. Ausbeute: 0,12 g weiße Kristalle.

### Beispiel 190

0,2 g Verbindung Z8, 0,2 g TBTU, 0,3 mL DIPEA werden in 5 mL Dichlormethan gelöst und 1h bei 25°C gerührt. Dann werden 0,13 g 4-Amino-1-benzylpiperidin zugegeben und für eine weiter Stunde bei 25°C gerührt. Die Lösung wird dann mit 10 mL Methylenchlorid verdünnt und mit 20 mL Wasser extrahiert. Anschließend wird das Produkt über Kieselgel gereinigt und mittels Ethylacetat und Ether kristallisiert. Ausbeute: 0,23 g der Verbindung Z8

0,23 g des Benzylamines Z8 werden in 10 mL Methanol gelöst, mit 50 mg Pd/C versetzt und 3h bei 3 bar bei 25°C hydriert. Duch Zugabe von Petrolether und Ethylacetat werden weiße Kristalle erhalten. Diese werden über Kieselgel chromatographiert und mit Hilfe von Ethylacetat und Ether kristallisiert. Ausbeute: 0,075 g weiße Kristalle.

### Beispiel 196

0,1g Verbindung Z10, 0,09 g TBTU, 0,3 mL DIPEA werden in 4 mLDichlormethan gelöst und 20 Minuten bei 25°C gerührt. Dann wird 67 mg 1,1-Dimethyl-N-methylpiperazin-1-yl-ethylamin zugegeben und für weitere 2 Stunden Bei 25°C gerührt. Die Lösung wird dann mit Dichlormethan verdünnt und mit Wasser extrahiert. Anschließend wird über Kieselgel chromtografiert und der Rückstand in Aceton gelöst, mit etherischer HCl versetzt und der entstandene Niederschlag isoliert. Ausbeute: 0,09 g hellgelber Feststoff

### Beispiel 166

0,1 g der Verbindung Z10, 0,11 g TBTU, 0,14 mL DIPEA werden in 2 mL Dimethylformamid gelöst und 3h bei 50°C gerührt. Dann wird 55 mg 4-Morpholinomethylphenylamin zugefügt. Anschließend wird die Reaktion innerhalb von 17 Std. auf Raumtemperatur abgekühlt. Dann wird das Dimethylformamid im Vakkum entfernt, der Rückstand in Dichlormethan aufgenommen und mit Wasser extrahiert. Anschließend wird über Kieselgel chromatographiert und das Produkt aus Ethylacetat und Ether kristallisiert. Ausbeute: 0,06 g gelbliche Kristalle

### Beispiel 81

0,2 g der Verbindung Z4, 0,2 g TBTU, 0,1 mL DIPEA werden in 10 mL Dichlormethan gelöst und 30 Minuten bei 25°C gerührt. Dann werden 0,1 g 1-Methyl-4-aminopiperidin zugegeben und für weitere 17 Stunden bei 25°C gerührt. Die Lösung wird dann mit wässriger Kaliumcarbonatlösung extrahiert und anschließend eingeengt. Der Produkt wird mit Hilfe von Ether kristallisiert. Ausbeute: 0,16 g weiße Kristalle.

### Beispiel 162

0,1 g der Verbindung Z5, 0,07 g TBTU, 0,15 mL DIPEA werden in 5 mL Dichlormethan gelöst und 20 Minuten bei 25°C gerührt. Dann werden 0,04 g 1-Methyl-4-aminopiperidin zugegeben und für weitere 2 Stunden bei 25°C gerührt. Die Lösung wird dann mit 15 mL Dichlormethan verdünnt und mit 20 mL Wasser extrahiert. Der Rückstand wird in MeOH und Aceton gelöst, mit 1 mL etherische HCl versetzt und eingeengt. Mit Hilfe von Ether, Ethylacetat und wenig MeOH wird ein kristallines Produkt erhalten. Ausbeute: 0,1 g weiße Kristalle.

### Beispiel 88

0,1 g der Verbindung Z6, 0,12 g TBTU, 0,12 mL DIPEA werden in 10 mL Dichlormethan gelöst und 30 Minuten bei 25°C gerührt. Dann werden 0,04 g 1-Methyl-4-aminopiperidin zugegeben und für weitere 2 Stunden bei 25°C gerührt. Die Lösung wird dann mit 10 mL Dichlormethan verdünnt und mit 10 mL Wasser extrahiert. Mit Hilfe von Ethylacetat , Ether und Petrtolether wird ein kristallines Produkt erhalten. Ausbeute: 0,6 g weiße Kristalle.

### Beispiel 89

0,1 g der Verbindung Z6, 0,08 g TBTU, 0,08 mL DIPEA werden in 10 mL Dichlormethan gelöst und 30 Minuten bei 25°C gerührt. Dann werden 37 µL N,N-Dimethylneopentandiamin zugegeben und für weitere 2 Stunden bei 25°C gerührt. Die Lösung wird dann mit 10 mL Dichlormethan verdünnt und mit 10 mL Wasser extrahiert. Das Produkt wird dann über Kieselgel chromatografiert und mit Hilfe von Ethylacetat , Ether und Petrtoletherkristallisiert. Ausbeute: 0,005 g weiße Kristalle.

### Beispiel 26

0,15 g der Verbindung Z7, 0,16 g TBTU, 1 mL DIPEA werden in 5 mL Dichlormethan gelöst und 30 Minuten bei 25°C gerührt. Dann werden 0,1 g 4-Morpholinocyclohexylamin zugegeben und für weitere 17 Stunden bei 25°C gerührt. Der Rückstand wird dann mit 10 mL 10%iger Kaliumcarbonatlösung versetzt, der Niederschlag isoliert und mit Wasser gewaschen. Dann wird in Dichlormethan gelöst und erneut eingeengt. Mit Hilfe von Ethylacetat, wird das Produkt kristallisiert. Ausbeute: 0,1 g weiße Kristalle.

### Beispiel 9

150 mg der Verbindung Z9 und 93 mg cis-4-Morpholino-cyclohexylamin werden in 5 mL Dichlormethan gelöst und mit 160 mg TBTU und 1 mL DIPEA 12 Std. bei RT gerührt. Das Lösungsmittel wird im Vakuum abgezogen, der Rückstand mit 10 mL 10%ige Kaliumcarbonatlösung versetzt. Der Niederschlag wird abgesaugt, mit Wasser gewaschen, in Dichlormethan aufgenommen, getrocknet und das Lösungsmittel im Vakuum abgezogen. Der Rückstand wird aus Essigester kristallisiert. Ausbeute: 82,0 mg.

### Beispiel 16

150 mg der Verbindung Z8 und 73 mg trans-4-Piperidino-cyclohexylamin werden in 5 mL Dichlormethan gelöst und mit 160 mg (0,50 mMol) TBTU und 1 mL DIPEA 12 Std. bei RT gerührt. Das Lösungsmittel wird im Vakuum abgezogen, der Rückstand mit 10 mL 10%ige Kaliumcarbonatlösung versetzt. Der Niederschlag wird abgesaugt, mit Wasser gewaschen, in Dichlormethan aufgenommen, getrocknet und das Lösungsmittel im Vakuum abgezogen. Der Rückstand wird aus Essigester kristallisiert. Ausbeute: 87,0 mg.

### Beispiel 37

100 mg der Verbindung Z9 und 42 mg 3-Amino-1-ethyl-pyrolidine werden in 10 mL Dichlormethan gelöst und mit 90 mg TBTU und 0,5 mL DIPEA 12 Std. bei RT gerührt. Das Lösungsmittel wird im Vakuum abgezogen, der Rückstand mit 10 ml 10%ige Kaliumcarbonatlösung versetzt. Der Niederschlag wird abgesaugt, mit Wasser gewaschen, in Dichlormethan aufgenommen, getrocknet und das Lösungsmittel im Vakuum abgezogen. Der Rückstand wird aus Essigester/Petrolether kristallisiert. Ausbeute: 24,0 mg.

### Beispiel 120

100 mg der Verbindung Z11 und 73 mg 4-Amino-1-tetrahydro-4H-pyran-4-yl-piperidin werden in 10 mL Dichlormethan gelöst und mit 90 mg TBTU und 0,5 mL DIPEA 1 Std. bei RT gerührt. Das Lösungsmittel wird im Vakuum abgezogen, der Rückstand mit 10 mL 10%ige Kaliumcarbonatlösung versetzt. Der Niederschlag wird abgesaugt, mit Wasser gewaschen, in Dichlormethan aufgenommen, getrocknet und das Lösungsmittel im Vakuum abgezogen. Der Rückstand wird aus Essigester/Petrolether kristallisiert. Ausbeute: 89 mg.

### Beispiel 212

150 mg der Verbindung Z5 und 150 mg trans-4-(4-Cyclopropylmethyl-piperazin-1-yl)-cyclohexylamin (als Hydrochlorid) werden in 5 mL Dichloromethan gelöst und mit 160 mg TBTU und 2 mL DIPEA 2 Std. bei RT gerührt. Das Lösungsmittel wird im Vakuum abgezogen, der Rückstand mit 10 mL 10%ige Kaliumcarbonatlösung versetzt. Der Niederschlag wird abgesaugt, mit Wasser gewaschen, in Dichlormethan aufgenommen, getrocknet und das Lösungsmittel im Vakuum abgezogen. Der Rückstand wird über eine Säule gereinigt (20 mL Kieselgel, 300 mL Essigester 90/ Methanol 10 + 2% konz. Ammoniak). Die geeigneten Fraktionen werden i.Vak eingeengt und aus Essigester kristallisiert. Ausbeute: 140 mg

### Beispiel 232

390 mg der Verbindung Z11 und 240 mg trans-4-(4-tButyloxycarbonyl-piperazin-1-yl)-cyclohexylamin werden in 2,5 mL NMP gelöst und mit 482 mg TBTU und 1 mL Triethylamin 2 Std. bei RT gerührt. Anschließend wird mit 100 mL Wasser und 200 mg Kaliumcarbonat versetzt, der Niederschlag wird abgesaugt, mit Wasser gewaschen und über eine Kieselgelsäule gereinigt. Die geeignete Fraktionen werden im Vakuum eingeengt, in 2 mL Dichlormethan gelöst, mit 2 mL Trifluoessigsäure versetzt und 2 Std. bei RT gerührt, erneut mit 100ml Wasser und 200 mg Kaliumcarbonat versetzt und der Niederschlag abgesaugt und mit Wasser gewaschen. Anschließend wird der Niederschlag über eine Kieselgelsäule gereinigt. Die geeignete Fraktionen werden im Vakuum eingeengt und der Rückstand aus Ethanol und konz. Salzsäure kristallisiert. Ausbeute: 95 mg.

### Beispiel 213

60 mg der Verbindung Beispiel 232 wird in 10 mL Essigester gelöst und mit 1 mL Essigsäureanhydrid und 1 mL Triethylamin 30 Min. bei RT gerührt. Das Lösungsmittel wird im Vakuum abgezogen, der Rückstand mit Wasser und Ammoniak versetzt, die ausgefallene Kristalle abgesaugt und mit Wasser und wenig kaltem Aceton gewaschen. Ausbeute: 40 mg.

### Beispiel 218

1,2 g der Verbindung Z9 und 0,5g 1,4-Dioxaspiro[4.5]dec-8-ylamin werden in 20 mL Dichlormethan gelöst und mit 1,28 g TBTU und 4 mL Triethylamin 12 Std. bei RT gerührt. Anschließend wird mit 50 mL Wasser und 0,5 g Kaliumcarbonat versetzt, die org. Phase abgetrennt, getrocknet und im Vakuum eingeengt. Der Rückstand wird aus Essigester kristallisiert, mit 25 mL 1 N Salzsäure und 20 mL Methanol versetzt und 30 Min. bei 50°C gerührt. Das Methanol wird im Vakuum abgezogen, der Niederschlag abgesaugt, mit Wasser gewaschen und getrocknet.

Der Rückstand wird in 20 mL Dichlormethan aufgenommen, mit 0,5 g Thiomorpholin und 0,5 g NaBH(OAc)₃ 12 Std. bei RT gerührt. Anschließend wird mit Wasser und Kaliumcarbonat versetzt, die org. Phase abgetrennt, getrocknet und das Lösungsmittel im Vakuum abgezogen. Der Rückstand wird über Kieselgelsäule gereinigt. Die geeigneten Fraktionen werden im Vakuum eingeengt und mit etherischer HCl das Hydrochlorid gefällt. Ausbeute: 86 mg trans-Isomer; amorphes Pulver.

### Beispiel 187

200 mg der Verbindung Z3 in 5 mL Dichlormethan wird mit 0,1 mL Diisopropylethylamin und 180 mg TBTU versetzt und 30 Min. gerührt. Anschließend werden 191 mg 4-(4-Methyl-piperazin-1-yl)-phenylamin zugegeben und über Nacht gerührt. Die Reaktionsmischung wird mit Wasser versetzt und die wässrige Phase mit Dichlormethan extrahiert. Die vereinten organischen Phasen werden über Na₂SO₄ getrocknet und eingeengt. Der Rückstand wird mittels Säulenchromatographie (Laufmittel: Dichlormethan/Methanol 100:7) gereinigt.

Ausbeute: 128 mg (leicht gelbe Kristalle)

Analog zu vorstehend beschriebener Vorgehensweise werden u.a. die in Tabelle 1 aufgeführten Verbindungen der Formel (I) erhalten.

Die in Tabelle 1 verwendeten Abkürzungen X₁, X₂, X₃, X₄ und X₅ stehen jeweils für eine Verknüpfung mit einer Position in der unter Tabelle 1 aufgeführten allgemeinen Formel anstelle der entsprechenden Reste R¹, R², R³, R⁴ und L-R⁵.

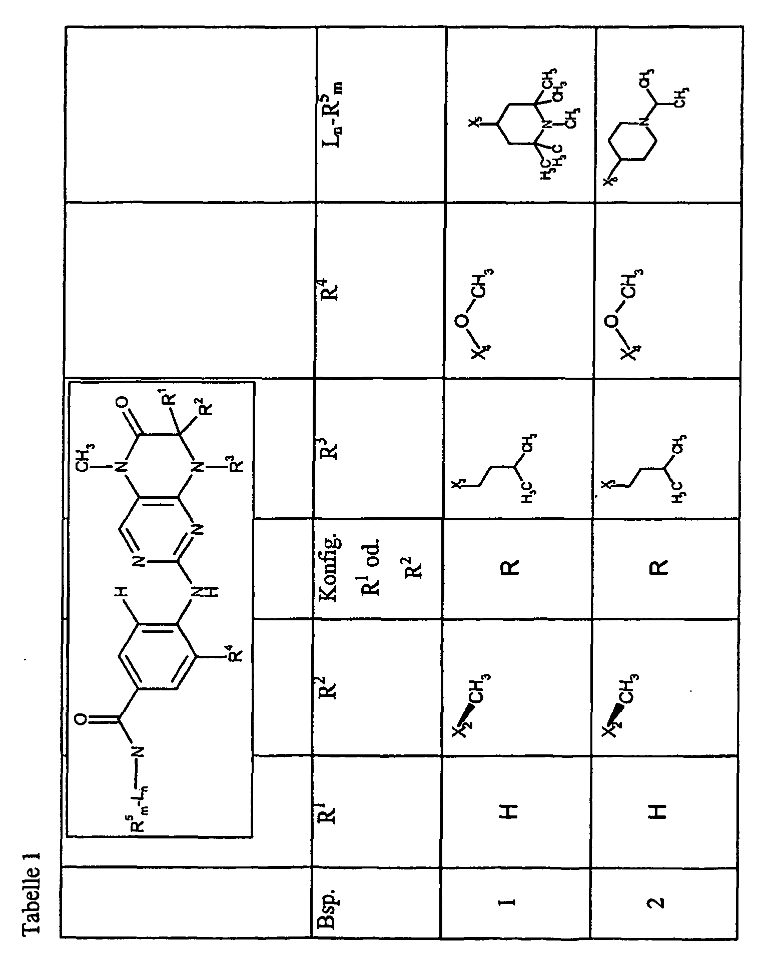

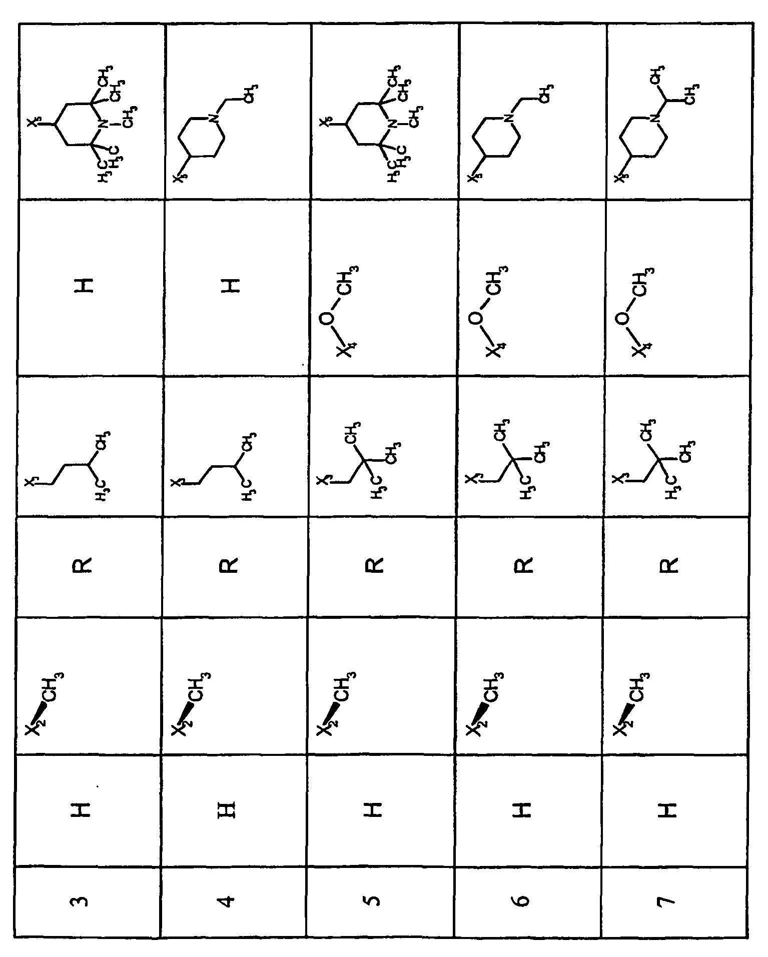

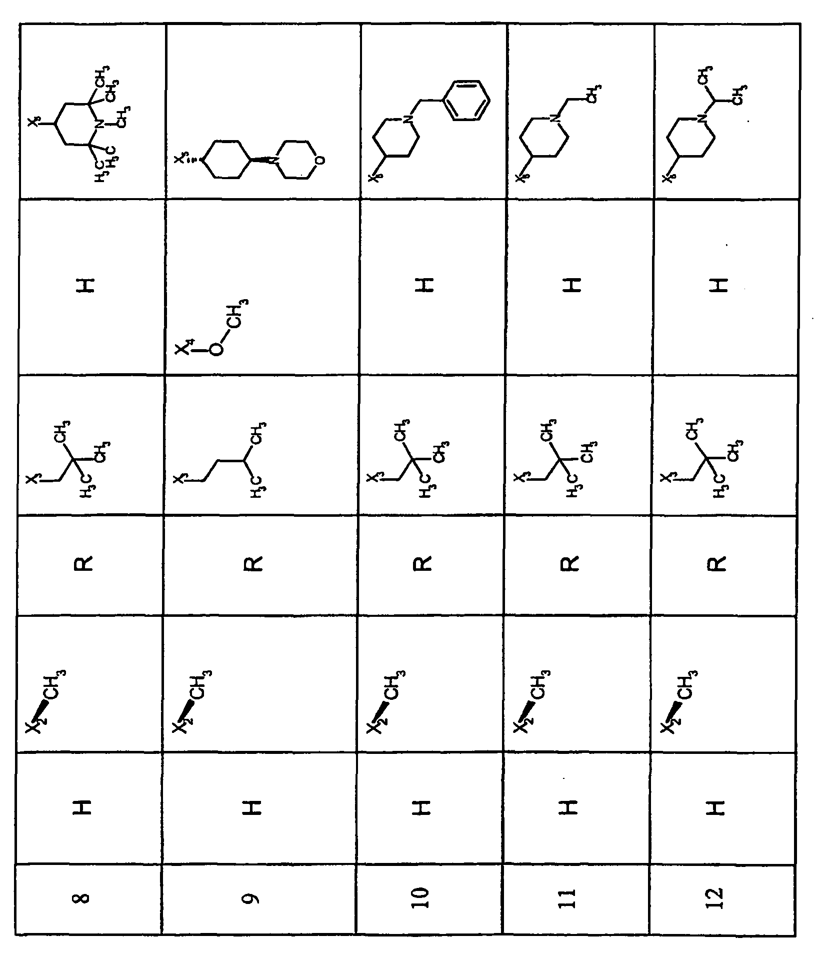

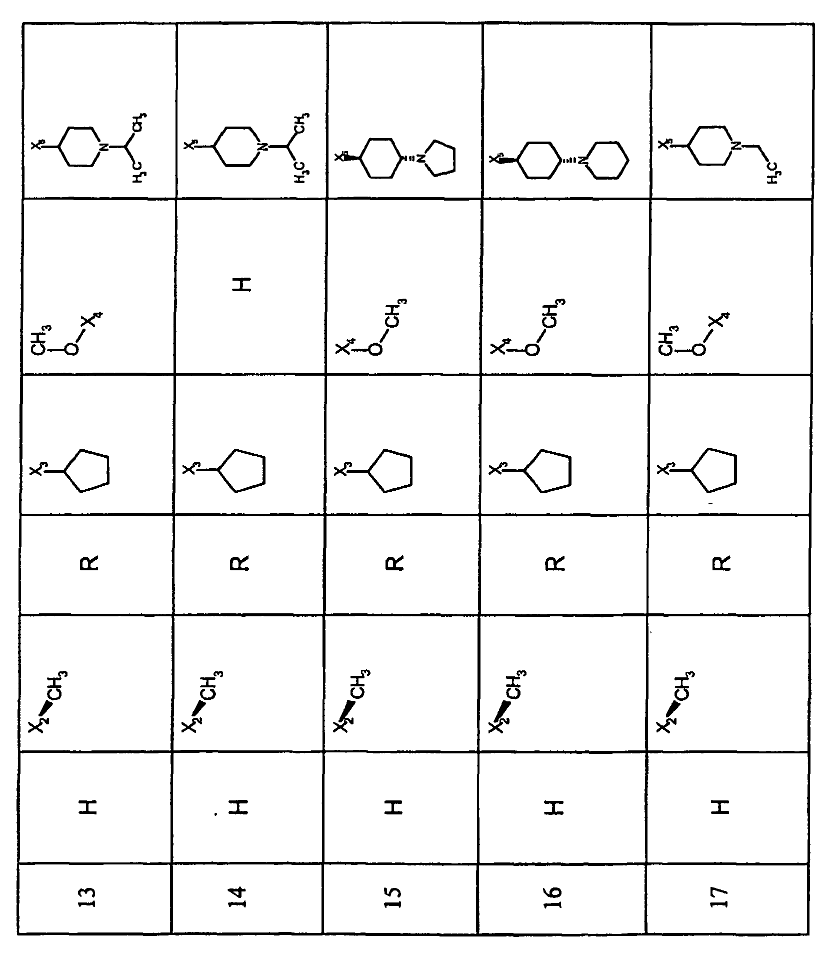

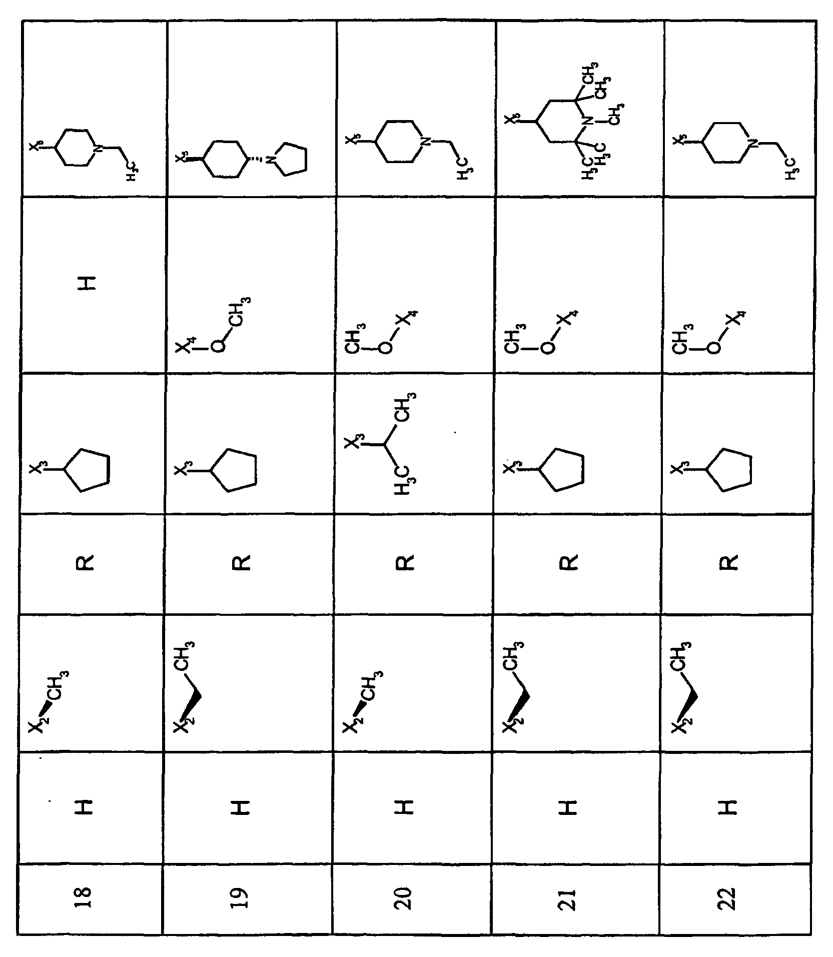

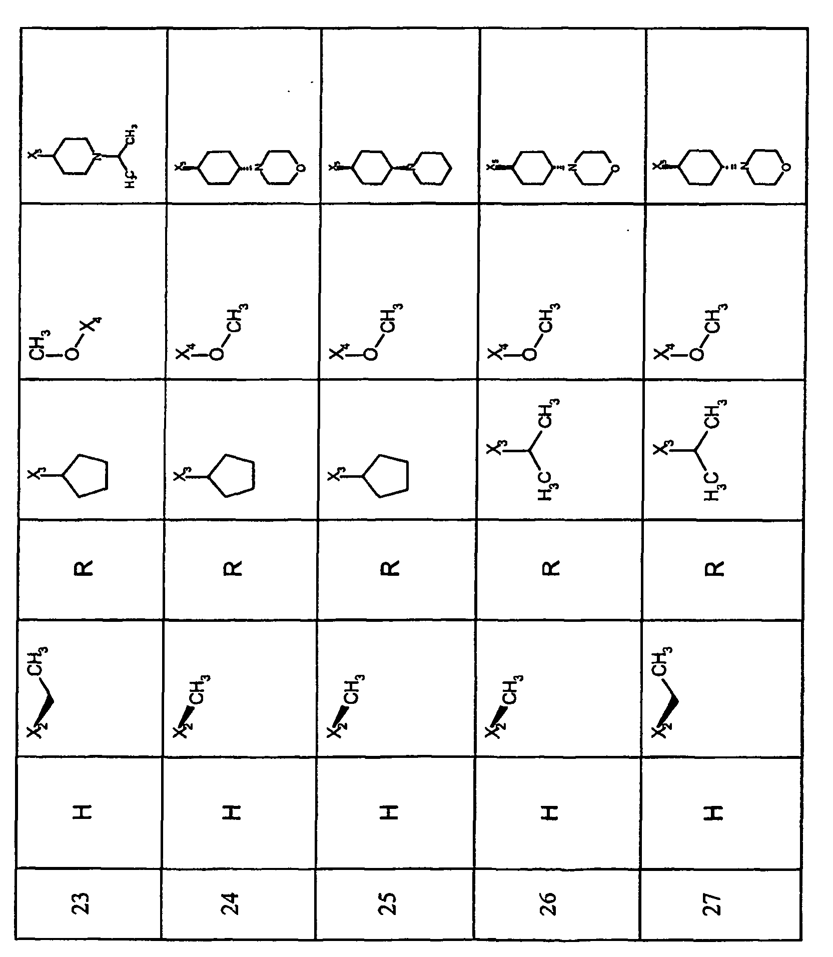

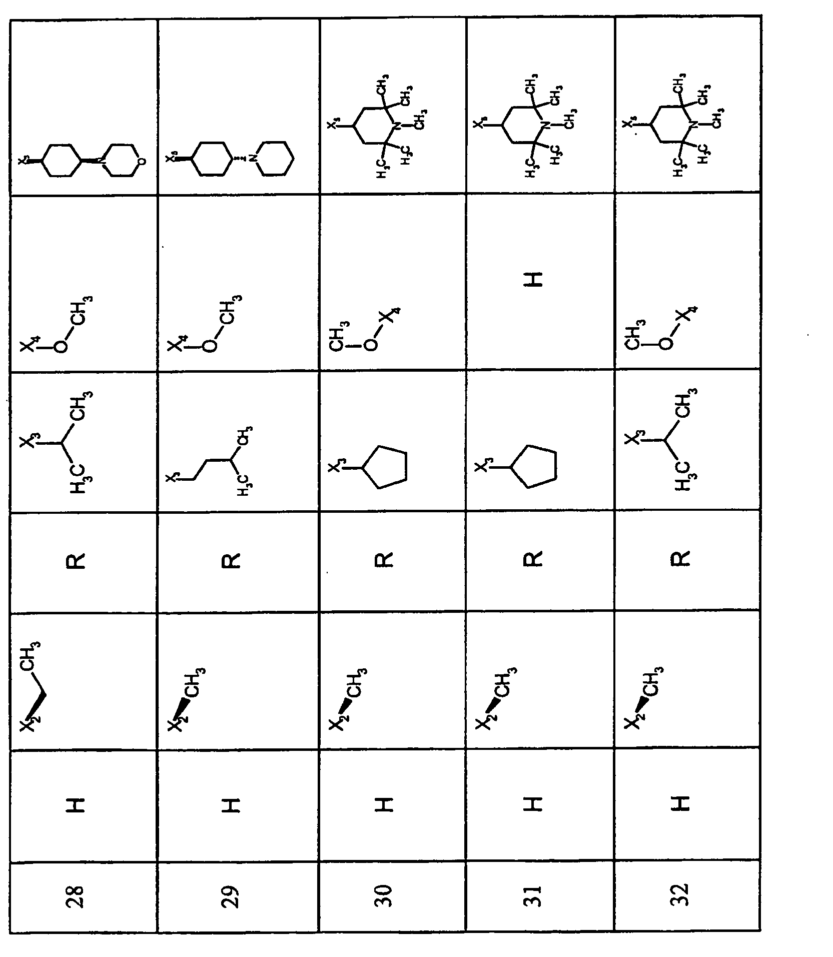

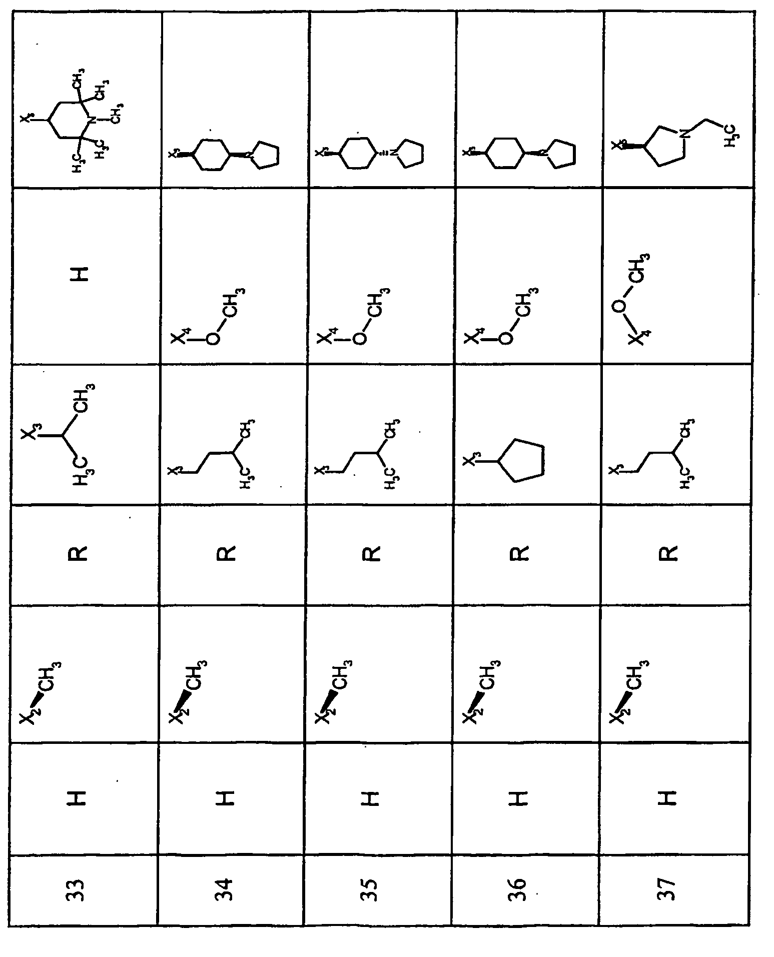

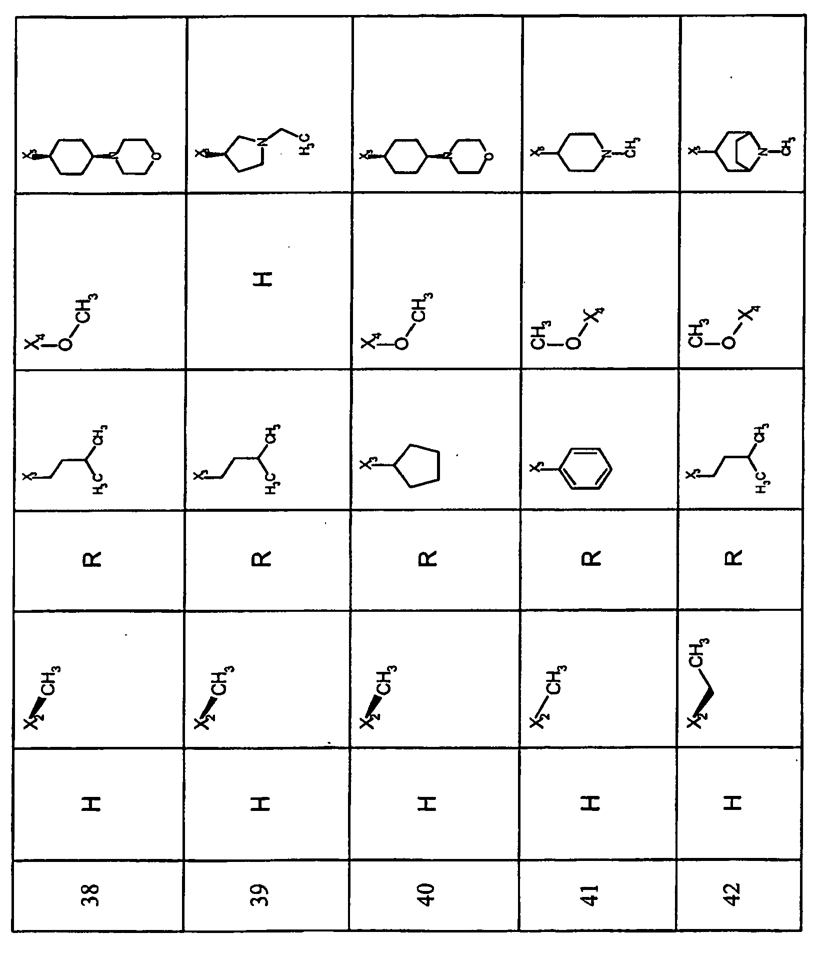

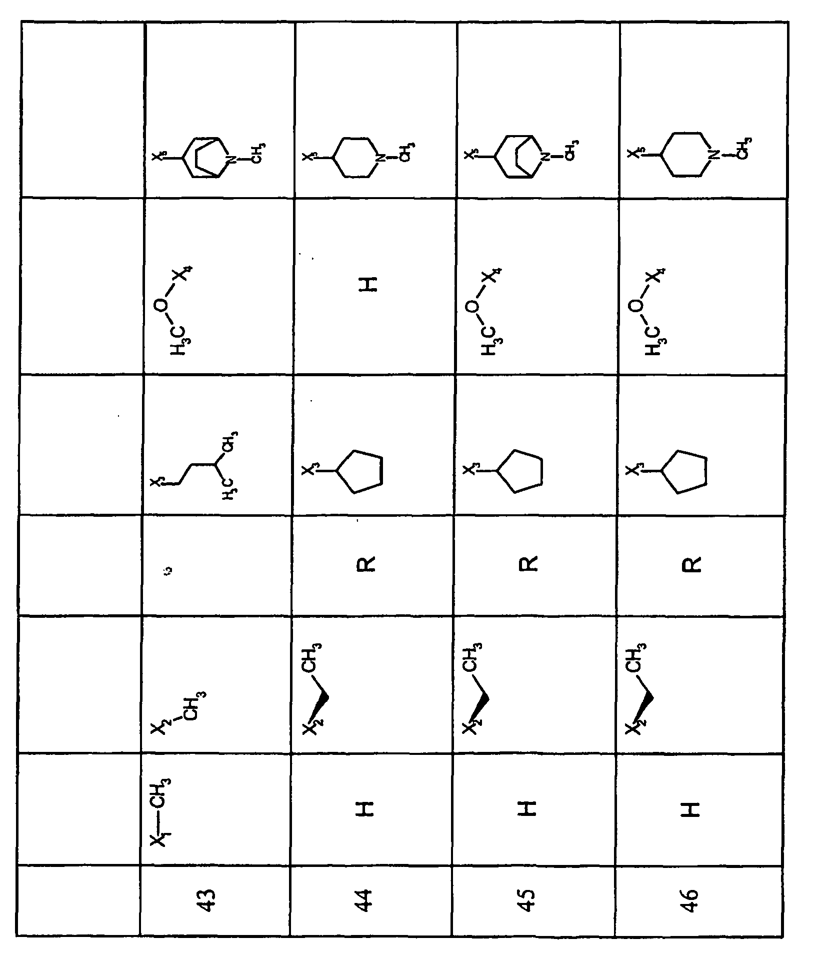

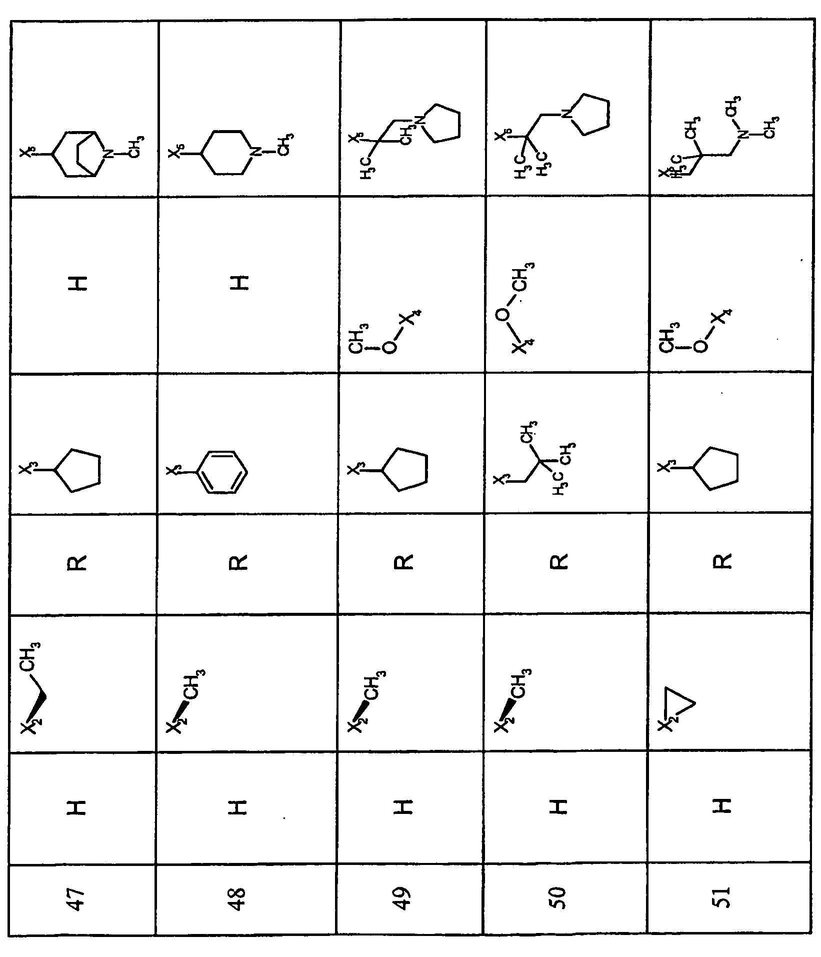

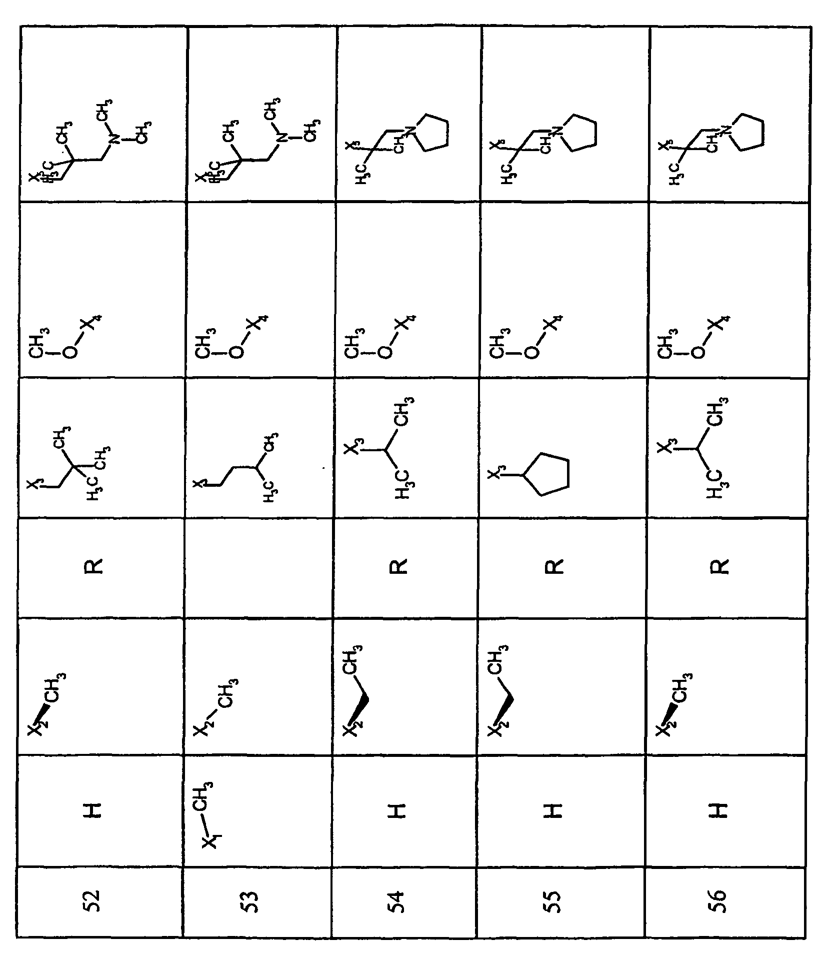

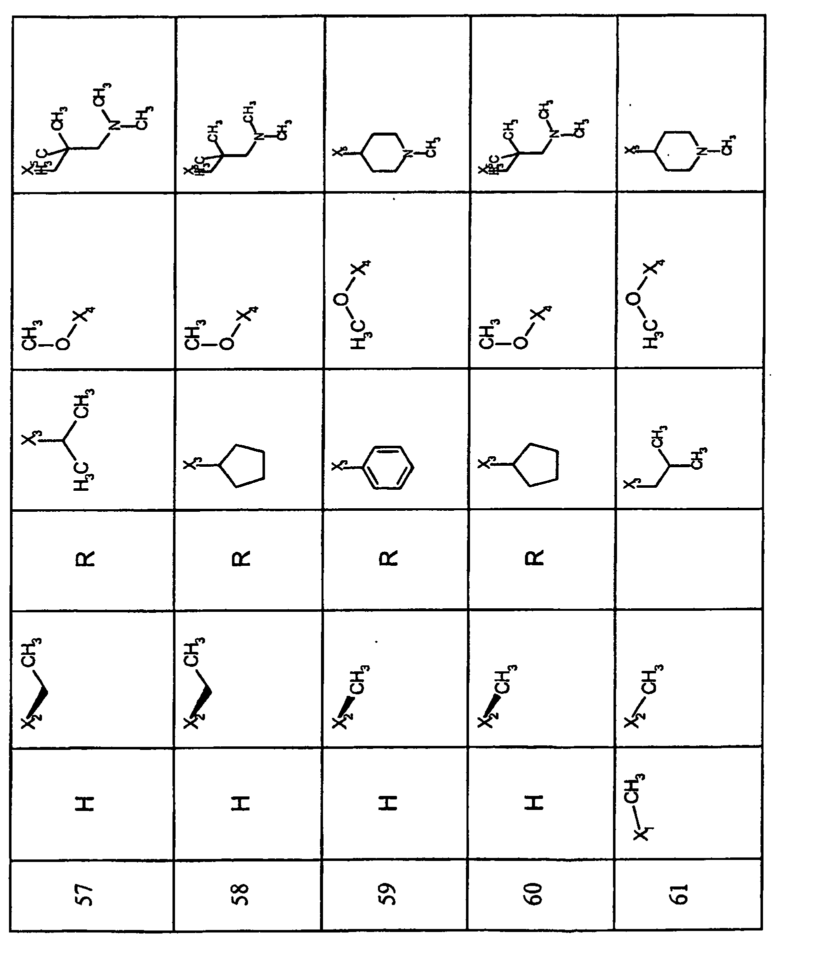

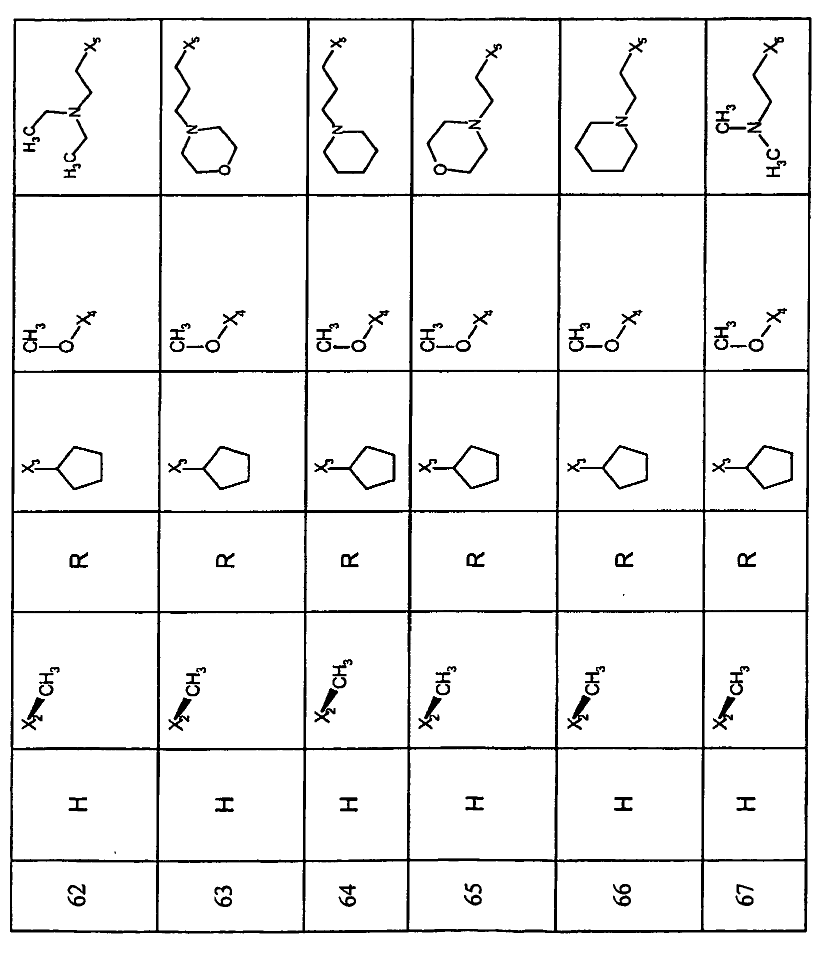

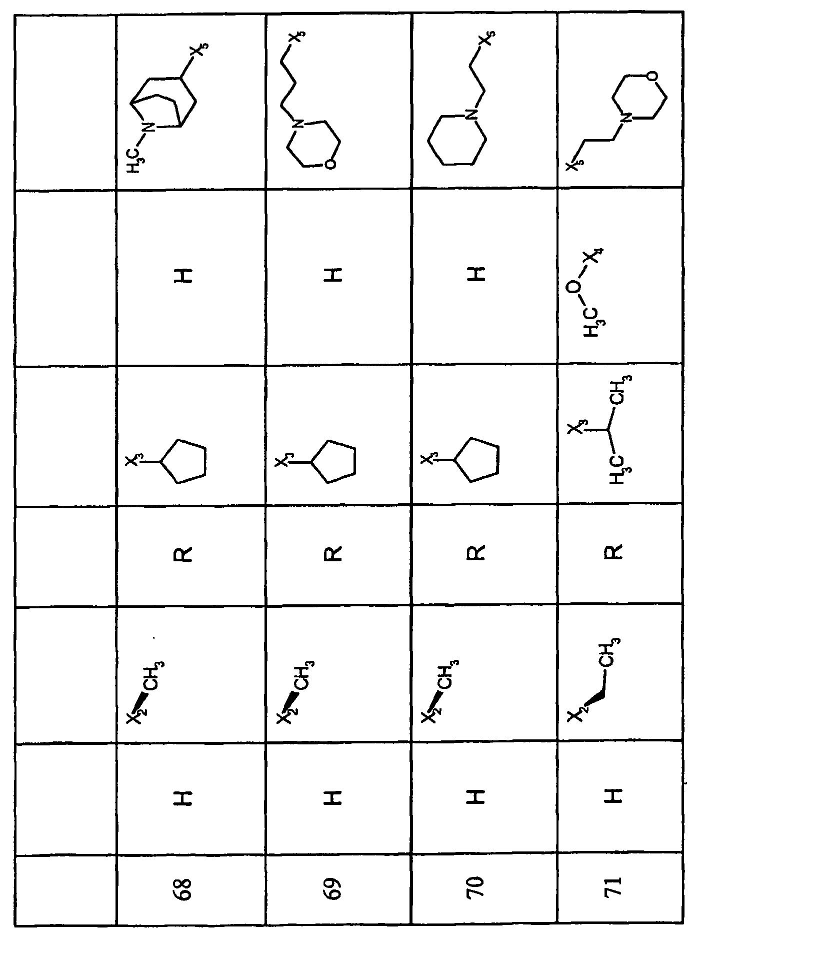

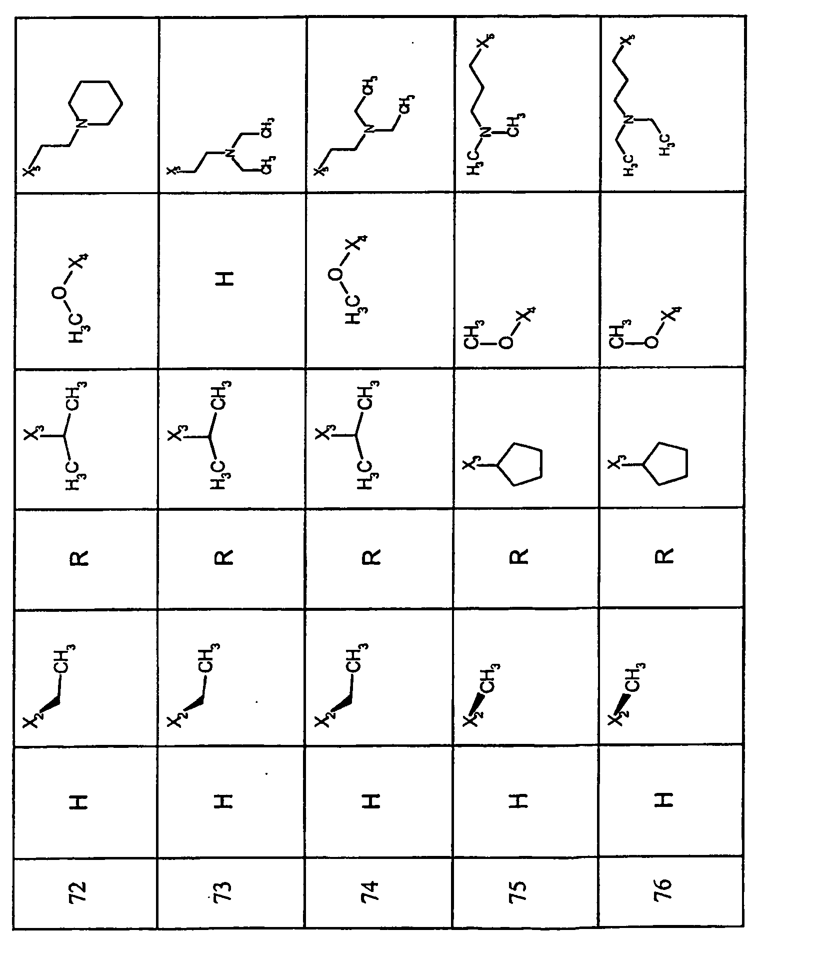

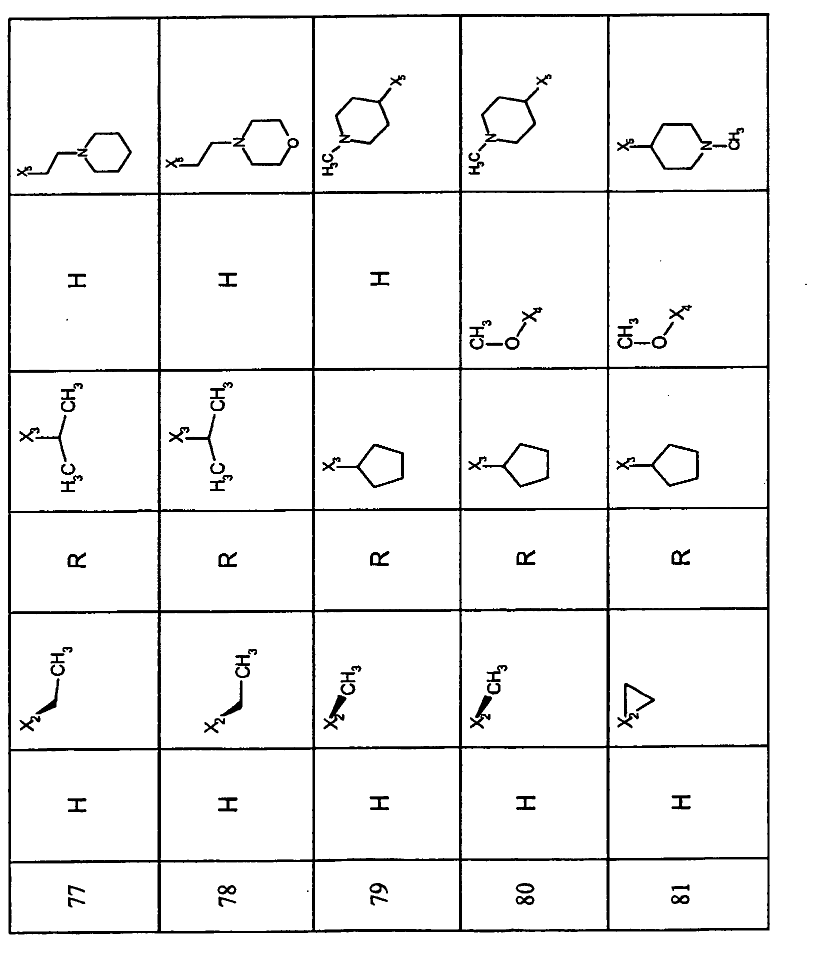

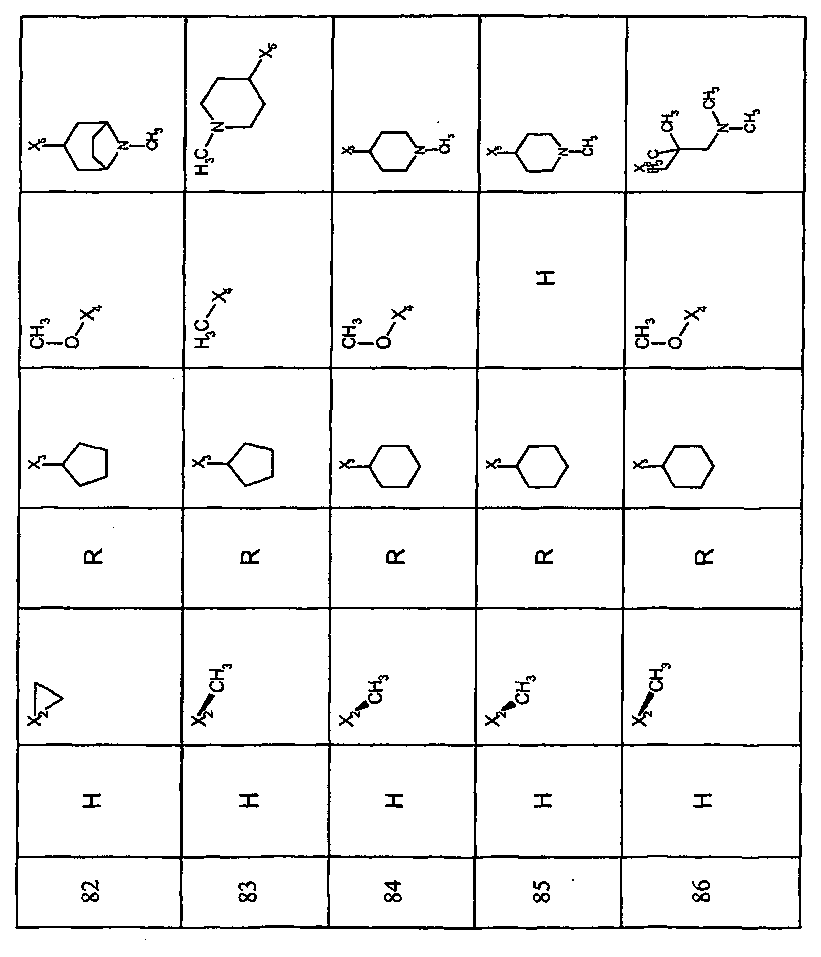

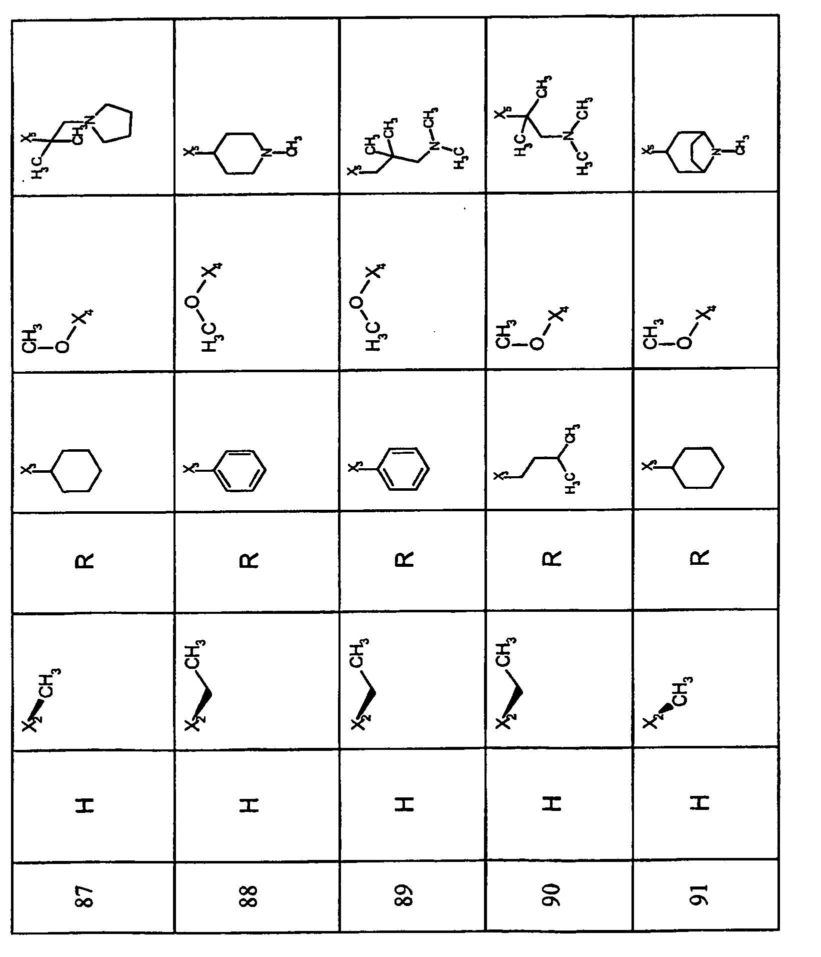

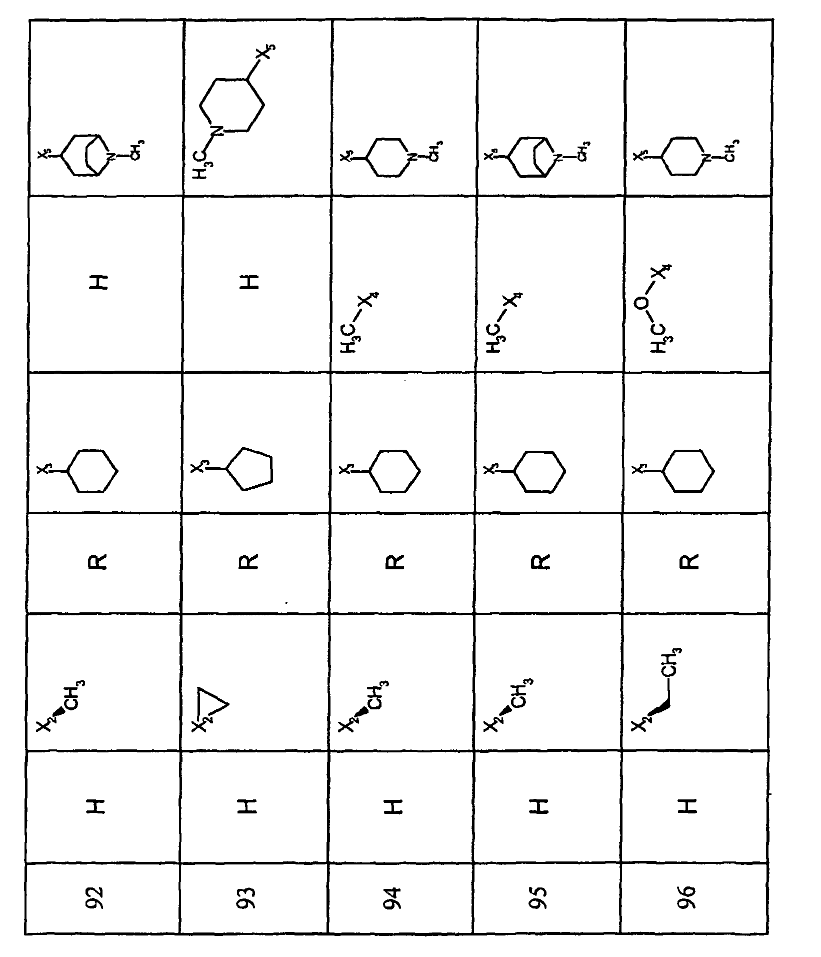

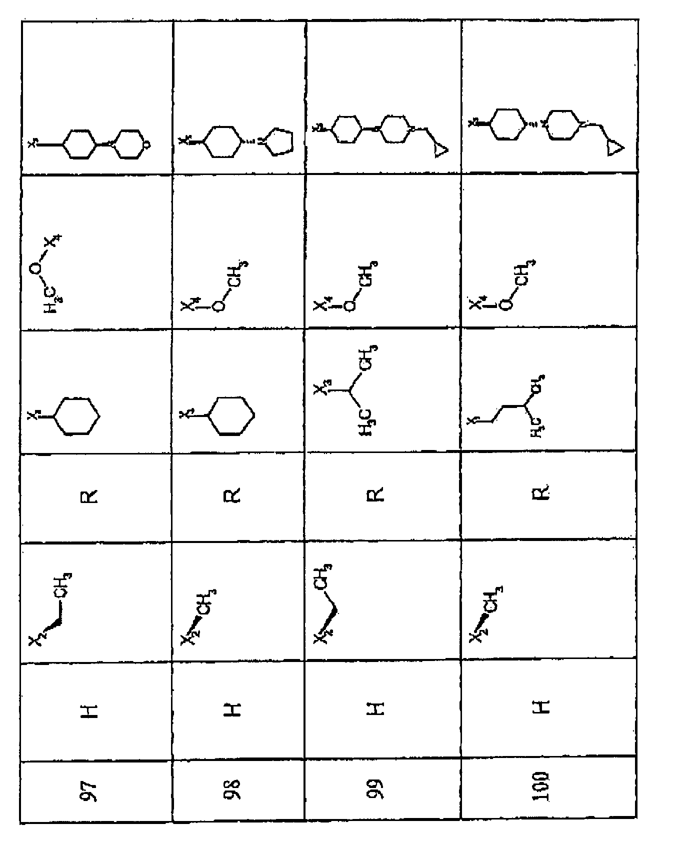

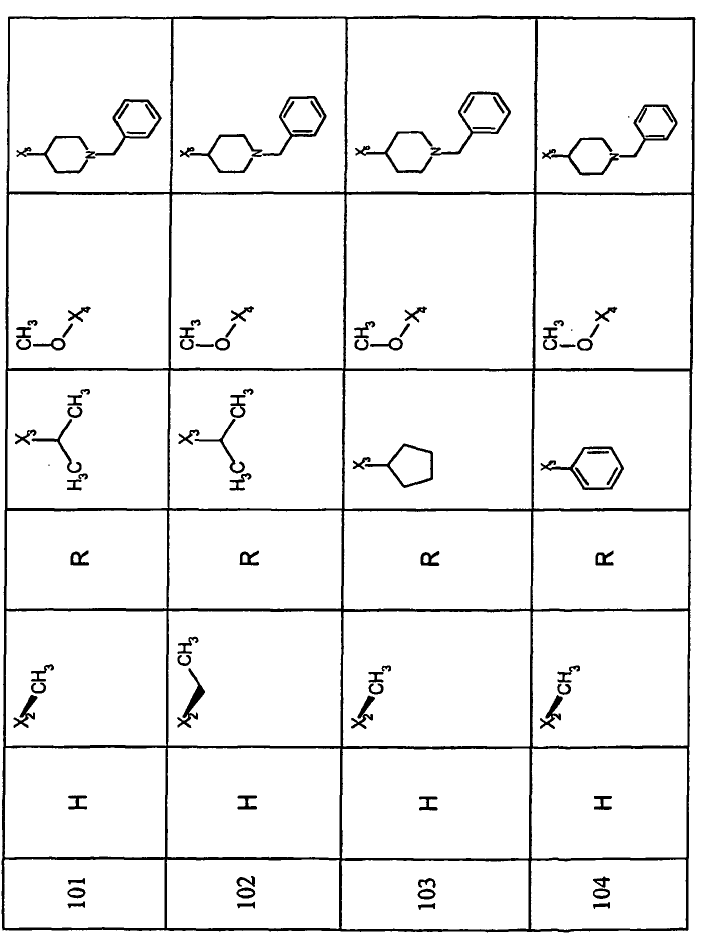

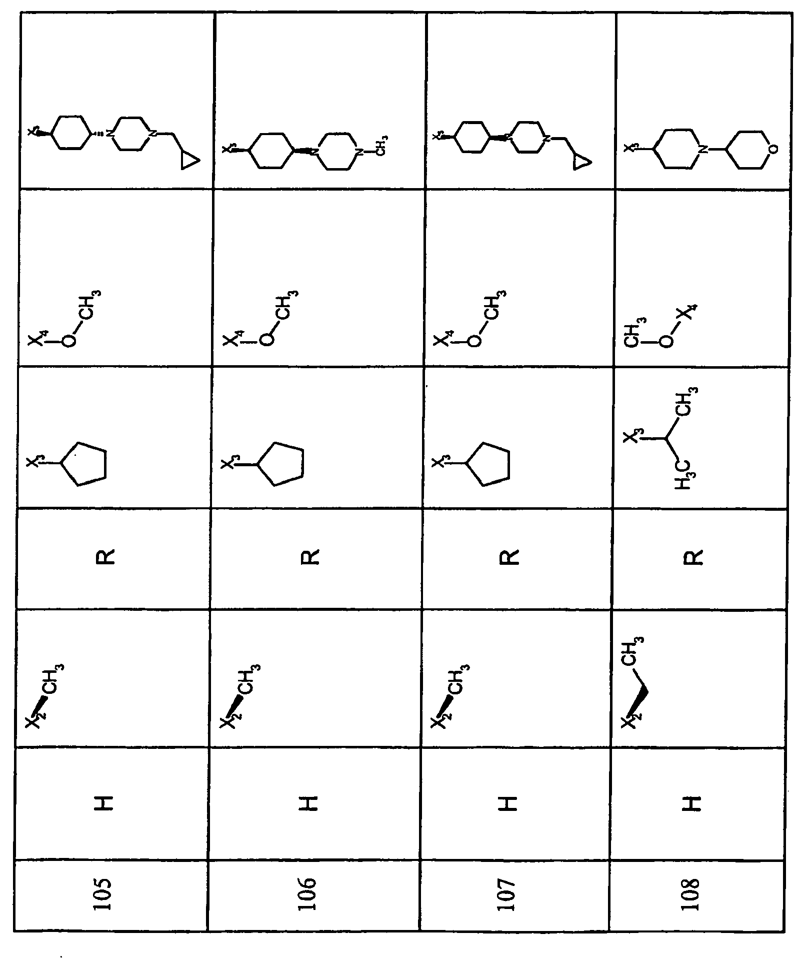

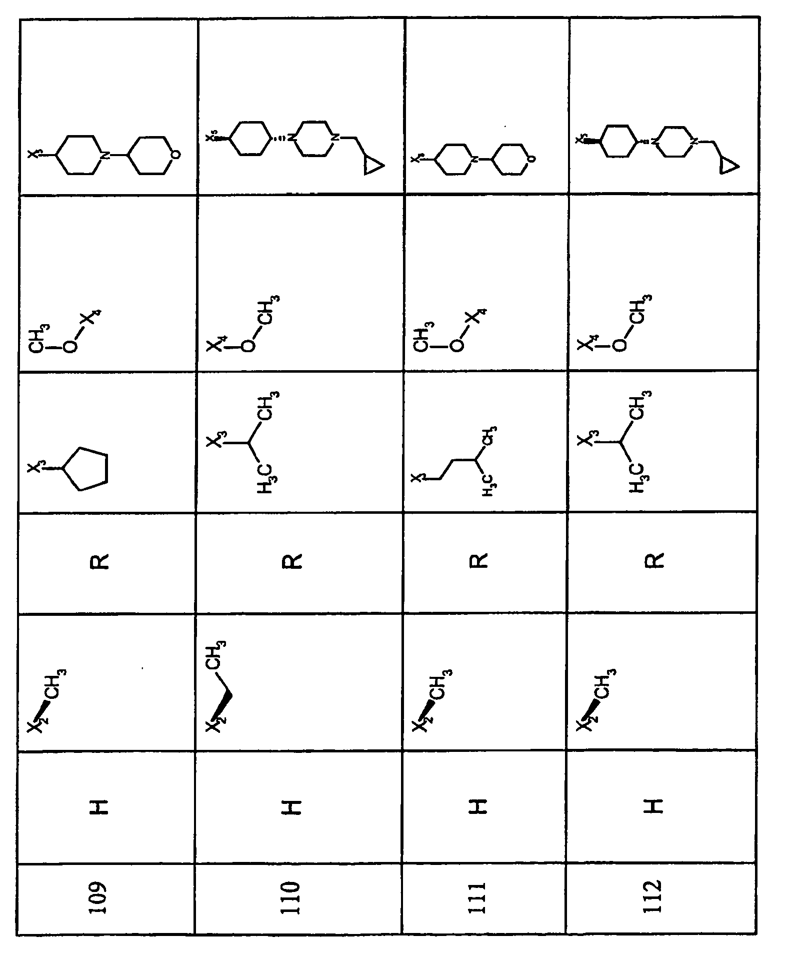

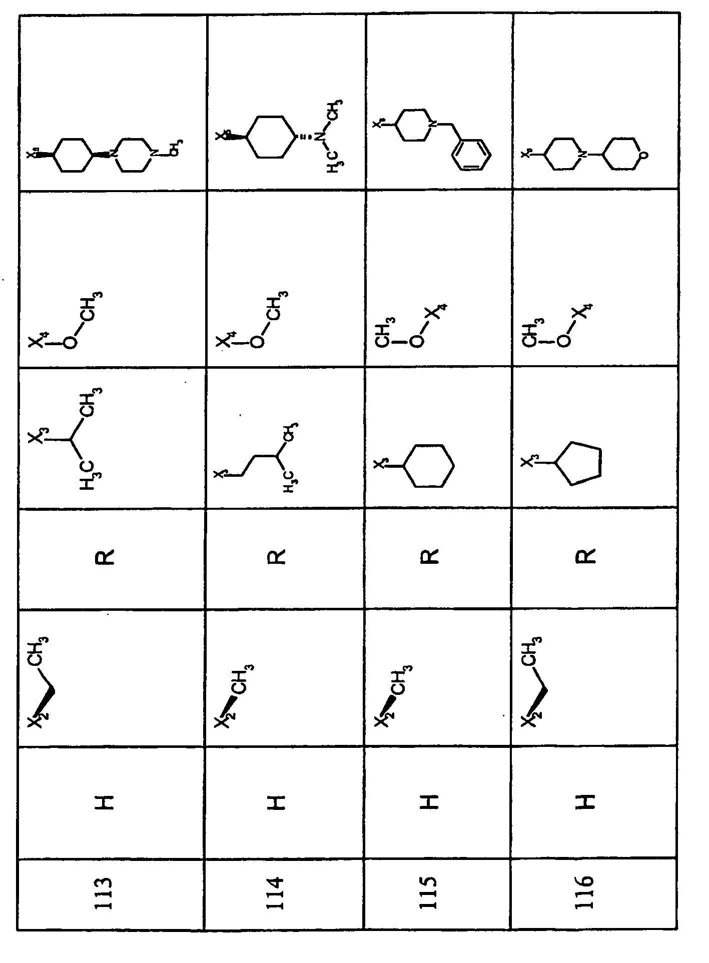

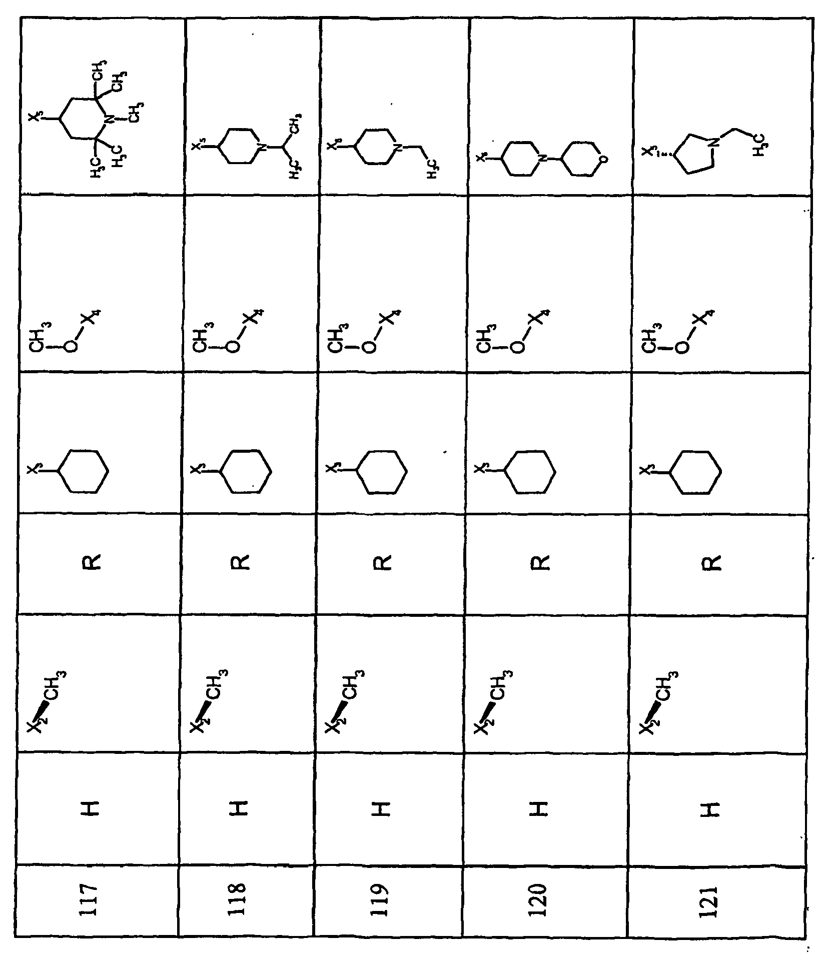

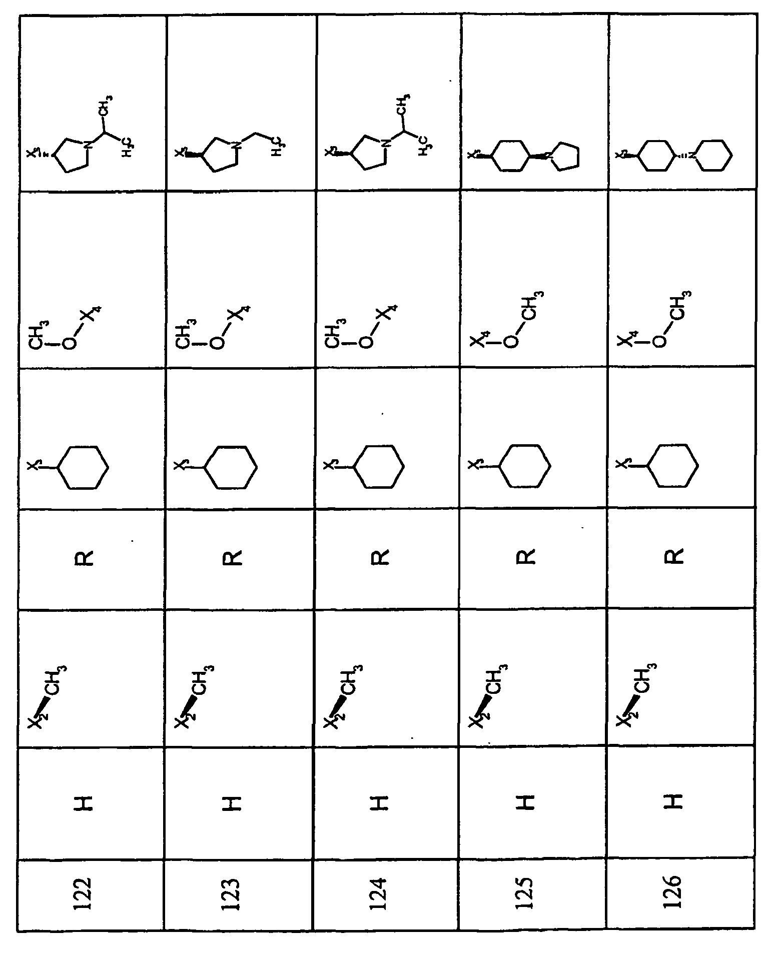

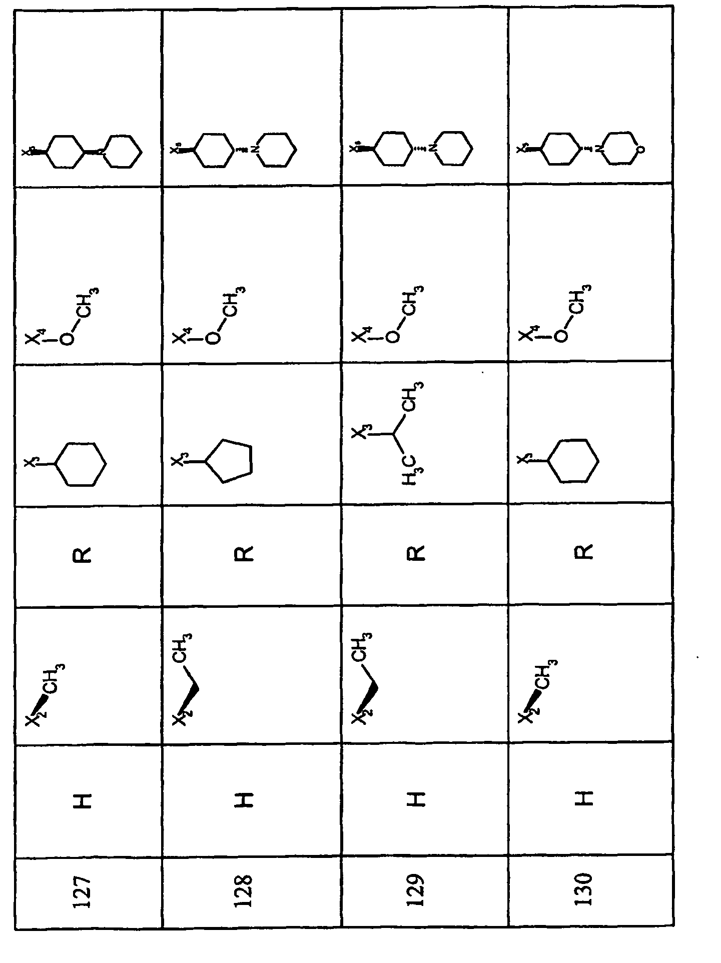

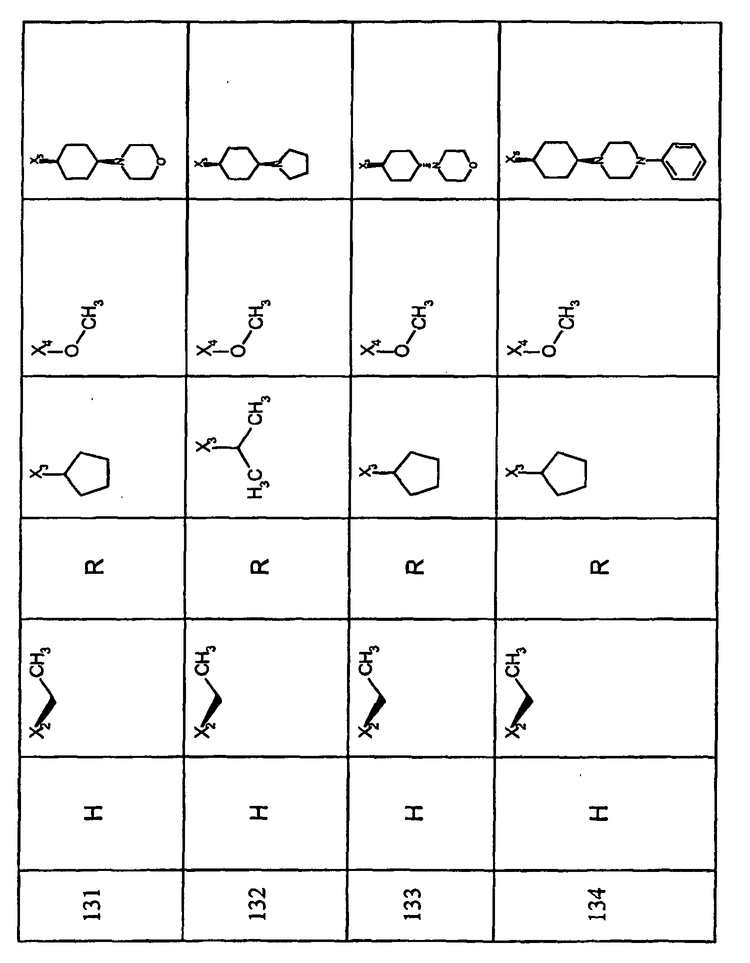

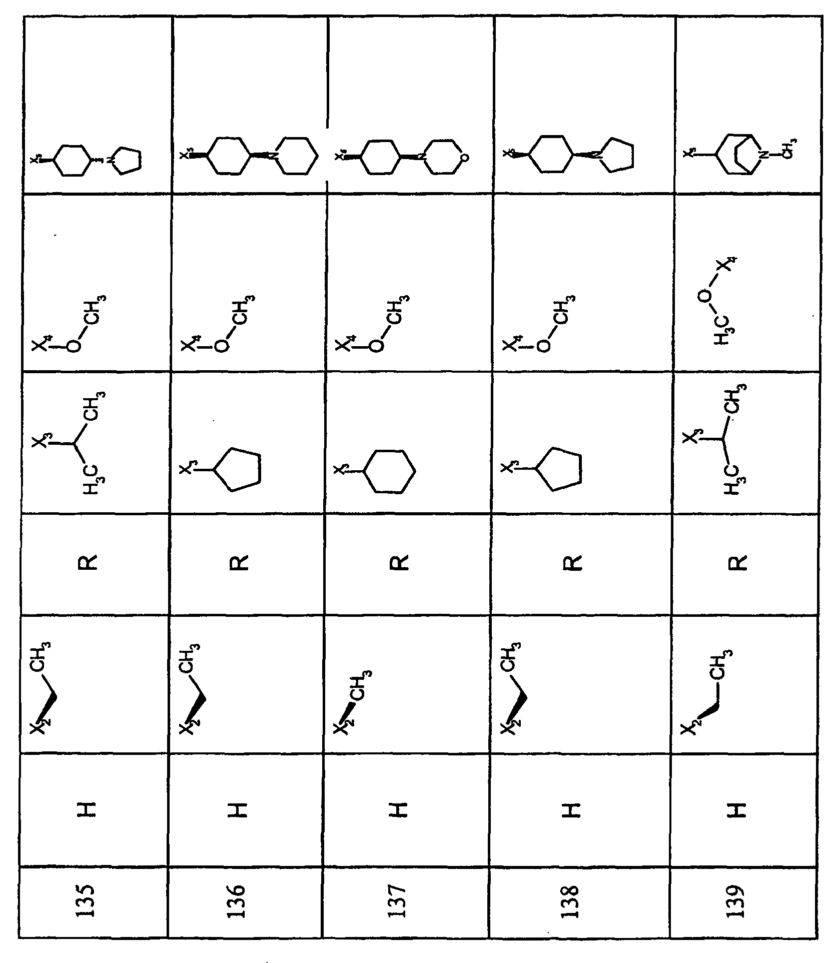

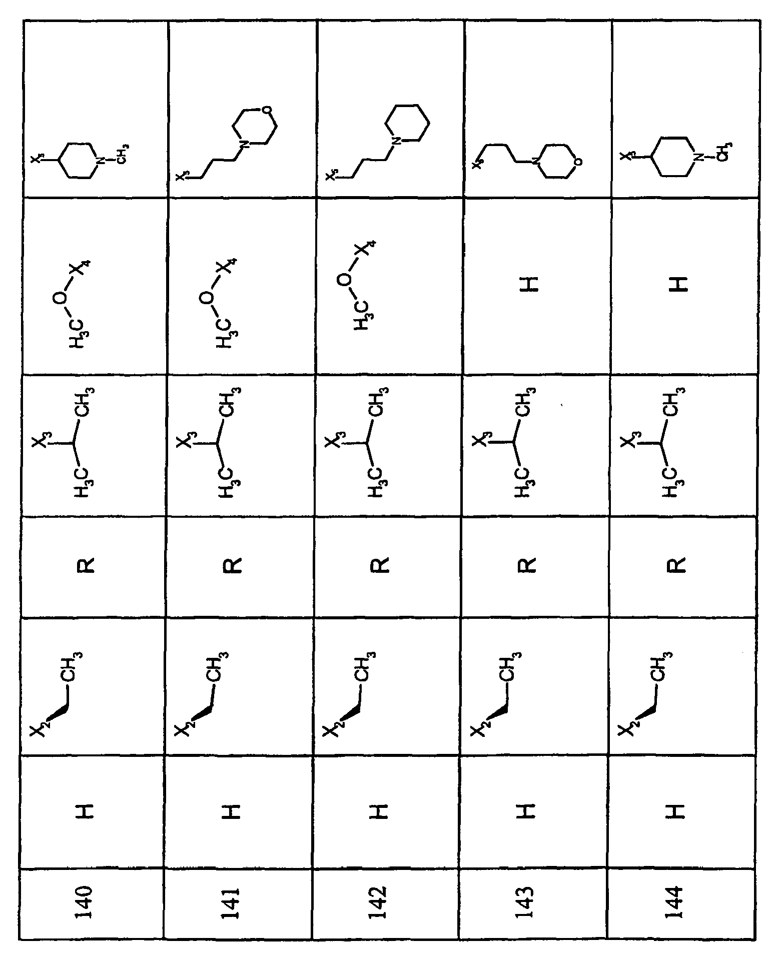

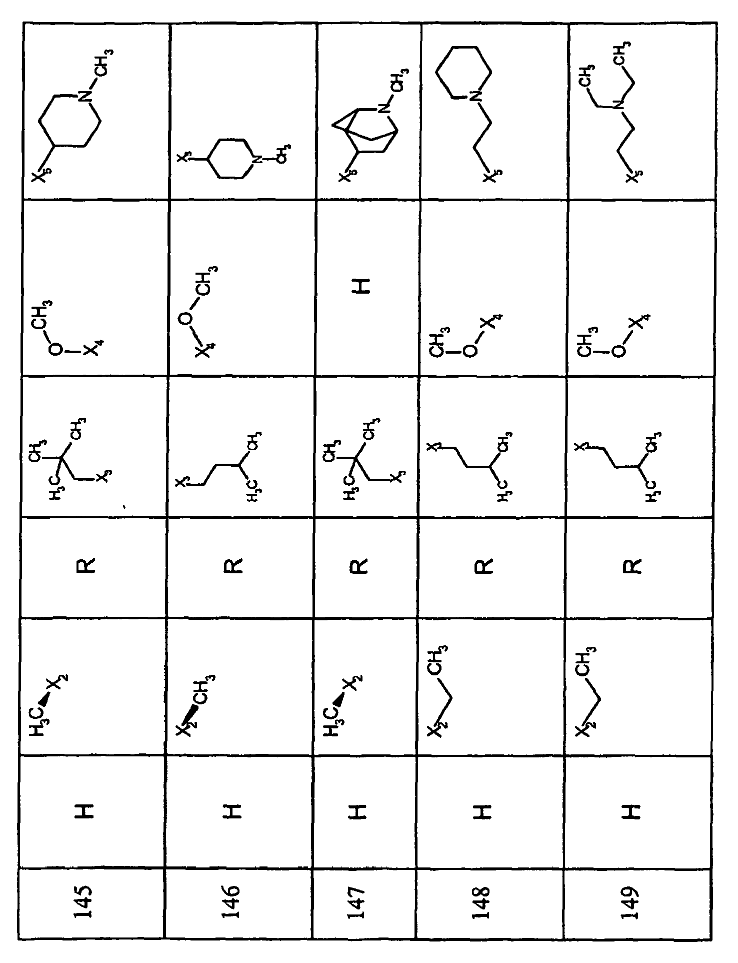

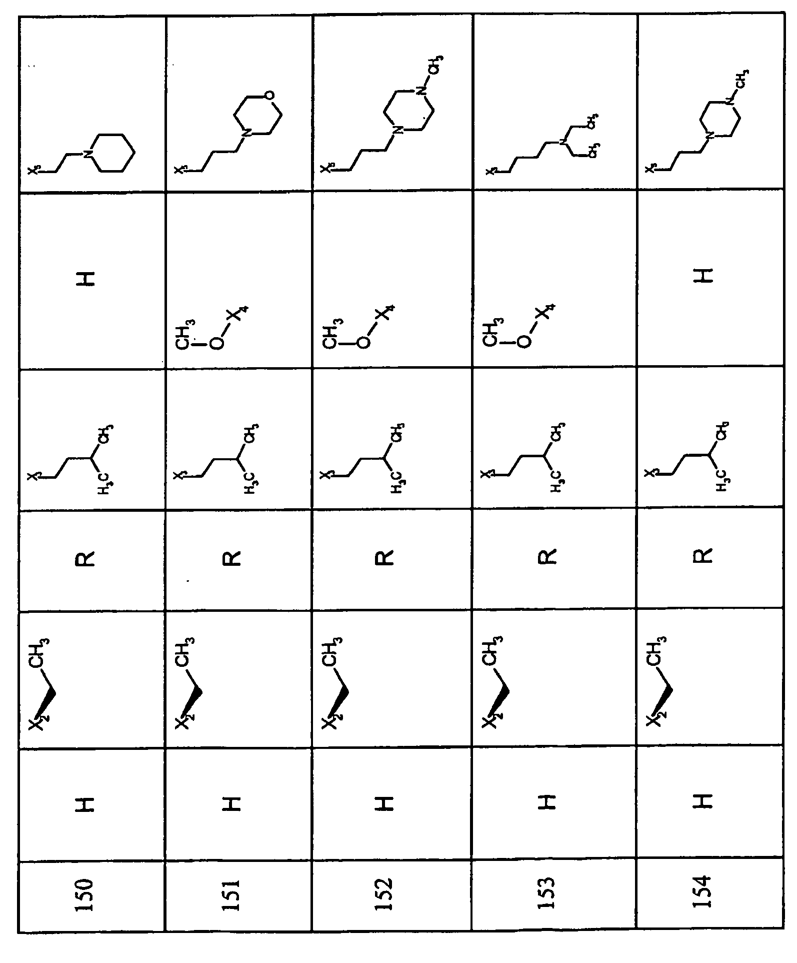

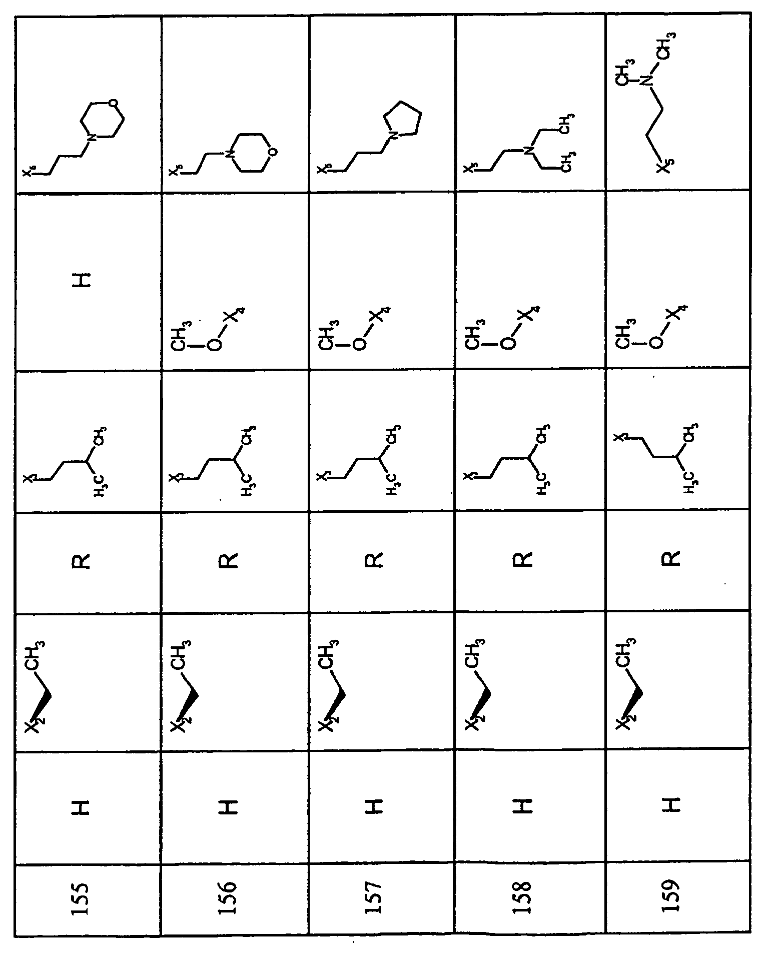

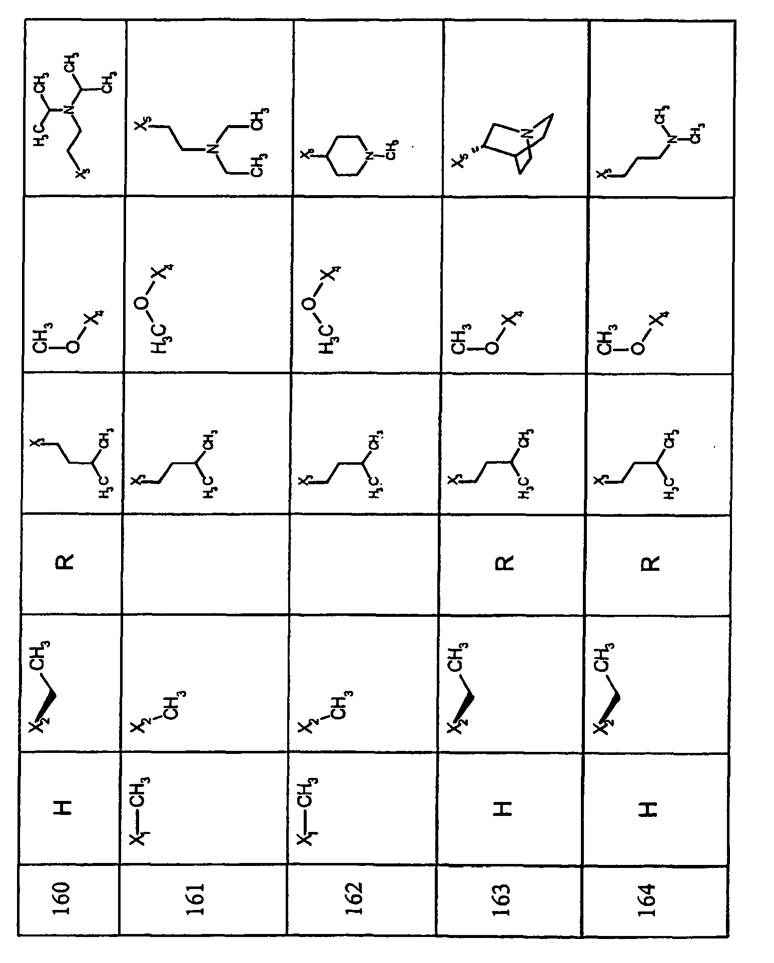

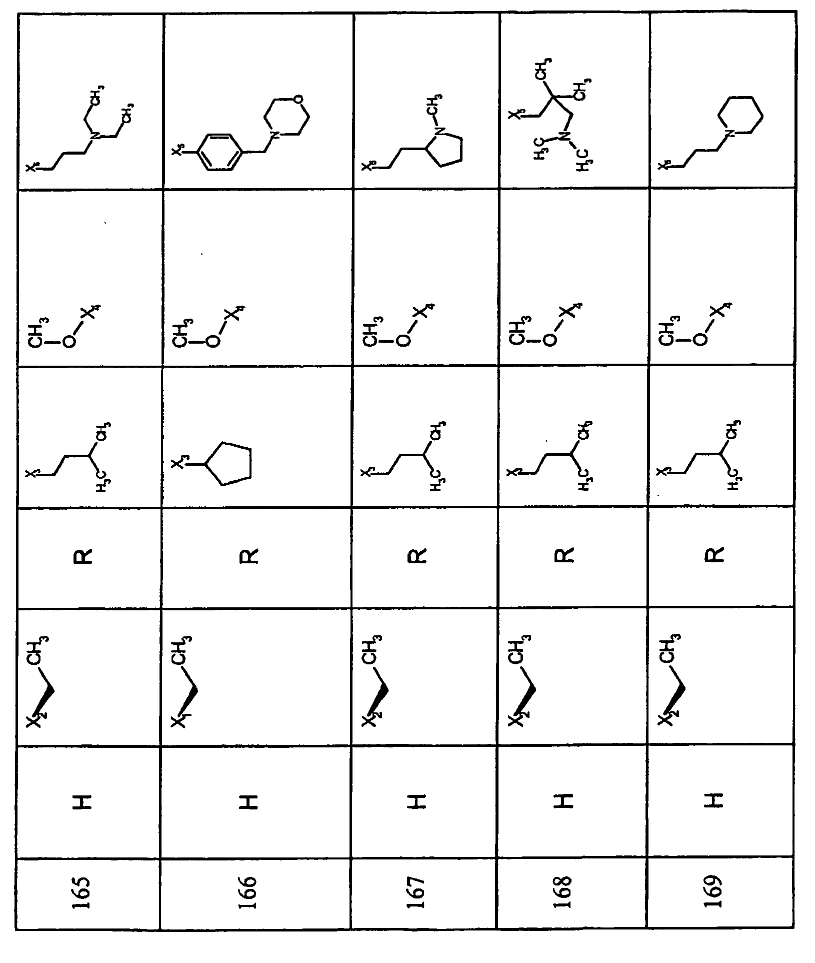

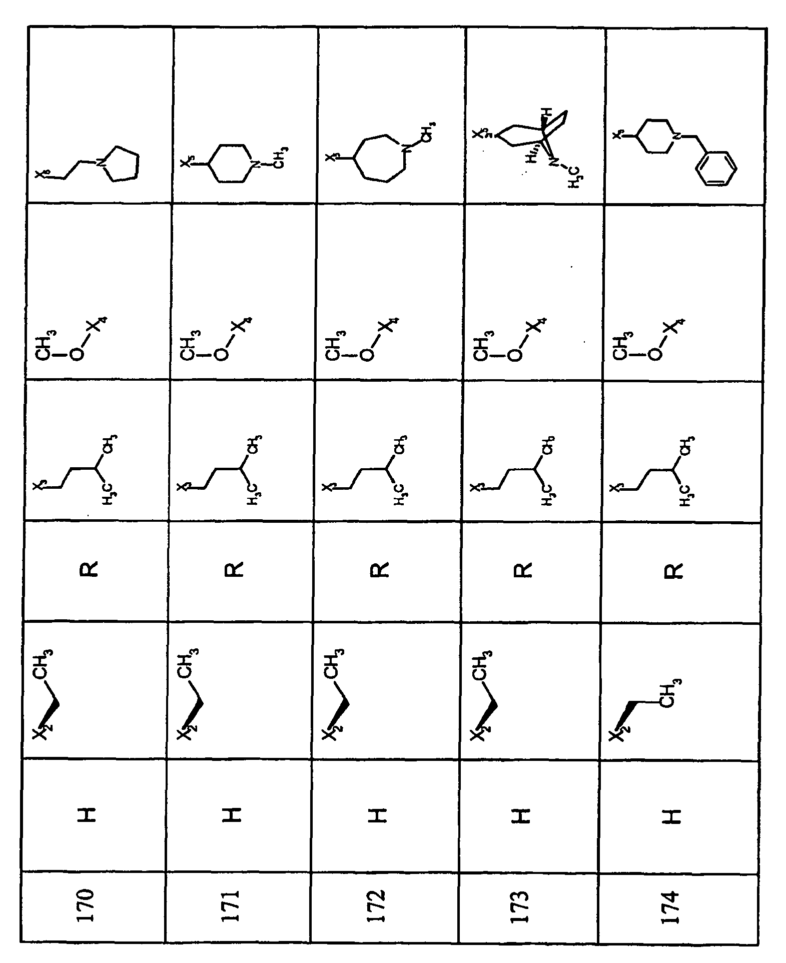

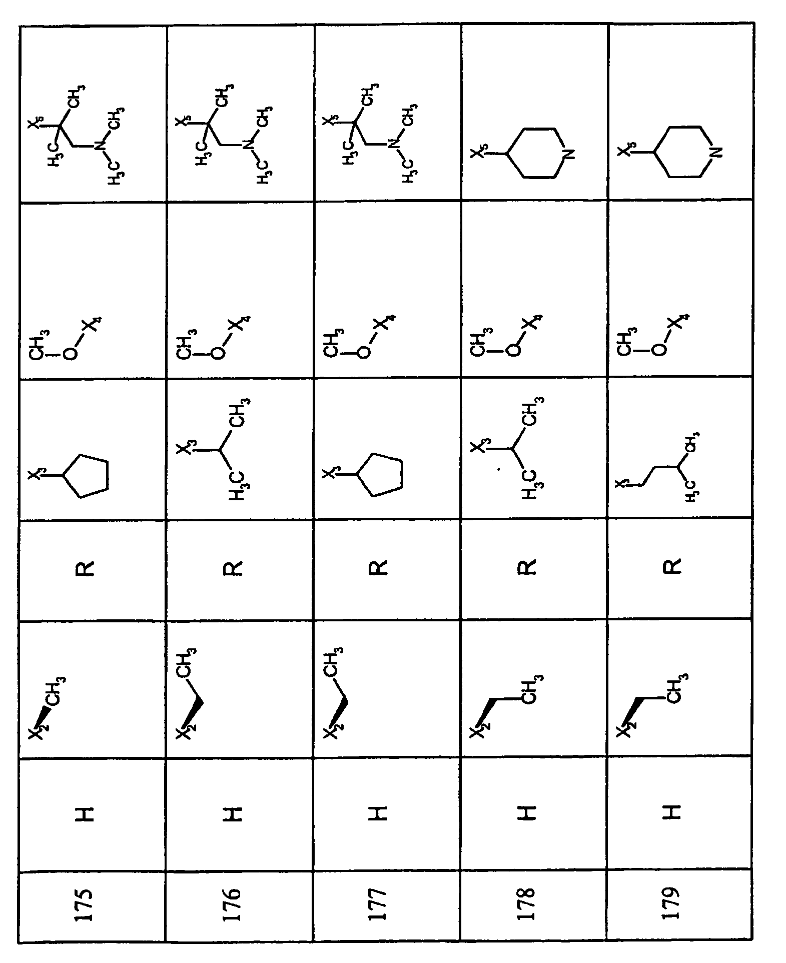

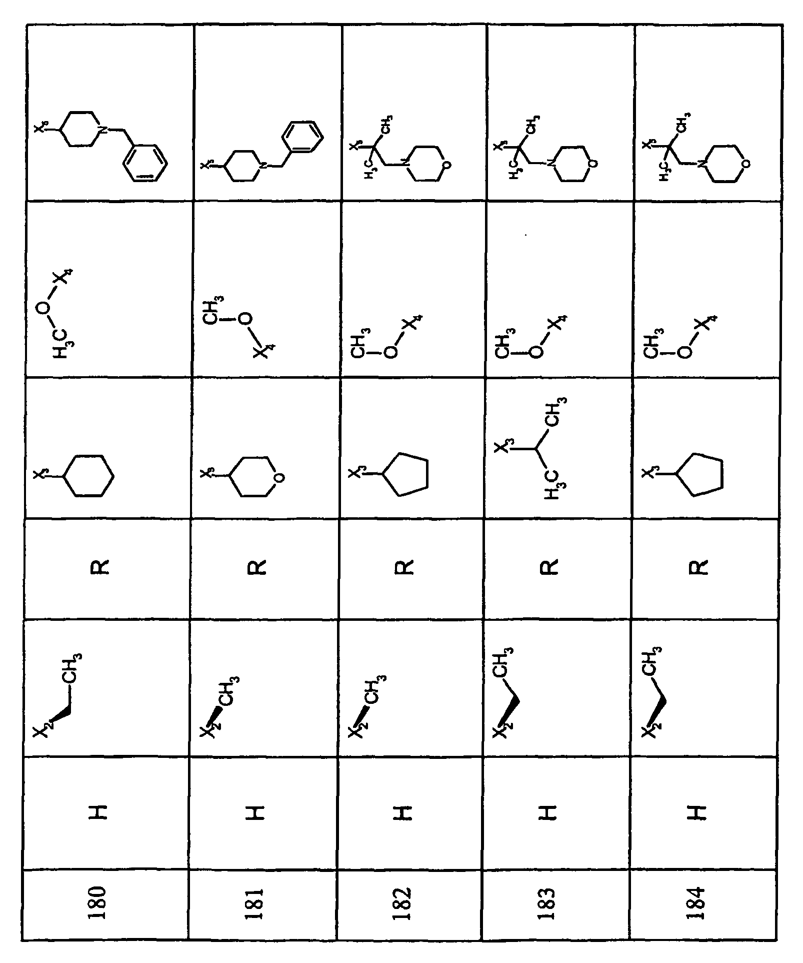

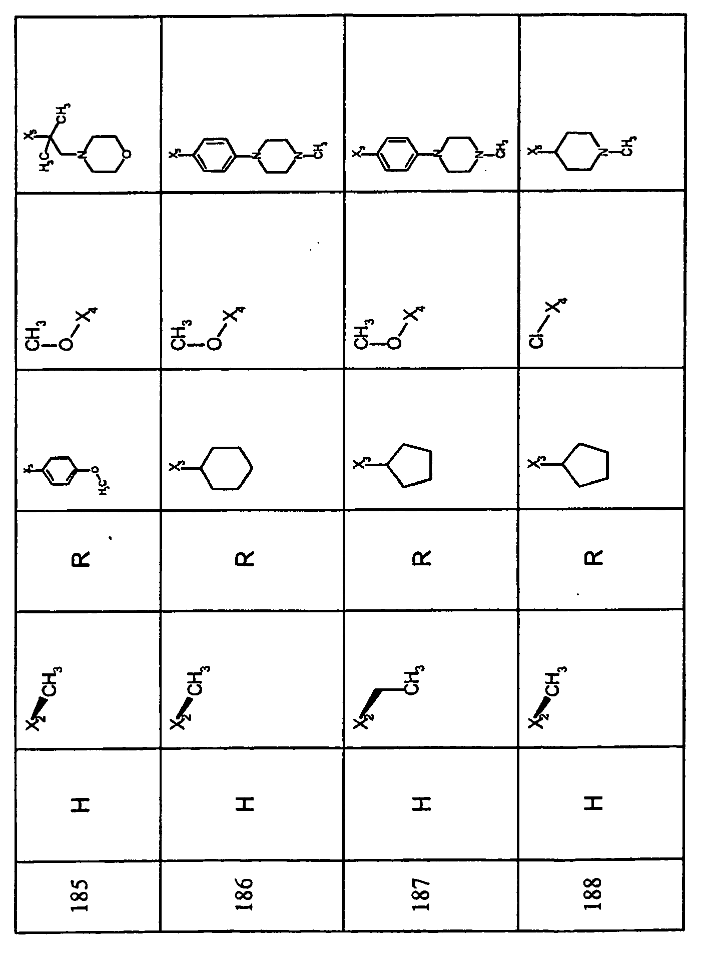

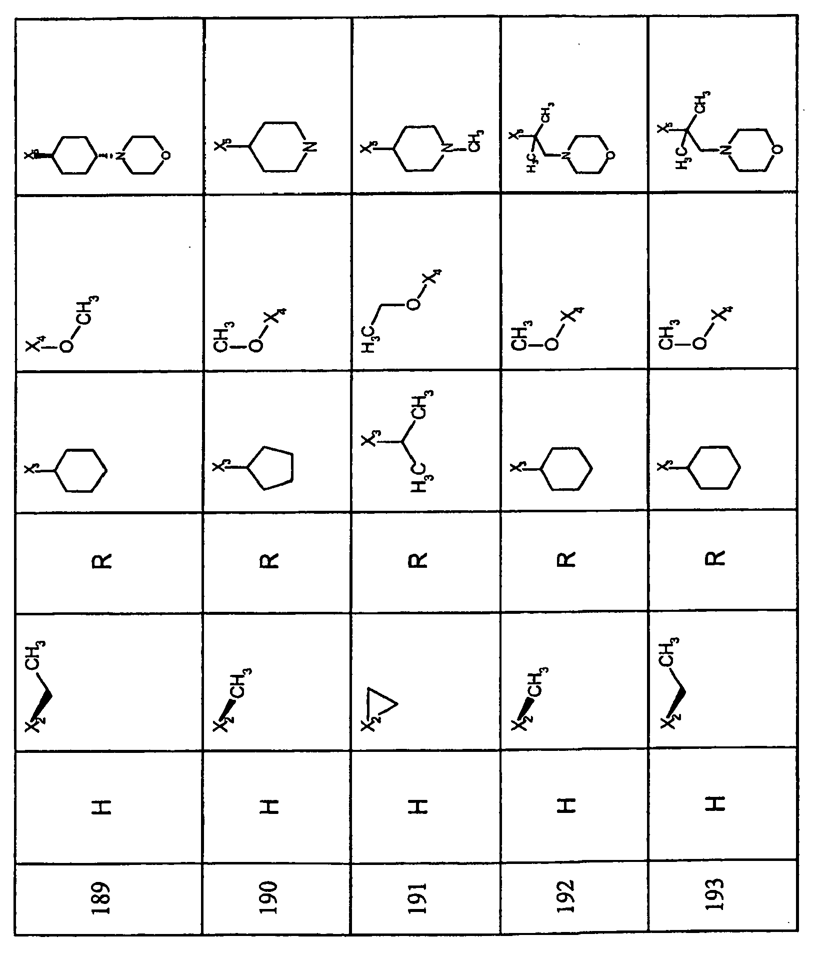

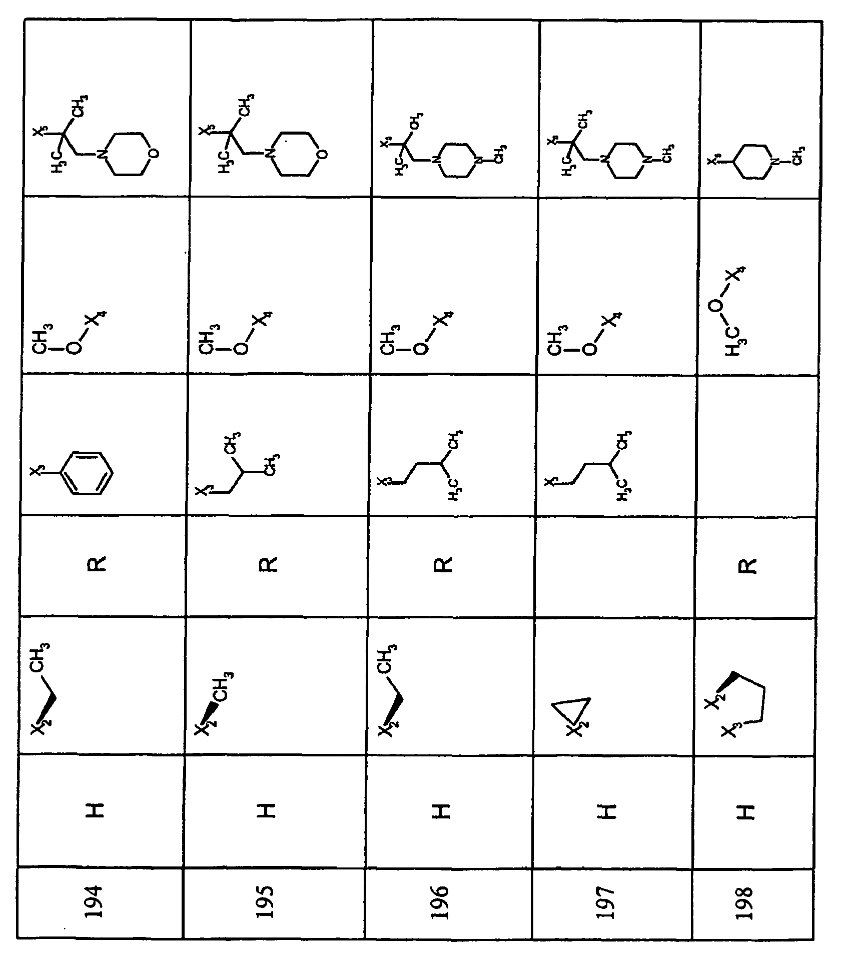

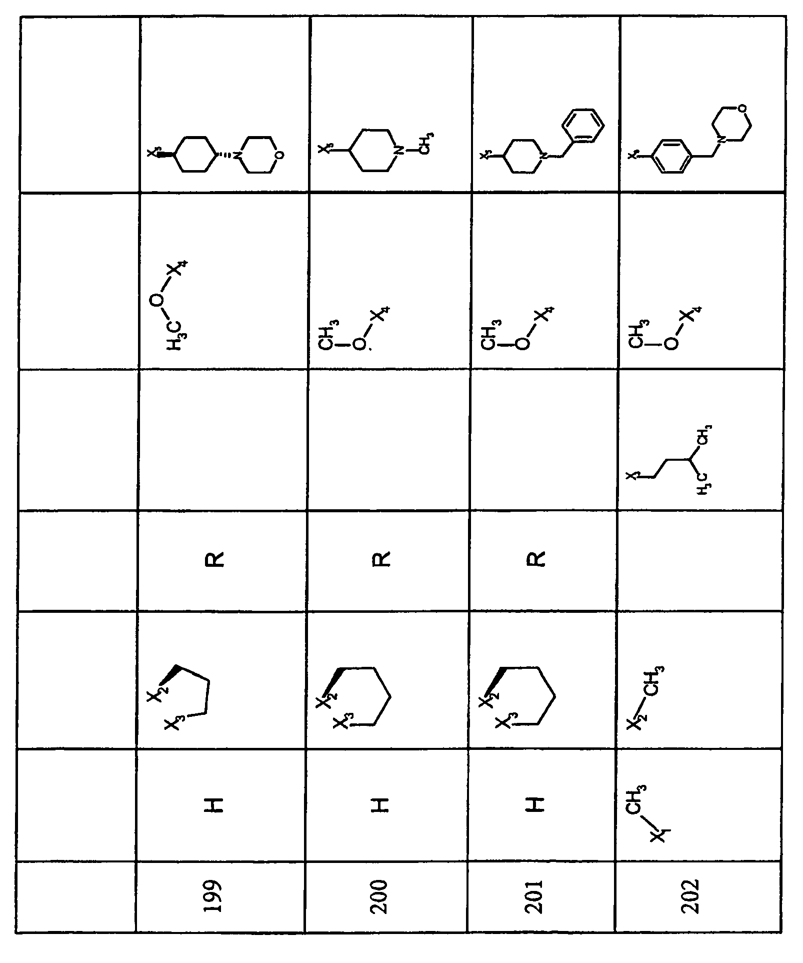

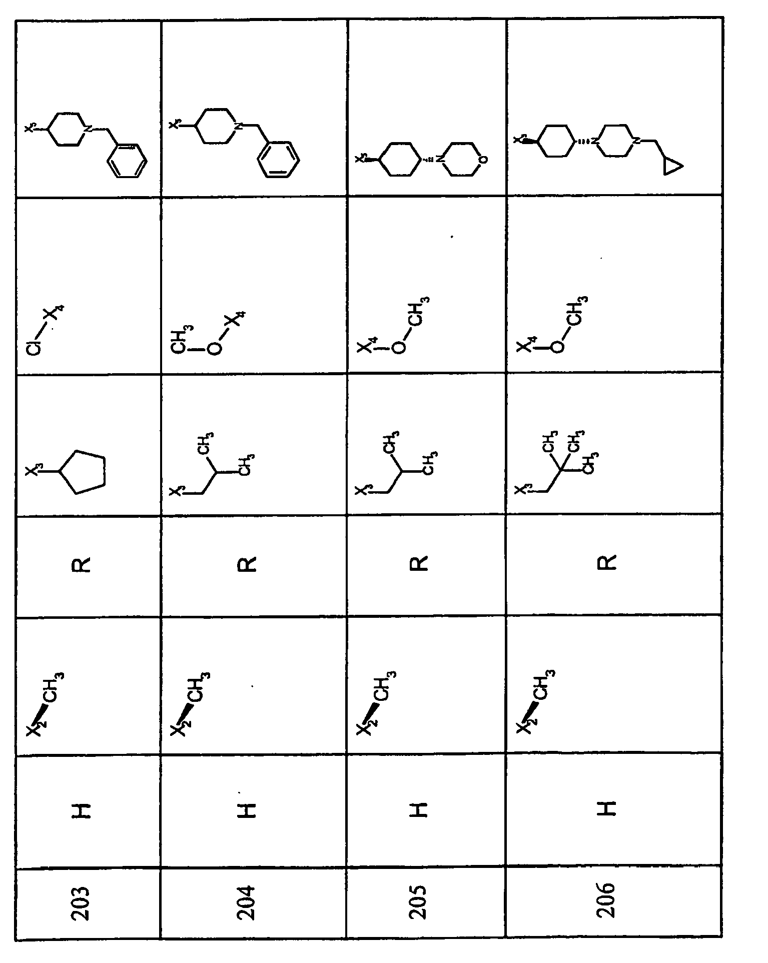

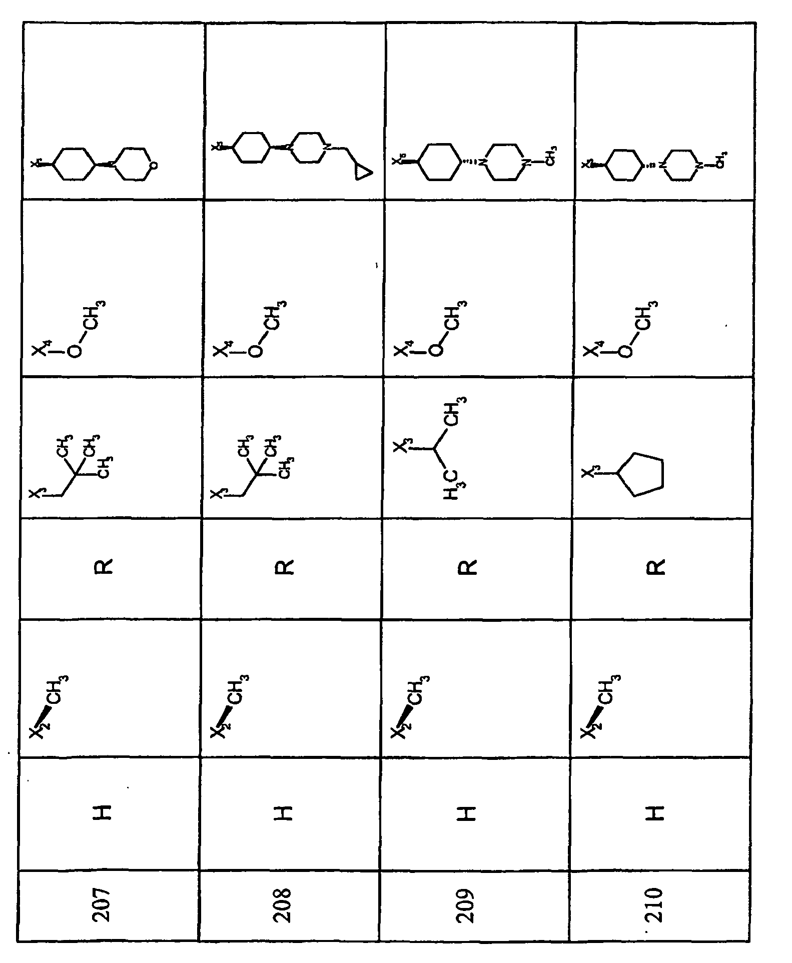

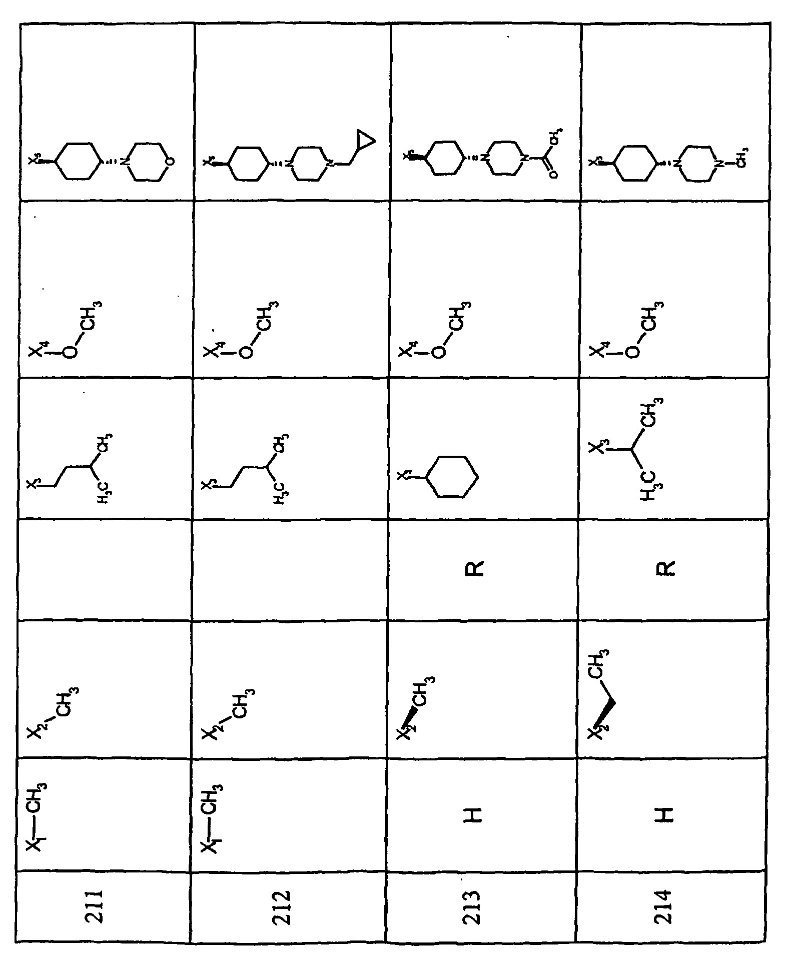

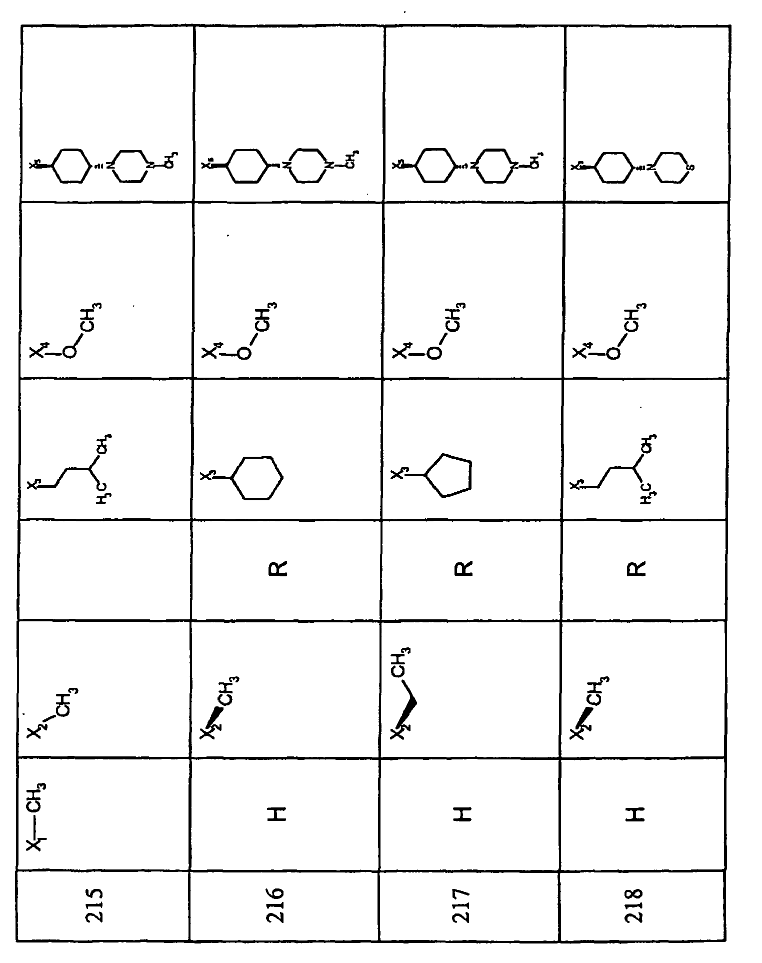

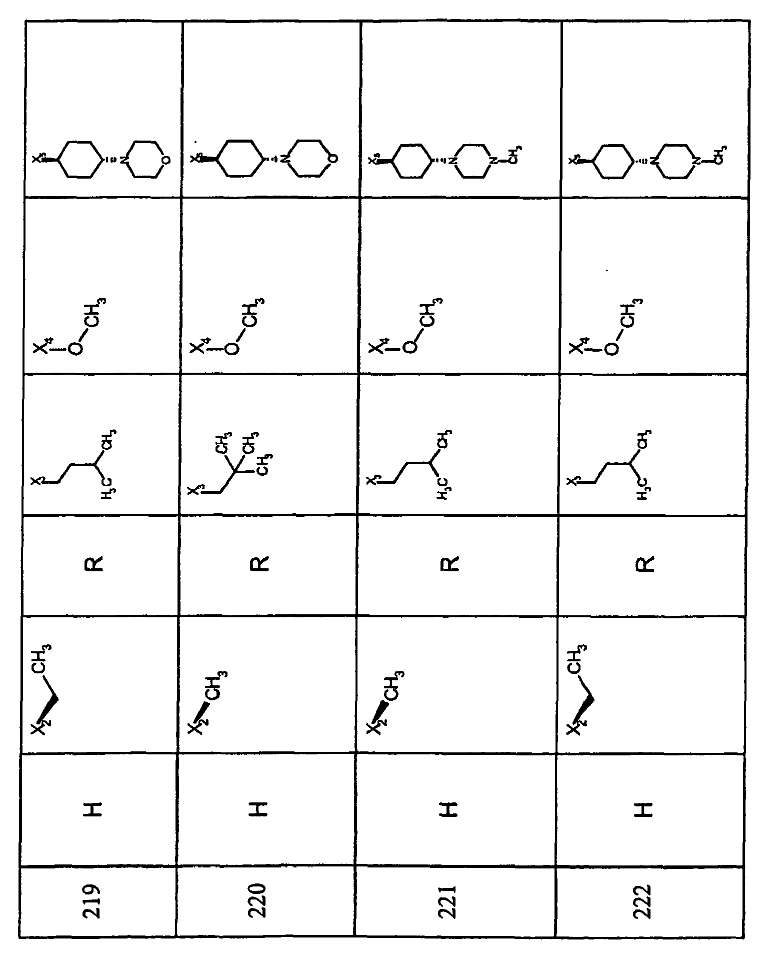

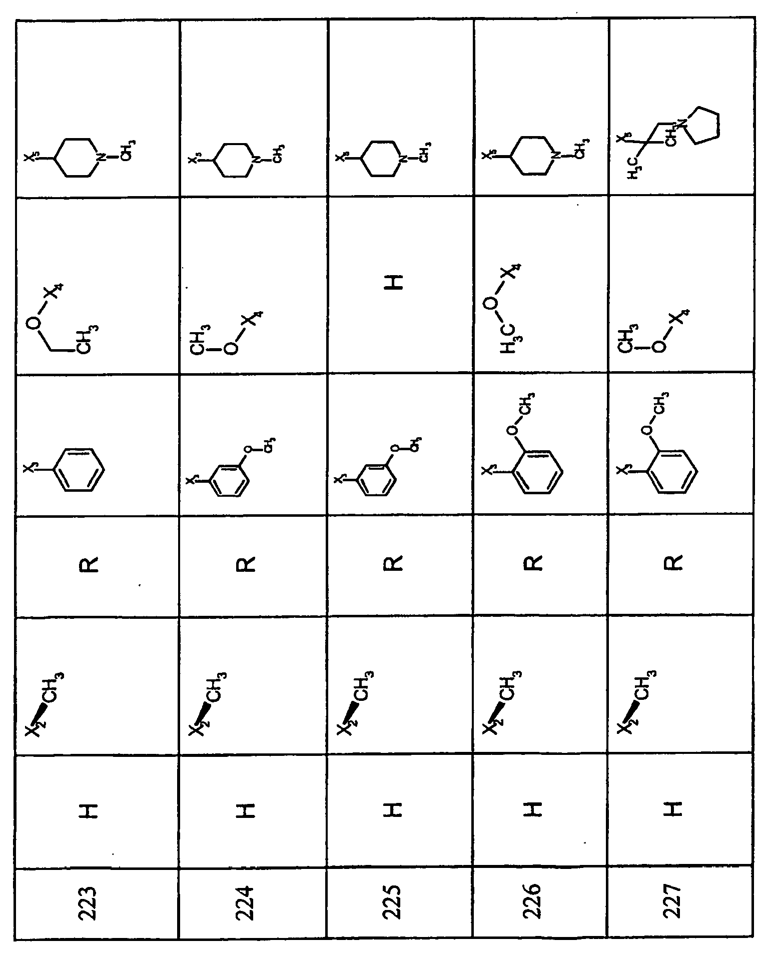

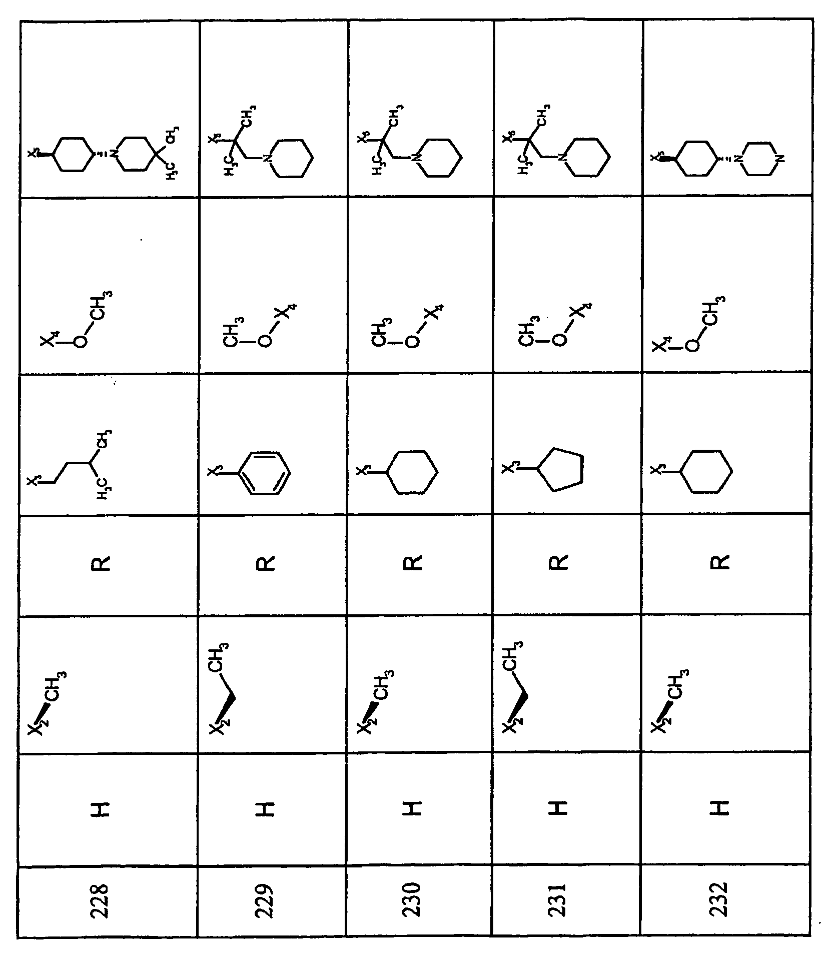

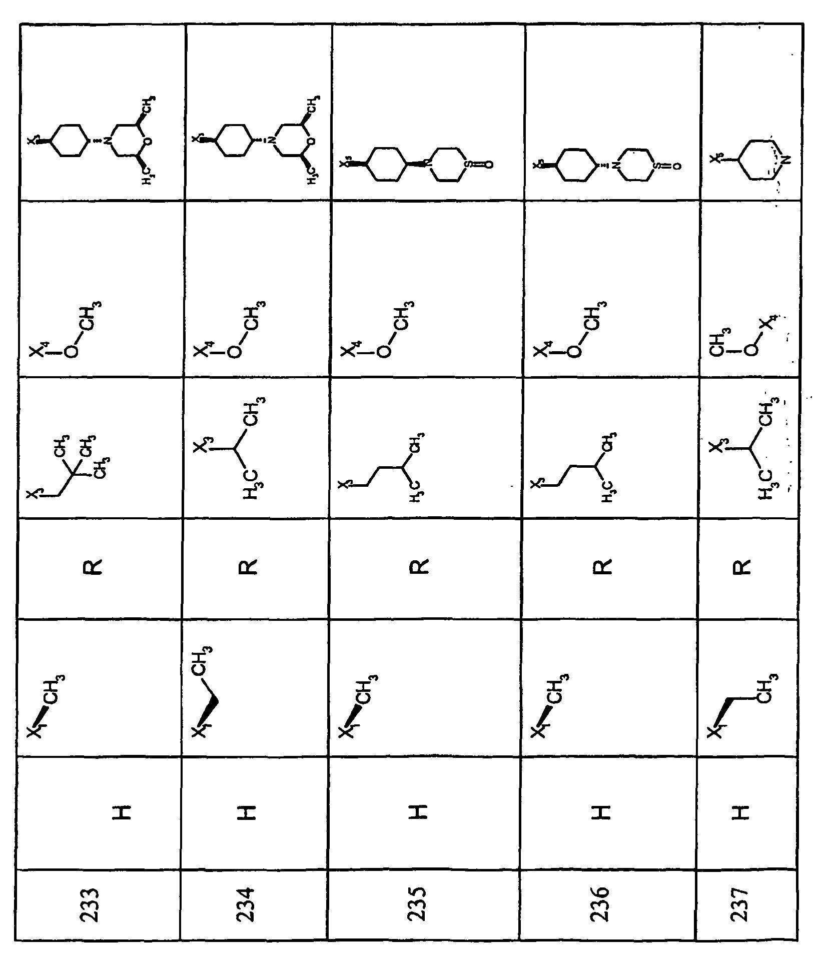

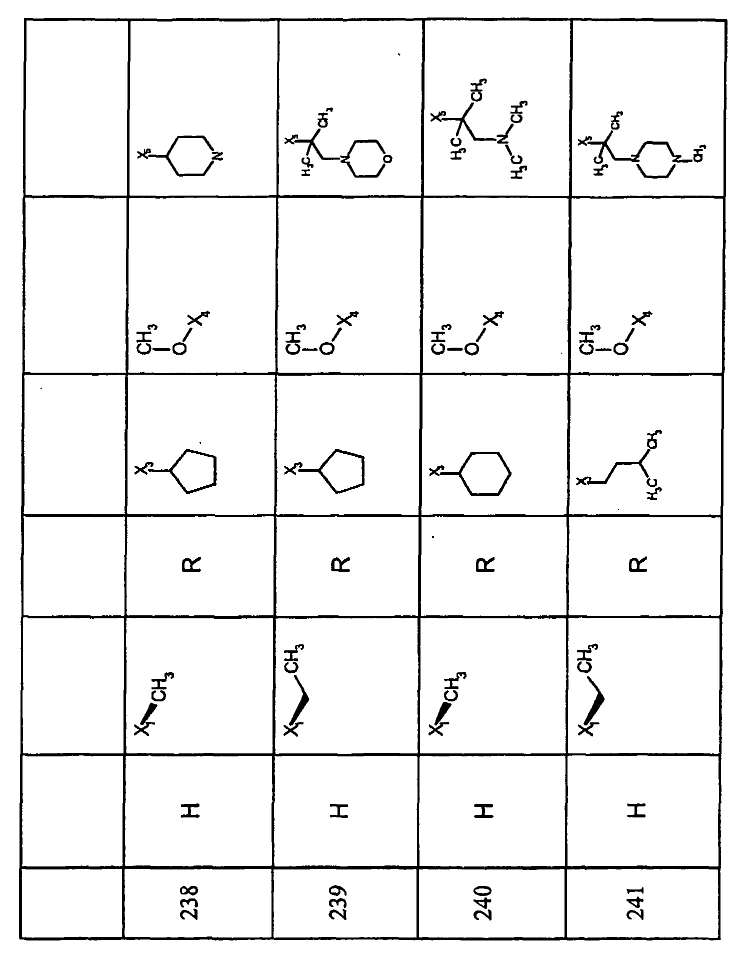

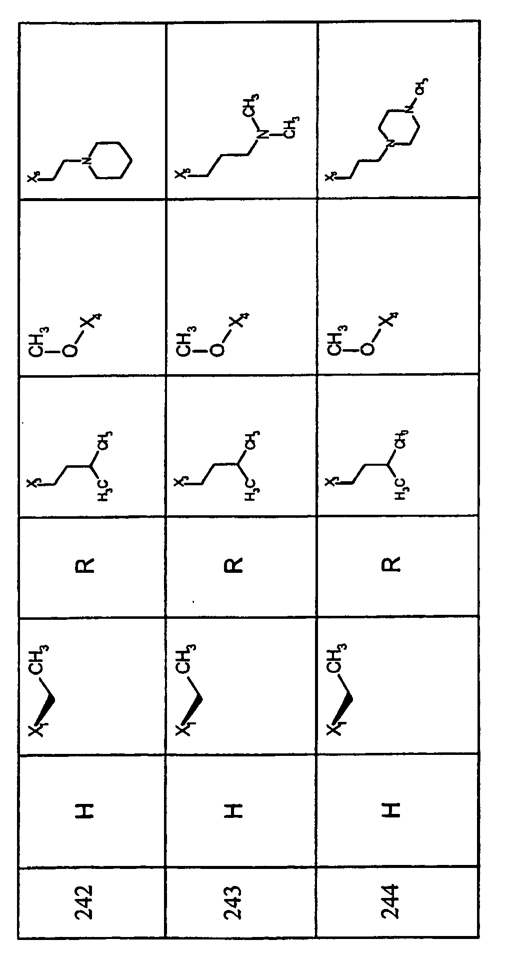

## Patentansprüche

1. Lagerstabile wässrige Infusions- bzw. Injektionslösungen enthaltend einen Wirkstoff der allgemeinen Formel (I) 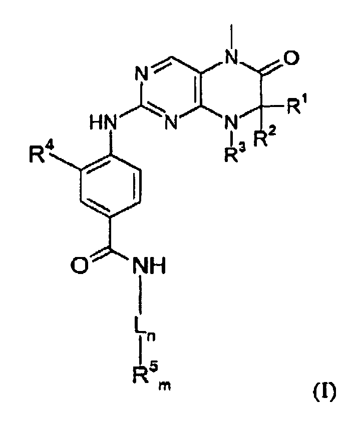 worin 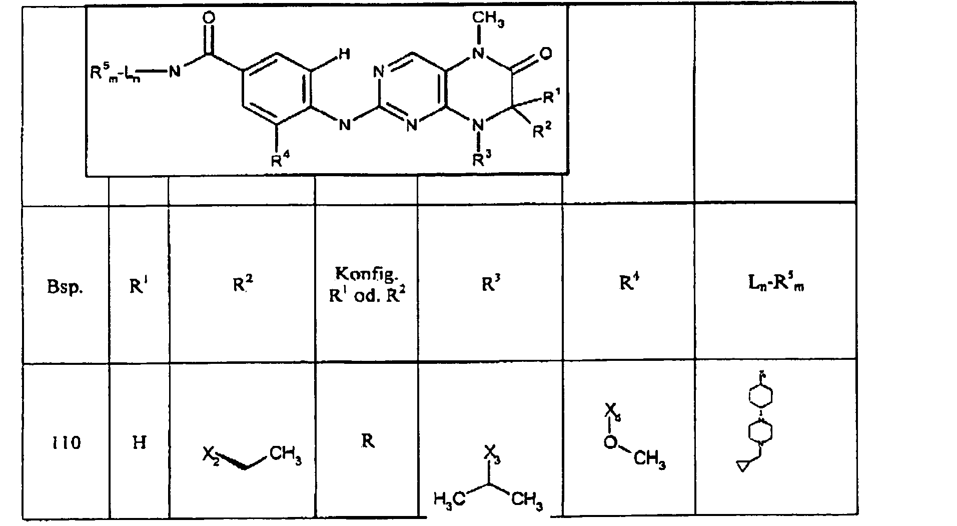 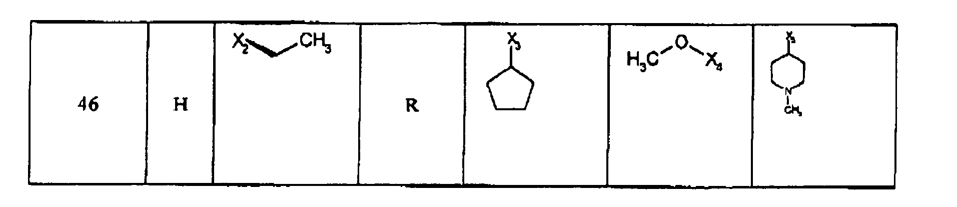 wobei die in der Tabelle verwendeten Abkürzungen X₁, X₂, X₃, X₄ und X₅ stehen jeweils für eine Verknüpfung mit einer Position in der unter der Tabelle aufgeführten allgemeinen Formel anstelle der entsprechenden Reste R¹, R², R³, R⁴ und Lₙ-R⁵ ₘ. oder dessen Tautomere, Racemate, Enantiomere, Diastereomere, sowie gegebenenfalls ihre pharmakologisch unbedenklichen Säureadditionssalze und eine zur Lösung des Wirkstoffs und zur Stabilisierung ausreichenden Menge einer physiologisch verträglichen Säure oder eines Säuregemisches, sowie gegebenenfalls weitere für die parenterale Anwendung geeignete Formulierungshilfsmittel.

2. Wässrige Infusions- bzw. Injektionslösungen nach Anspruch 1, **dadurch gekennzeichnet dass** sie zusätzlich EDTA enthält.

3. Wässrige Infusions- bzw. Injektionslösungen nach Anspruch 1 oder 2, **dadurch gekennzeichnet, dass** der Gehalt an gelöstem Wirkstoff 0,1 mg bis 10,0 mg in 1 ml Infusions- bzw. Injektionslösung beträgt.

4. Wässrige Infusions- bzw. Injektionslösungen nach einem der Ansprüche 1 bis 3, **dadurch gekennzeichnet, dass** eine oder mehrere zur Erzielung der Lager- und Verdünnungsstabilität eingesetzten Säuren ausgewählt sind aus der Gruppe bestehend aus Salzsäure, Essigsäure, Hydroxyessigsäure, Methansulfonsäure, Ethansulfonsäure, Phosphorsäure, Salpetersäure, Schwefelsäure, Zitronensäure, Weinsäure, Fumarsäure, Bernsteinsäure, Glutarsäure, Adipinsäure, Propionsäure, Ascorbinsäure, Maleisäure, Äpfelsäure, Glutaminsäure, Glukonsäure, Glukuronsäure, Galakturonsäure und Milchsäure.

5. Wässrige Infusions- bzw. Injektionslösungen nach einem der Ansprüche 1 bis 4, **dadurch gekennzeichnet, dass** das molare Verhältnis der physiologisch verträglichen Säure oder des Säuregemisches zum Wirkstoff maximal 3:1 beträgt

6. Wässrige Infusions- bzw. Injektionslösungen nach einem der Ansprüche 1 bis 5, **dadurch gekennzeichnet, dass** sie ein oder mehrere weitere Formulierungshilfsmittel ausgewählt aus der Gruppe bestehend aus Komplexierungsmitteln, Lichtschutzmitteln, Kristallisationsverzögerern, Verdickungsmitteln, Isotonisierungsmitteln, Antioxidantien und Euhydrierungsmittel enthalten.

7. Wässrige Infusions- bzw. Injektionslösungen nach einem der Ansprüche 1 bis 6, **dadurch gekennzeichnet, dass** die Osmolalität der Infusions- bzw. Injektionslösungen 200 - 600 mOsmol/kg beträgt.

8. Wässrige Infusions- bzw. Injektionslösungen nach einem der Ansprüche 1 bis 7, **dadurch gekennzeichnet, dass** sie einen pH-Wert von 2,4 bis 5,3 aufweisen.

9. Wässrige Infusion- bzw. Injektionslösungen nach einem der Ansprüche 1 bis 8, **dadurch gekennzeichnet, dass** sie 1,25 bis 3,0 Mol Salzsäure pro Mol Wirkstoff, bezogen auf 100 ml Infusions- bzw. Injektionslösung 0,75 bis 1,2 g NaCL enthalten, und eine Osmolalität von 260 bis 350 mOsmol/kg sowie einen pH-Wert von pH 3,5 bis 5,0 aufweisen.

10. Lyophilisate, Konzentrate und Suspensionen, **dadurch** gekenntzeichnet, dass sie durch Zugabe von Wasser eine wässrige Infusions- bzw. Injektionslösung gemäß einem der Ansprüche 1 bis 9 ergeben.

11. Infusions- bzw. Injektionslösungen gemäß einem der Ansprüche 1 bis 9 zur Verwendung als Arzneimittel mit antiproliferativer Wirkung.

12. Verwendung einer Infusions- bzw. Injektionslösung gemäß einem der Ansprüche 1 bis 9 zur Herstellung eines Arzneimittel zur Behandlung von Tumorerkrankungen, Infektionen, Entzündung- und Autoimmunerkrankungen.

13. Verwendung einer Infusions- bzw. Injektionslösung gemäß einem der Ansprüche 1 bis 9, welche einem Dosierungsbereich von 0,1 mg bis 50 mg Wirkstoff/ kg Körpergewicht entspricht.

14. Für Parenteralia geeignete Glascontainer oder flexible Plastikcontainer enthaltend Infusions- bzw. Injektionslösungen gemäß einem der Ansprüche 1 bis 9.

## Claims

1. Storage stable aqueous infusible or injectable solutions containing an active substance of general formula (I) 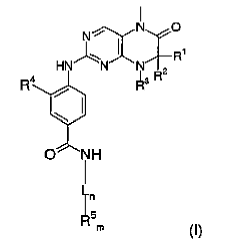 wherein 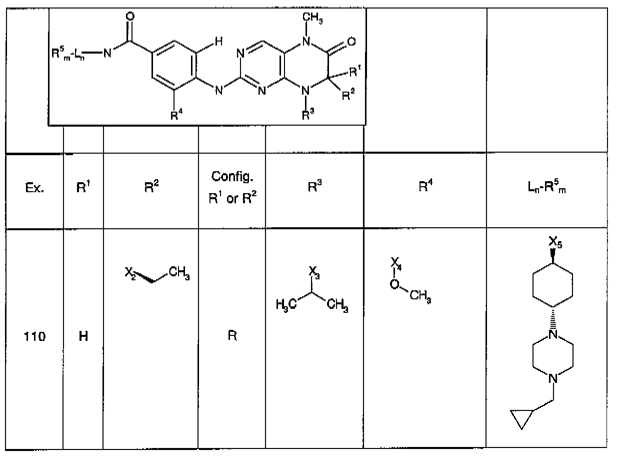 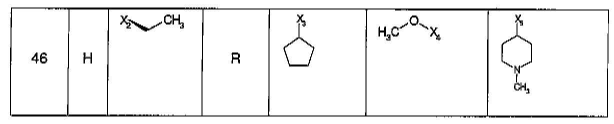 wherein the abbreviations X₁, X₂, X₃, X₄ and X₅ used in the Table in each case denote a link to a position in the general formula listed in the Table instead of the corresponding groups R¹, R², R³, R⁴ and Lₙ-R⁵ₘ,
or the tautomers, racemates, enantiomers, diastereomers and optionally the pharmacologically acceptable acid addition salts thereof and an amount of a physiologically acceptable acid or mixture of acids sufficient to dissolve the active substance and act as a stabiliser, optionally together with other formulating excipients suitable for parenteral administration

2. Aqueous infusible or injectable solutions according to claim 1, **characterised in that** they additionally contain EDTA.

3. Aqueous infusible or injectable solutions according to claim 1 or 2, **characterised in that** the content of dissolved active substance is 0.1 mg to 10.0 mg in 1 ml of infusible or injectable solution.

4. Aqueous infusible or injectable solutions according to one of claims 1 to 3, **characterised in that** one or more acids used as storage and dilution stabilisers are selected from among hydrochloric acid, acetic acid, hydroxyacetic acid, methanesulphonic acid, ethanesulphonic acid, phosphoric acid, nitric acid, sulphuric acid, citric acid, tartaric acid, fumaric acid, succinic acid, glutaric acid, adipic acid, propionic acid, ascorbic acid, maleic acid, malic acid, glutamic acid, gluconic acid, glucuronic acid, galacturonic acid and lactic acid.

5. Aqueous infusible or injectable solutions according to one of claims 1 to 4, **characterised in that** the molar ratio of the physiologically acceptable acid or mixture of acids to the active substance is at most 3:1.

6. Aqueous infusible or injectable solutions according to one of claims 1 to 5, **characterised in that** they contain one or more other formulating excipients selected from among complexing agents, light protecting agents, crystallisation inhibitors, thickeners, isotonic agents, antioxidants and euhydration agents.

7. Aqueous infusible or injectable solutions according to one of claims 1 to 6, **characterised in that** the osmolality of the infusible or injectable solutions is 200-600 mOsmol/kg.

8. Aqueous infusible or injectable solutions according to one of claims 1 to 7, **characterised in that** they have a pH of 2.4 to 5.3.

9. Aqueous infusible or injectable solutions according to one of claims 1 to 8, **characterised in that** they contain 1.25 to 3.0 mol hydrochloric acid per mol active substance, based on 100 ml infusible or injectable solution, 0.75 to 1.2 g NaCl, and have an osmolality of 260 to 350 mOsmol/kg and a pH of 3.5 to 5.0.

10. Lyophilisates, concentrates and suspensions, **characterised in that** by the addition of water they yield an aqueous infusible or injectable solution according to one of claims 1 to 9.

11. Infusible or injectable solutions according to one of claims 1 to 9 for use as medicaments with an antiproliferative activity.

12. Use of an infusible or injectable solution according to one of claims 1 to 9 for preparing a medicament for the treatment of tumoral diseases, infections, inflammatory and autoimmune diseases

13. Use of an infusible or injectable solution according to one of claims 1 to 9, which corresponds to a dosage range of from 0.1 mg to 50 mg of active substance/ kg body weight.

14. Glass containers or flexible plastic containers suitable for parenteral preparations, containing infusible or injectable solutions according to one of claims 1 to 9.

## Revendications

1. Solutions de perfusion ou d'injection aqueuses stables au stockage contenant une substance active de formule générale (I) 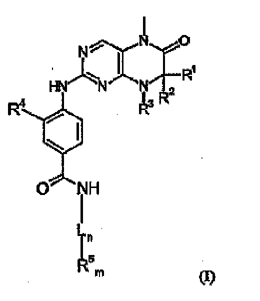 dans laquelle 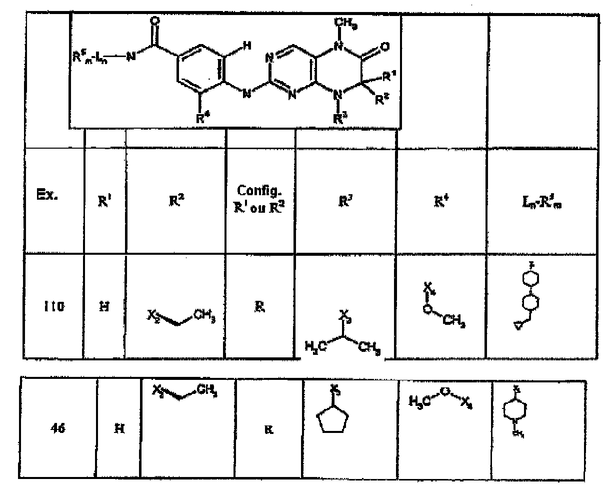 les abréviations X₁, X₂, X₃, X₄ et X₅ utilisées dans le tableau désignent respectivement une liaison avec une position dans la formule générale figurant dans le tableau à la place des radicaux correspondants R¹, R², R³, R⁴ et Lₙ-R⁵ₘ,
ou leurs tautomères, racémates, énantiomères, diastéréomères et éventuellement leurs sels d'addition acides pharmacologiquement acceptables et une quantité suffisante d'un acide ou d'un mélange d'acides physiologiquement acceptable, pour la dissolution et pour la stabilisation de la substance active, et éventuellement d'autres auxiliaires de formulation appropriés pour l'utilisation parentérale.

2. Solutions de perfusion ou d'injection aqueuses selon la revendication 1, **caractérisées en ce qu'**elles contiennent en outre de l'EDTA.

3. Solutions de perfusion ou d'injection aqueuses selon la revendication 1 ou 2, **caractérisées en ce que** la teneur en substance active dissoute est de 0,1 mg à 10,0 mg dans 1 ml de solution pour perfusion ou pour injection.

4. Solutions de perfusion ou d'injection aqueuses selon l'une des revendications 1 à 3, **caractérisées en ce qu'**un ou plusieurs des acides utilisés pour obtenir la stabilité au stockage et à la dilution sont choisis dans le groupe comprenant l'acide chlorhydrique, l'acide acétique, l'acide hydroxy-acétique, l'acide méthanesulfonique, l'acide éthanesulfonique, l'acide phosphorique, l'acide nitrique, l'acide sulfurique, l'acide citrique, l'acide tartrique, l'acide fumarique, l'acide succinique, l'acide glutarique, l'acide adipique, l'acide propionique, l'acide ascorbique, l'acide maléique, l'acide malique, l'acide glutamique, l'acide gluconique, l'acide glucuronique, l'acide galacturonique et l'acide lactique.

5. Solutions de perfusion ou d'injection aqueuses selon l'une des revendications 1 à 4, **caractérisées en ce que** le rapport molaire de l'acide ou du mélange d'acides physiologiquement acceptable à la substance active est au maximum de 3:1.

6. Solutions de perfusion ou d'injection aqueuses selon l'une des revendications 1 à 5, **caractérisées en ce qu'**elles contiennent un ou plusieurs auxiliaires de formulation choisis dans le groupe comprenant des agents complexants, des photoprotecteurs, des agents retardant la cristallisation, des épaississants, des agents isotoniques, des anti-oxydants et des agents d'hydrogénation.

7. Solutions de perfusion ou d'injection aqueuses selon l'une des revendications 1 à 6, **caractérisées en ce que** l'osmolalité des solutions de perfusion ou d'injection est de 200 à 600 mOsmol/kg_{.}

8. Solutions de perfusion ou d'injection aqueuses selon l'une des revendications 1 à 7, **caractérisées en ce qu'**elles présentent un pH de 2,4 à 5,3.

9. Solutions de perfusion ou d'injection aqueuses selon l'une des revendications 1 à 8, **caractérisées en ce qu'**elles présentent 1,25 à 3,0 moles d'acide chlorhydrique par mole de substance active par rapport à 100 ml de solution pour perfusion ou injection, 0,75 à 1,2 g de NaCl et une osmolalité de 260 à 350 mOsmol/kg et un pH de 3,5 à 5,0.

10. Lyophilisats, concentrés et suspensions, **caractérisés en ce que** par addition d'eau, ils donnent une solution pour perfusion ou injection aqueuse selon l'une des revendications 1 à 9.

11. Solutions de perfusion ou d'injection aqueuses selon l'une des revendications 1 à 9, pour l'utilisation commue médicament à effet anti-prolifératif.

12. Utilisation d'une solution pour perfusion ou injection selon l'une des revendications 1 à 9, pour la production d'un médicament pour le traitement de maladies tumorales, d'infections, de maladies inflammatoires et auto-immunes.

13. Utilisation d'une solution pour perfusion ou injection selon l'une des revendications 1 à 9, qui correspond à une plage de dosage de 0,1 mg à 50 mg de substance active/kg de poids corporel.

14. Conteneur en verre ou conteneur en plastique flexible approprié pour l'administration parentérale contenant des solutions pour perfusion ou injection selon l'une des revendications 1 à 9.
